(19) Europäisches Patentamt / European Patent Office / Office européen des brevets

(11) EP 2 595 997 B1

(12) **EUROPÄISCHE PATENTSCHRIFT**

(45) Veröffentlichungstag und Bekanntmachung des Hinweises auf die Patenterteilung:
**16.09.2015 Patentblatt 2015/38**

(21) Anmeldenummer: **11746501.3**

(22) Anmeldetag: **20.07.2011**

(51) Int Cl.:
***C07F 9/50*** (2006.01) ***C07F 9/6561*** (2006.01)
***C07D 471/04*** (2006.01) ***C07F 5/02*** (2006.01)
***C09K 11/06*** (2006.01) ***H01L 51/50*** (2006.01)
***H01L 51/00*** (2006.01)

(86) Internationale Anmeldenummer:
**PCT/EP2011/062491**

(87) Internationale Veröffentlichungsnummer:
**WO 2012/010650 (26.01.2012 Gazette 2012/04)**

(54) **KUPFER(I)-KOMPLEXE FÜR OPTO-ELEKTRONISCHE VORRICHTUNGEN**

COPPER(I) COMPLEXES FOR OPTOELECTRONIC DEVICES

COMPLEXES DE CUIVRE(I) POUR LES APPAREILES OPTOELECTRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.07.2010 DE 102010031831**

(43) Veröffentlichungstag der Anmeldung:
**29.05.2013 Patentblatt 2013/22**

(73) Patentinhaber: **cynora GmbH**
**76646 Bruchsal (DE)**

(72) Erfinder:
- **YERSIN, Hartmut**
  **93161 Sinzing (DE)**
- **CZERWIENIEC, Rafal**
  **93083 Obertraubling (DE)**
- **MONKOWIUS, Uwe**
  **A-4020 Linz (AT)**

(74) Vertreter: **Hoppe, Georg Johannes**
**Darani Anwaltskanzlei**
**Beuckestrasse 20**
**14163 Berlin (DE)**

(56) Entgegenhaltungen:
**WO-A1-2010/086089 DE-A1-102007 031 261**
**DE-A1-102008 048 336**

- **ZHANG Q ET AL: "Highly efficient green phosphorescent organic light-emitting diodes based on Cu(I) complexes", ADVANCED MATERIALS, WILEY VCH VERLAG, DE, Bd. 16, Nr. 5, 1. Januar 2004 (2004-01-01) , Seiten 432-436, XP002359145, ISSN: 0935-9648, DOI: 10.1002/ADMA.200306414**
- **ZHANG Q ET AL: "Novel Heteroleptic CuI Complexes with tunable Emission Color for Efficient Phosphorescent Light-Emitting Diodes", ADVANCED FUNCTIONAL MATERIALS, WILEY - V C H VERLAG GMBH & CO. KGAA, DE, Bd. 17, Nr. 15, 1. Oktober 2007 (2007-10-01), Seiten 2983-2990, XP002634723, ISSN: 1616-301X, DOI: 10.1002/ADFM.200601053 [gefunden am 2007-08-31]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 595 997 B1

## Beschreibung

Die vorliegende Erfindung betrifft die Verwendung von löslichen Kupfer(I)komplexen (Cu(I)-Komplexen) als Emitter in OLEDs (organic light emitting diodes) und in anderen opto-elektronischen Anordnungen.

## Einleitung

Aktuell setzen sich im Bereich der Bildschirm- und Licht-Technik neue Verfahren durch. Es wird möglich sein, flache Displays oder Leuchtflächen mit einer Dicke von unter 0,5 mm zu fertigen. Diese sind durch viele faszinierende Eigenschaften ausgezeichnet. So werden z. B. Leuchtflächen als Tapeten mit sehr geringem Energieverbrauch realisierbar sein. Besonders interessant ist aber, dass Farbbildschirme mit bisher nicht erreichbarer Farb-Echtheit, Helligkeit und Blickwinkelunabhängigkeit, mit geringem Gewicht sowie sehr niedrigem Stromverbrauch herstellbar sein werden. Die Bildschirme werden sich als Mikro-Displays oder Großbildschirme mit mehreren Quadratmetern Fläche in starrer Form oder flexibel, aber auch als Transmissions- oder Reflexions-Displays gestalten lassen. Ferner wird es möglich sein, einfache und kostensparende Herstellungsverfahren wie Siebdruck oder Tintenstrahldruck einzusetzen. Dadurch wird im Vergleich zu herkömmlichen Flachbildschirmen eine sehr preiswerte Fertigung ermöglicht. Diese neue Technik basiert auf dem Prinzip der OLEDs, den Organic Light Emitting Diodes. Darüber hinaus zeichnen sich durch die Verwendung spezieller metall-organischer Materialien (Moleküle) viele neue opto-elektronische Anwendungen, z. B. im Bereich organischer Solarzelle, organischer Feldeffekttransistoren, organischer Photodioden usw. ab.

So lässt sich besonders für den OLED-Bereich erkennen, dass derartige Anordnungen bereits jetzt wirtschaftlich bedeutend sind, da eine Massenfertigung in Kürze zu erwarten ist. Derartige OLEDs bestehen vorwiegend aus organischen Schichten, die auch flexibel und kostengünstig zu fertigen sind. OLED-Bauelemente lassen sich großflächig als Beleuchtungskörper, aber auch klein als Pixels für Displays gestalten.

Gegenüber herkömmlichen Technologien, wie etwa Flüssigkristall-Displays (LCDs), Plasma-Displays oder Kathodenstrahlenröhren (CRTs) weisen OLEDs zahlreiche Vorteile auf, wie eine geringe Betriebsspannung von einigen Volt, eine dünne Struktur von einigen hundert nm, hoch-effizient selbst-leuchtende Pixel, einen hohen Kontrast und eine gute Auflösung sowie die Möglichkeit, alle Farben darzustellen. Weiterhin wird in einem OLED Licht beim Anliegen elektrischer Spannung direkt erzeugt, anstelle es nur zu modulieren.

Einen Überblick über die Funktion von OLEDs findet sich beispielsweise bei H. Yersin, Top. Curr. Chem. 2004, 241, 1 und H. Yersin, "Highly Efficient OLEDs with Phosphorescent Materials"; Wiley-VCH, Weinheim, Germany, 2008.

Seit den ersten Berichten über OLEDs (siehe z. B. Tang et al., Appl. Phys. Lett. 1987, 51, 913) sind diese Vorrichtungen insbesondere im Hinblick auf die eingesetzten Emittermaterialien weiterentwickelt worden, wobei insbesondere in den letzten Jahren sogenannte Triplett- oder auch andere phosphoreszierende Emitter von Interesse sind.

OLEDs werden in der Regel in Schichtenstrukturen realisiert. Zum besseren Verständnis ist in Abb. 1 ein prinzipieller Aufbau eines OLEDs gezeigt. Aufgrund der angelegten äußeren Spannung an einer transparenten Indium-Zinn-Oxid-Anode (ITO) und einer dünnen Metall-Kathode werden von der Anode positive Löcher und von der Kathode negative Elektronen injiziert. Diese verschieden geladenen Ladungsträger gelangen über Zwischenschichten, zu denen auch hier nicht gezeichnete Loch- bzw. Elektronen-Blockierschichten gehören können, in die Emissionsschicht. Dort treffen die entgegengesetzt geladenen Ladungsträger an oder in der Nähe von dotierten Emitter-Molekülen zusammen und rekombinieren. Die Emitter-Moleküle sind in der Regel in Matrix-Molekülen oder Polymermatrizen (in z. B. 2 bis 10 Gew.-%) eingelagert, wobei die Matrix-Materialien so gewählt sind, dass sie auch einen Loch- und Elektronentransport ermöglichen. Durch die Rekombination entstehen Exzitonen (= Anregungszustände), die ihre Überschussenergie auf die jeweilige elektrolumineszierende Verbindung übertragen. Diese elektrolumineszierende Verbindung kann daraufhin in einen bestimmten elektronischen Anregungszustand übergehen, der dann möglichst vollständig und unter weitgehender Vermeidung strahlungsloser Desaktivierungsprozesse durch Lichtemission in den zugehörigen Grundzustand umgewandelt wird.

Als elektronischer Anregungszustand, der auch durch Energieübertragung von einem geeigneten Vorläufer-Exziton gebildet werden kann, kommt, von wenigen Ausnahmen abgesehen, entweder ein Singulett- oder ein Triplett-Zustand, bestehend aus drei Unterzuständen, in Betracht. Da beide Zustände aufgrund der Spinstatistik in der Regel im Verhältnis 1:3 besetzt werden, ergibt sich, dass bei einer Emission aus dem Singulett-Zustand, die als Fluoreszenz bezeichnet wird nur maximal 25 % der erzeugten Exzitonen wieder zur Emission führen. Dagegen können bei einer Triplett-Emission, die als Phosphoreszenz bezeichnet wird, sämtliche Exzitonen ausgenutzt, umgewandelt und als Licht emittiert werden (Triplett-Harvesting), so dass in diesem Fall die Innere Quantenausbeute den Wert von 100 % erreichen kann, sofern der mit angeregte und energetisch über dem Triplett-Zustand liegende Singulett-Zustand vollständig in den Triplett-Zustand relaxiert (Inter-System-Crossing, ISC) und strahlungslose Konkurrenzprozesse bedeutungslos bleiben. Somit sind Triplett-Emitter nach dem bisherigen Stand der Technik effizientere Elektro-Luminophore und besser geeignet, in einer organischen Leuchtdiode für eine hohe Lichtausbeute zu sorgen.

Bei den für das Triplett-Harvesting geeigneten Triplett-Emittern werden in der Regel Übergangsmetall-Komplexverbindungen eingesetzt, in denen das Metall aus der dritten Periode der Übergangsmetalle gewählt wird. Hierbei handelt es sich vorwiegend um sehr teure Edelmetalle wie Iridium, Platin oder auch Gold. (Siehe dazu auch H. Yersin, Top. Curr. Chem. 2004, 241, 1 und M. A. Baldo, D. F. O'Brien, M. E. Thompson, S. R. Forrest, Phys. Rev. B 1999, 60, 14422). Der wesentliche Grund dafür liegt in der hohen Spin-Bahn-Kopplung (SBK) der Edelmetall-Zentralionen (SBK-Konstante Ir(III): ≈ 4000 $cm^{-1}$; Pt(II): ≈ 4500 $cm^{-1}$; Au(I): ≈ 5100 $cm^{-1}$; Ref.: S. L. Murov, J. Carmicheal, G. L. Hug, Handbook of Photochemistry, 2nd Edition, Marcel Dekker, New York 1993, p. 338 ff). Durch diese quantenmechanische Eigenschaft wird der ohne SBK für optische Übergänge strikt verbotene Triplett-Singulett-Übergang erlaubt und die für die OLED-Anwendung erforderliche kurze Emissionslebensdauer von wenigen μs erreicht.

Es wäre von großem wirtschaftlichen Vorteil, wenn diese teuren Edelmetalle durch preiswerte Metalle ersetzt werden könnten. Darüber hinaus ist eine Vielzahl der bisher bekannten OLED-Emitter-Materialien aus ökologischer Sicht nicht unbedenklich, so dass die Verwendung von weniger toxischen Materialien wünschenswert wäre. Hierfür kämen z. B. Kupfer(I)-Komplexe in Betracht. Allerdings weisen diese eine wesentlich geringere SBK auf (SBK-Konstanten von Cu(I): ≈ 850 $cm^{-1}$, Ref.: S. L. Murov, J. Carmicheal, G. L. Hug, Handbook of Photochemistry, 2nd Edition, Marcel Dekker, New York 1993, p. 338 ff), als die oben genannten Zentralionen. Damit wären die sehr wichtigen Triplett-Singulett-Übergänge von Cu(I)-Komplexen relativ stark verboten, und Emissionslebensdauern wären für OLED-Anwendungen mit einigen 100 μs bis ms zu lang. Bei derart langen Emissionsabklingzeiten ergeben sich mit wachsenden Stromdichten und die dadurch resultierende Besetzung eines Großteils oder aller Emittermoleküle Sättigungseffekte. In der Folge können weitere Ladungsträger-Ströme nicht mehr vollständig zur Besetzung der angeregten und emittierenden Zustände führen. Es resultieren dann nur unerwünschte ohmsche Verluste. Infolgedessen ergibt sich mit steigender Stromdichte ein deutlicher Effizienz-Abfall des OLED-Devices (sog. "Roll-Off" Verhalten). In ähnlich ungünstiger Weise wirken sich die Effekte der Triplett-Triplett-Annihilation und des Self-Quenchens aus (siehe dazu z. B. H. Yersin, "Highly Efficient OLEDs with Phosphorescent Materials", Wiley-VCH, Weinheim 2008 und S. R. Forrest et al., Phys. Rev. B 2008, 77, 235215). So zeigen sich insbesondere Nachteile bei der Verwendung derartiger Emitter für OLED-Beleuchtungen, bei denen eine hohe Leuchtdichte, z. B. von über 1000 $cd/m^2$ gefordert wird. (Vergleiche: J. Kido et al. Jap. J. Appl. Phys. 2007, 46, L10.) Darüber hinaus sind Moleküle in elektronisch angeregten Zuständen in der Regel chemisch reaktiver als Moleküle in Grundzuständen, so dass die Wahrscheinlichkeit unerwünschter chemischer Reaktionen mit der Länge der Emissionslebensdauer wächst. Durch derartige unerwünschte chemische Reaktionen wird dann die Device-Lebensdauer verringert.

Darüber hinaus erfolgen in der Regel in Cu(I)-Komplexen nach dem Anregungsprozess (durch Elektron-Loch-Rekombination oder durch optische Anregung) ausgeprägte Geometrie-Veränderungen, wodurch die Emissionsquantenausbeuten stark reduziert werden. Ferner werden durch diese Prozesse die Emissionsfarben in unerwünschter Weise rot-verschoben , siehe z.B. DE102007031261 A1.

Darüber hinaus sind viele der bekannten Kupfer-Komplexe in den für den technischen Einsatz geforderten Lösungsmitteln nicht löslich. Auch das spricht in der Regel gegen die Verwendung dieser Komplexe.

Es ist Ziel dieser Erfindung, neue Materialien auf der Basis von Kupfer(I)-Komplexen zu entwickeln, die die oben genannten Nachteile nicht aufweisen.

## Beschreibung der Erfindung

Überraschender Weise wird das Ziel der Erfindung durch die hier beschriebenen Cu(I)-Verbindungen erreicht. Das heißt, die Erfindung beinhaltet die Schaffung und Bereitstellung neuer Cu(I)-Verbindungen, die folgende Kombination von Eigenschaften zeigen:

- relativ kurze Emissionslebensdauer von nur wenigen μs,
- hohe Emissionsquantenausbeuten größer 40 %,
- weitgehende Verhinderung von unerwünschten Geometrie-Veränderungen und
- Löslichkeit in verschiedenen, den technologischen Anforderungen genügenden Lösungsmitteln.

Unter organischen Lösungsmitteln im Sinne der Erfindung sind zu verstehen

- Alkane, auch halogenierte Alkane wie Pentan, Hexan, Heptan, einschließlich verzweigter Alkane,
- Dichlormethan, Chloroform, 1,2-Dichlorethan, 1,1,1-Trichlorethan, Tetrachlorkohlenstoff, Perchlorethylen
- Aromatische Kohlenwasserstoffe, auch halogeniert: Benzol, Toluol, Chlorbenzol, 1,2-Dichlorbenzol
- Ether: Tetrahydrofuran, Diethylether
- Ketone: Aceton, Methylethylketon
- sowie: Acetonitril, Nitromethan, Dimethylsulfoxid, Dimethylformamid, Methanol, Ethanol und Essigsäureethylester.

In bevorzugten Ausgestaltungen der Erfindung ist der Kupfer(I)komplex insbesondere in mindestens einem

der folgenden Lösungsmittel gut löslich: polaren Kohlenwasserstoffe wie z. B. Dichlormethan, Chloroform, 1,2-Dichlorethan, 1,1,1-Trichlorethan, Perchlorethylen, Toluol, Chlorbenzol, 1,2-Dichlorbenzol, Tetrahydrofuran, Diethylether, Aceton, Methylethylketon, Nitromethan, Dimethylsulfoxid, Dimethylformamid, Methanol und Ethanol.

**Singulett-Harvesting**

Von besonderer Bedeutung ist es, das starke Übergangsverbot vom angeregten Triplett-Zustand $T_1$ zum Singulett-Zustand $S_0$ zu lockern und Emitter-Moleküle mit möglichst kurzer Emissionslebensdauer, aber dennoch hoher Emissionsquantenausbeute zu entwickeln. OLEDs unter Verwendung derartiger Emitter zeigen dann ein deutlich geringeres Roll-Off-Verhalten der Effizienz und ermöglichen darüber hinaus eine längere Lebensdauer der opto-elektronischen Vorrichtung.

Überraschenderweise lässt sich die oben beschriebene Problematik durch die vorliegende Erfindung lösen, indem Emitter-Moleküle zur Verwendung kommen, die bestimmte elektronische Strukturen bzw. Singulett-Triplett-Energieabstände aufweisen und die erfindungsgemäß durch den hier erstmals für Cu(I)-Komplexe vorgeschlagenen Singulett-Harvesting-Effekt zeigen. In Fig. 2a ist ein Energieniveauschema für Übergangsmetall-Komplexe mit kleiner oder sich nur in geringem Maße auswirkender Spin-Bahn-Kopplung (z. B. Metall-Komplexe der ersten Periode der Übergangsmetalle oder Metall-Komplexe mit liganden-zentrierten Triplett-Zuständen) schematisch dargestellt. Anhand dieses Schemas sollen die photophysikalischen Elektrolumineszenz-Eigenschaften dieser Moleküle erläutert werden. Die Loch-Elektron-Rekombination, wie sie beispielsweise in einem opto-elektronischen Bauelement erfolgt, führt im statistischen Mittel zu 25 % zur Besetzung des Singulett-Zustandes (1 Singulettpfad) und zu 75 % zur Besetzung des um $\Delta E_1(S_1\text{-}T_1)$ tiefer liegenden Triplett-Zustandes (3 Triplettpfade). Die in den $S_1$-Zustand gelangende Anregung relaxiert aufgrund des Inter-System-Crossing (ISC)-Prozesses, der bei übergangsmetall-organischen Komplexen in der Regel schneller als in $10^{-12}$ s erfolgt, in den $T_1$-Zustand. Die radiative Emissionslebensdauer des Triplett-Zustandes ist für diese Metall-Komplexe der ersten Periode der Übergangsmetalle sehr lang (z. B. 100 µs bis 1000 µs oder länger). Emitter mit derart langen Abklingzeiten sind kaum für OLED-Anwendungen geeignet.

Erfindungsgemäß lässt sich der Nachteil des oben beschriebenen Standes der Technik vermeiden, indem Cu(I)-Komplexe gewählt werden, die einen $\Delta E_2(S_1\text{-}T_1)$-Wert zwischen dem untersten angeregten Singulett ($S_1$)- und dem darunter liegenden Triplett ($T_1$)-Zustand von kleiner als 2500 $cm^{-1}$ aufweisen. Das ist durch das in Fig. 2b gezeigte Energieniveaudiagramm für Cu(I)-Komplexe veranschaulicht. Diese Energiedifferenz ist klein genug, um eine thermische Rückbesetzung des $S_1$-Zustandes aus dem $T_1$-Zustand gemäß einer Boltzmann-Verteilung bzw. gemäß der thermischen Energie $k_BT$ zu ermöglichen. Damit kann eine thermisch aktivierte Lichtemission aus dem $S_1$-Zustand erfolgen. Dieser Vorgang läuft gemäß Gleichung (1) ab

$$\mathrm{Int}(S_1 \rightarrow S_0) / \mathrm{Int}(T_1 \rightarrow S_0) = k(S_1) / k(T_1) \exp(-\Delta E(S_1\text{-}T_1)/k_BT) \qquad (1)$$

Hierin stellt $\mathrm{Int}(S_1 \rightarrow S_0) / \mathrm{Int}(T_1 \rightarrow S_0)$ das Intensitätverhältnis der Emission aus dem $S_1$-Zustand und aus dem $T_1$-Zustand dar, $k_B$ ist die Boltzmann-Konstante und T die absolute Temperatur. $k(S_1) / k(T_1)$ ist das Ratenverhältnis der entsprechenden Übergangsprozesse in den elektronischen Grundzustand $S_0$. Für Cu(I)-Komplexe liegt dieses Verhältnis zwischen $10^2$ bis $10^4$. Erfindungsgemäß besonders bevorzugt sind Moleküle mit einem Ratenverhältnis von $10^3$ bis $10^4$. $\Delta E(S_1\text{-}T_1)$ steht für die Energiedifferenz $\Delta E_2(S_1\text{-}T_1)$ gemäß Fig. 2b.

Durch den beschriebenen Prozess der thermischen Rückbesetzung wird aus dem besetzten Triplett ein Emissionskanal über den Singulett-Zustand $S_1$ geöffnet. Da der Übergang aus dem $S_1$- in den $S_0$- Zustand stark erlaubt ist, wird die Triplett-Anregungsenergie praktisch vollständig als Lichtemission über den Singulett-Zustand gewonnen. Dieser Effekt ist umso ausgeprägter, je kleiner die Energiedifferenz $DE(S_1\text{-}T_1)$ ist. Daher sind Cu(I)-Komplexe bevorzugt, die einen $\Delta E(S_1\text{-}T_1)$-Wert zwischen dem untersten angeregten Singulett- und dem darunter liegenden Triplett-Zustand von kleiner als 1500 $cm^{-1}$, bevorzugt von kleiner als 1000 $cm^{-1}$ und besonders bevorzugt von kleiner als 500 $cm^{-1}$ aufweisen.

Anhand eines Zahlenbeispiels soll dieser Effekt erläutert werden. Bei einer typischen Energiedifferenz von $\Delta E(S_1\text{-}T_1)$ = 800 $cm^{-1}$ ergibt sich für Raumtemperaturanwendungen (T = 300 K) mit $k_BT$ = 210 $cm^{-1}$ und einem Ratenverhältnis von $10^3$ ein Intensitätsverhältnis gemäß Gleichung (1) von ca. 20. Das heißt, der Singulett-Emissionsprozess dominiert für ein Molekül mit diesen Beispielwerten sehr stark.

Die Emissionslebensdauer dieses Beispiel-Moleküls verändert sich auch deutlich. Durch die thermische Rückbesetzung ergibt sich eine mittlere Lebensdauer $\tau_{av}$. Diese lässt sich näherungsweise gemäß Gleichung (2) beschreiben

$$\tau_{av} \approx \tau(S_1) \cdot \exp\,(\Delta E(S_1\text{-}T_1) / k_BT) \qquad (2)$$

Hierin ist $\tau(S_1)$ die Fluoreszenzlebensdauer ohne Rückbesetzung und $\tau_{av}$ die Emissionslebensdauer, die bei Öffnung des Rückbesetzungskanals durch die beiden Zustände $T_1$ und $S_1$ bestimmt wird (Siehe Fig. 2b). Die anderen Größen wurden oben definiert.

Gleichung (2) soll wieder durch ein Zahlenbeispiel erläutert werden. Für die angenommene Energiedifferenz von $DE(S_1-T_1) = 800\ cm^{-1}$ und einer Abklingzeit des fluoreszierenden $S_1$ - Zustandes von 50 ns ergibt sich eine Emissionsabklingzeit (der beiden Zustände) von $\tau_{av} \approx 2\ \mu s$. Diese Abklingzeit ist kürzer, als die der meisten sehr guten Ir(III)- oder Pt(II)-Triplett-Emitter.

Zusammenfassend lassen sich also unter Verwendung dieses Singulett-Harvesting-Verfahrens für Cu(I)-Komplexe im Idealfall nahezu sämtliche, d. h. maximal 100 % der Exzitonen erfassen und über eine Singulett-Emission in Licht umwandeln. Darüber hinaus gelingt es, die Emissionsabklingzeit drastisch unter den Wert der reinen Triplett-Emission von Cu(I)-Komplexen, die in der Regel bei einigen hundert $\mu$s bis ms liegt, zu verkürzen. Daher ist die erfindungsgemäße Verwendung entsprechender Komplexe für opto-elektronische Bauelemente besonders geeignet.

Die erfindungsgemäßen Cu(I)-Komplexe mit den oben beschriebenen Eigenschaften, d. h. u. a. mit kleiner Singulett-Triplett-Energiedifferenz $\Delta E(S_1-T_1)$, sind bevorzugt mit der unten angegebenen generellen Formel A zu beschreiben. Die elektronischen Übergänge, die das optische Verhalten dieser Cu(I)-Komplexe steuern, weisen einen ausgeprägten Metall-zu-Ligand-Charge-Transfer Charakter auf. Mit diesem Übergangstyp ist ein relativ kleiner Wert des - dem Fachmann bekannten - quantenmechanischen Austauschintegrals verbunden. Damit resultiert dann die gewünschte kleine Energiedifferenz $\Delta E(S_1-T_1)$.

Die Erfindung betrifft in einem weiteren Aspekt ein Verfahren zur Auswahl von Cu(I)-Komplexen, deren $\Delta E(S_1-T_1)$-Wert zwischen dem untersten angeregten Singulett- ($S_1$) und dem darunter liegenden Triplett-Zustand ($T_1$) kleiner als 2500 $cm^{-1}$, bevorzugt kleiner als 1500 $cm^{-1}$, besonders bevorzugt kleiner als 1000 $cm^{-1}$, ganz besonders bevorzugt kleiner 500 $cm^{-1}$ ist.

Die Bestimmung des $\Delta E(S_1-T_1)$-Wertes kann sowohl durch quantenmechanische Berechnungen mittels im Stand der Technik bekannten Computerprogrammen (z. B. mittels Turbomole-Programmen unter Ausführung von TDDFT- und unter Berücksichtigung von CC2-Rechnungen) oder - wie weiter unten erläutert wird - experimentell durchgeführt werden.

Die Energiedifferenz $\Delta E(S_1-T_1)$, insbesondere der durch die Formel A beschriebenen Komplexe, lässt sich näherungsweise quantenmechanisch durch das mit dem Faktor 2 multiplizierte sogenannte Austauschintegral beschreiben. Dessen Wert hängt direkt ab von der Ausgeprägtheit des sogenannten Charge-Transfer-Charakters unter Beteiligung der Metall-d-Orbitale und der Liganden-$\pi^*$-Orbitale. Das heißt, ein elektronischer Übergang zwischen den verschiedenen Orbitalen repräsentiert einen Metall-zu-Ligand-Charge-Transfer (CT)-Übergang. Je geringer die Überlappung der oben beschriebenen Molekülorbitale ist, desto ausgeprägter ist der elektronische Charge-Transfer Charakter. Das ist dann mit einer Abnahme des Austausch-Integrals und somit einer Abnahme der Energiedifferenz $\Delta E(S_1-T_1)$ verbunden. Aufgrund dieser photophysikalischen (quantenmechanischen) Eigenschaften lassen sich die erfindungsgemäßen Energiedifferenzen mit $\Delta E(S_1-T_1)$ kleiner 2500 $cm^{-1}$ oder kleiner 1500 $cm^{-1}$ oder kleiner 1000 $cm^{-1}$oder sogar kleiner 500 $cm^{-1}$ erreichen.

Die Bestimmung des $\Delta E(S_1-T_1)$-Wertes kann experimentell folgendermaßen erfolgen:

Für einen vorgegebenen Cu(I)-Komplex lässt sich der Energieabstand $\Delta E(S_1-T_1)$ unter Verwendung der oben angegebenen Gleichung (1) einfach bestimmen. Eine Umformung ergibt:

$$\ln\{Int(S_1 \rightarrow S_0)/Int(T_1 \rightarrow S_0)\} = \ln\{k(S_1)/k(T_1)\} - (\Delta E(S_1-T_1)/k_B)(1/T) \quad (3)$$

Für die Messung der Intensitäten $Int(S_1 \rightarrow S_0)$ und $Int(T_1 \rightarrow S_0)$ kann jedes handelsübliche Spektralphotometer verwendet werden. Eine graphische Auftragung der bei verschiedenen Temperaturen gemessenen (logarithmierten) Intensitätsverhältnisse $\ln\{Int(S_1 \rightarrow S_0)/Int(T_1 \rightarrow S_0)\}$ gegen den Kehrwert der absoluten Temperatur T ergibt in der Regel eine Gerade. Die Messung wird in einem Temperaturbereich von Raumtemperatur (300 K) bis 77 K oder bis 4,2 K durchgeführt, wobei die Temperatur mittels eines Kryostaten eingestellt wird. Die Intensitäten werden aus den (korrigierten) Spektren bestimmt, wobei $Int(S_1 \rightarrow S_0)$ bzw. $Int(T_1 \rightarrow S_0)$ die integrierten Fluoreszenz- bzw. Phosphoreszenz-Bandenintensitäten repräsentieren, welche sich mittels der zum Spektralphotometer gehörenden Programme bestimmen lassen. Die jeweiligen Übergänge (Bandenintensitäten) lassen sich leicht identifizieren, da die Triplett-Bande bei niedrigerer Energie liegt als die Singulett-Bande und mit sinkender Temperatur an Intensität gewinnt. Dabei werden die Messungen in sauerstofffreien verdünnten Lösungen (ca. $10^{-2}$ mol $L^{-1}$) oder an dünnen Filmen aus den entsprechenden Molekülen oder an mit den entsprechenden Molekülen dotierten Filmen durchgeführt. Verwendet man als Probe eine Lösung, so empfiehlt es sich, ein Lösemittel bzw. Lösemittelgemisch zu verwenden, das bei tiefen Temperaturen Gläser bildet, wie 2-Methyltetrahydrofuran, Butyronitril, Toluol, Ethanol oder aliphatische Kohlenwasserstoffe. Verwendet man

als Probe einen Film, so eignet sich die Verwendung einer Matrix mit einer deutlich größeren Singulett- sowie Triplett-Energie als die der Cu(I)-Komplexe (Emittermoleküle), z. B. PMMA (Polymethylmethacrylat). Dieser Film kann aus Lösung aufgebracht werden.

Die Geradensteigung beträgt $-\Delta E(S_1-T_1)/k_B$. Mit $k_B = 1{,}380\ 10^{-23}\ JK^{-1} = 0{,}695\ cm^{-1}\ K^{-1}$ lässt sich der Energieabstand direkt bestimmen.

Eine einfache, näherungsweise Abschätzung des $\Delta E(S_1-T_1)$-Wertes kann auch dadurch vorgenommen werden, dass bei tiefer Temperatur (z. B. 77 K oder 4.2 K unter Verwendung eines Kryostaten) die Fluoreszenz- und Phosphoreszenz-Spektren registriert werden. Der $\Delta E(S_1-T_1)$-Wert entspricht dann in Näherung der Energiedifferenz zwischen den hoch-energetischen Anstiegsflanken der Fluoreszenz- bzw. Phosphoreszenz-Bande.

Ein anderes Bestimmungsverfahren für den $\Delta E(S_1-T_1)$-Wert ist durch Messung der Emissionsabklingzeiten mit einem handelsüblichen Messgerät gegeben. Hierbei wird die Emissionslebensdauer $\tau_{av}$ als Funktion der Temperatur mit Hilfe eines Kryostaten für den Bereich zwischen 4,2 K oder z. B. 20 K und 300 K gemessen. Unter Verwendung der Formel (4) und der bei tiefer Temperatur gemessenen Emissionslebensdauer für den Triplett-Zustand $\tau(T_1)$ lässt sich ein Fit der Messwerte mit der Formel (4) durchführen, und man erhält den $\Delta E(S_1-T_1)$-Wert. (Hinweis: Der $\tau(T_1)$-Wert ist häufig durch das sich bei der Auftragung der Messwerte ergebene Plateau bestimmt. Falls sich eine Ausbildung eines derartigen Plateaus zeigt, ist in der Regel eine Kühlung auf 4,2 K nicht mehr erforderlich. Ein Beispiel ist in Figur 8 wiedergegeben.)

$$\tau_{av} = \frac{1 + \exp\left(-\frac{\Delta E(S_1 - T_1)}{k_B T}\right)}{\frac{1}{\tau(T_1)} + \frac{1}{\tau(S_1)}\exp\left(-\frac{\Delta E(S_1 - T_1)}{k_B T}\right)} \qquad (4)$$

Je ausgeprägter der CT-Charakter eines organischen Moleküls ist, desto stärker verändern sich die elektronischen Übergangsenergien als Funktion der Lösungsmittelpolarität. So gibt bereits eine ausgeprägte Polaritätsabhängigkeit der Emissionsenergien einen Hinweis auf das Vorliegen kleiner $\Delta E(S_1-T_1)$-Werte.

## Stabilisierung der Molekularstruktur

Vierfach koordinierte Cu(I) Komplexe weisen im elektronischen Grundzustand eine annähernd tetraedrische Koordination des Metallatoms auf. Bei Anregung in einen elektronischen angeregten Zustand mit ausgeprägtem Metall-zu-Ligand-Charge-Transfer Charakter und der damit verbundenen formalen Oxidierung des Metallatoms zu Cu(II) kann es zu wesentlichen Veränderungen der Geometrie des Komplexes in Richtung einer quadratisch-planaren Koordination kommen, was daher als "Planarisierung" des Komplex-Moleküls bezeichnet werden kann. Dieser Prozess liefert einen sehr effektiven Mechanismus für das Löschen (Quenchen) der Lumineszenz.

In den erfindungsgemäßen Kupfer(I)komplexen wird dieser Löschmechanismus durch Anwesenheit sterisch anspruchvoller Substituenten am Diiminliganden NnN (insbesondere in den Positionen 2 und 9 von 1,10-Phenanthrolin oder in Positionen 3 und 3' von 2,2'-Bipyridin) verhindert oder sehr reduziert durch eine Verhinderung der Geometrie-Veränderungen um das Cu-Atom. Gleichzeitig tragen derartige Substitutionen zum Schutz des Cu-Zentrums vor unerwünschten chemischen Reaktionen mit nukleophilen Substanzen (Lösungsmitteln, Verunreinigungen, leicht koordinierenden Matrixmaterialien) bei. Bereits eine Methylgruppe führt zu einer merklichen "Versteifung" der resultierenden Cu-Komplexe. Ein sterisch anspruchsvoller Substituent ist daher neben Methyl insbesondere ein Alkylrest $-(CH_2)_n-CH_3$ (n = 0 - 20) (auch verzweigt), ein Arylrest mit 6 - 20 Kohlenstoffatomen (z. B. - Ph), Alkoxyrest $-O-(CH_2)_n-CH_3$ (n = 0 - 20), ein Aryloxyrest (z. B. -OPh) oder ein Silanrest (z. B $-SiMe_3$). Die Alkyl- und Arylreste können auch substituiert sein (z. B. mit Halogenen, Alkoxy- oder Silan-Gruppen, usw.) oder zu annelierten Ringsystemen führen (siehe Beispiel 2).

## Chemische Leitstruktur

Der zu schützende Emitter A soll folgende Merkmale umfassen:

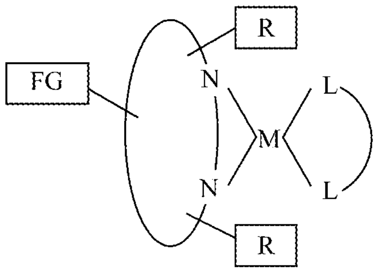

Formel A

- M ist Cu(I).
- Bei LnL handelt es sich um einen einfach negativ geladenen, zweizähnigen Liganden.
- Der Ligand NnN ist ein substituierter Diiminligand, insbesondere substituierte 2,2'-Bipyridin-Derivate (bpy) oder 1,10-Phenanthrolin-Derivate (phen).
- R ist ein sterisch anspruchsvoller Substituent in 3,3'-Position (bpy) oder 2,9-Position (phen), der eine Geometrieveränderung in Richtung einer Planarisierung des Komplexes im angeregten Zustand verhindert. Ein sterisch anspruchsvoller Substituent ist insbesondere ein Alkylrest $-(CH_2)_n-CH_3$ (n = 0 - 20) (auch verzweigt), ein Arylrest mit 6 - 20 Kohlenstoffatomen (z. B. -Ph), Alkoxyrest $-O-(CH_2)_n-CH_3$ (n = 0 - 20), ein Aryloxyrest (z. B. -OPh) oder ein Silanrest (z. B $-SiMe_3$). Die Alkyl- und Arylreste können auch substituiert sein (z. B. mit Halogenen, Alkoxy- oder Silan-Gruppen, usw.) oder zu annelierten Ringsystemen führen. Obwohl in der Formel A zwei Reste A dargestellt sind, kann ein erfindungsgemäßer Komplex in einer Ausführungsform der Erfindung auch nur einen Rest R aufweisen.
- Bei FG = Funktions-Gruppe handelt es sich um einen weiteren Substituenten, der eine zusätzliche Funktion in den Komplex einbringt, den dieser ansonsten nicht aufweisen würde. Die Funktions-Gruppen FG werden entweder direkt oder über geeignete Brücken (siehe unten) an die Diimin-Substituenten angebracht.

  - Dabei kann es sich entweder um eine Gruppe handeln, die Eigenschaften eines Elektronenleiters besitzt.
  - Es kann sich um eine Gruppe handeln, die Eigenschaften eines Lochleiters aufweist.
  - Es kann sich um einen Gruppe handeln, die die Löslichkeit des Komplexes bestimmt.

**Diimin-Ligand**

Bei dem Diiminliganden handelt es sich bevorzugt entweder um einen substituierten 2,2'-Bipyridin- oder einen substituierten 1,10-Phenanthrolin-Liganden. Die Synthesen unterschiedlicher, substituierter bpy- und phen-Liganden wurde bereits mehrfach in wissenschaftlichen Fachartikeln besprochen (G. Chelucci, R. P. Thummel, Chem. Rev. 2002, 102, 3129; C. Kaes, A. Katz, M. W. Hosseini, Chem. Rev. 2000, 100, 3553; M. Schmittel, H. Ammon, Eur. J. Inorg. Chem. 1998, 785. M. Heller, U. S. Schubert J. Org. Chem. 2002, 67, 8269.) und sind daher dem Fachmann bekannt.

**Substituierte 2,2'-Bipyridin-Liganden**

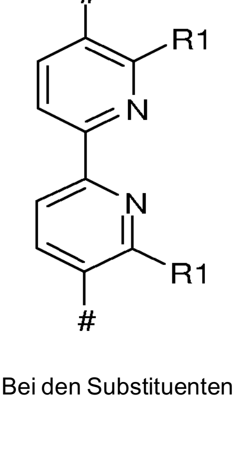 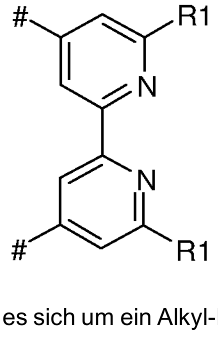 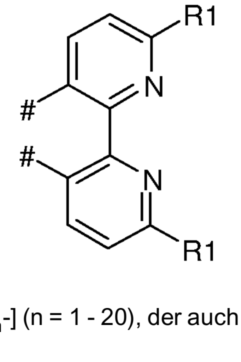

Bei den Substituenten R1 kann es sich es sich um ein Alkyl-Rest $[CH_3-(CH_2)_n-]$ (n = 1 - 20), der auch verzweigt

oder mit Halogenen (F, Cl, Br, I) substituiert sein kann oder um einen Aryl-Rest (insbesondere Phenyl) handeln, der mit Alkyl-Gruppen, Halogenen (F, Cl, Br, I), Silan- (-$SiR'_3$) oder Ethergruppen -OR' (R' definiert wie R1) substituiert sein kann.

An den mit "#" gekennzeichneten Positionen ist der Diimin-Ligand mit einer Funktions-Gruppe FG substituiert. Ebenso findet die Anbindung der unten angeführten Funktionsgruppen an der dort ebenfalls mit "#" gekennzeichneten Position statt (s. u.). Die Bindung zwischen der Funktions-Gruppe und dem Diiminliganden kann einerseits direkt an den mit "#" gekennzeichneten Positionen stattfinden. So bilden sich direkte $C_{FG}$-$C_{NN}$ Bindungen, wobei $C_{NN}$ das C-Atom des Diiminliganden darstellt, das mit "#" markiert ist, und $C_{FG}$ das mit "#" gekennzeichnet C-Atom der Funktions-Gruppe. Ist das mit "#" gekennzeichnete Atom ein Stickstoffatom, so ergeben sich $N_{FG}$-$C_{NN}$ Bindungen, wobei $N_{FG}$ für das mit "#" markierte Stickstoffatom steht. Andererseits kann die Funktions-Gruppe über eine Brücke an den Diiminliganden angeknüpft werden, wobei sich z. B. Ether-, Thioether-, Ester-, Amid-, Methylen-, Silan-, Ethylen-, Ethin-Brücken anbieten. Damit können sich als Brücken beispielsweise folgende Funktionen ergeben: $C_{FG}$-O-$C_{NN}$, $C_{FG}$-S-$C_{NN}$, -$C_{FG}$-C(O)-O-$C_{NN}$-, $C_{FG}$-C(O)-NH-$C_{NN}$-, $C_{PG}$-$CH_2$-$C_{NN}$, $C_{PG}$-$SiR'_2$-$C_{NN}$, $C_{FG}$-CH=CH-$C_{NN}$, $C_{FG}$-C≡C-$C_{NN}$. $N_{FG}$-$CH_2$-$C_{NN}$.

Die Verfahren zur Verknüpfung der Funktionsgruppe an den Diiminliganden, entweder direkt oder über eine Brücke, sind dem Fachmann bekannt (Suzuki-, Still-, Heck-, Sonogashira-, Kumuda-, Ullmann-, Buchwald-Hartwig-Kupplung sowie deren Varianten; (Thio)Veretherung, Veresterung, nucleophile und elektrophile Substitutionen am $sp^3$-Kohlenstoff oder Aromaten, usw.). Beispielsweise illustriert der in der Literatur beschriebene Ligand (4,4'-Bis(5-(hexylthio)-2,2'-bithien-5'-yl)-2,2'-bipyridine) die Möglichkeit einer Anbindung eines elektronenleitenden Substituenten an einen bpy Liganden mittels einer Stille Kupplung (C.-Y. Chen, M. Wang, J.-Y. Li, N. Pootrakulchote, L. Alibabaei, C.-h. Ngoc-le, J.-D. Decoppet, J.-H. Tsai, C. Grätzel, C.-G. Wu, S. M. Zakeeruddin, M. Grätzel, ACS Nano 2009, 3, 3103).

Eine weitere Möglichkeit zur Synthese substituierter bpy besteht in der Kupplung zweier, bereits mit dem Rest R sowie der Funktions-Gruppe FG substituierter Pyridine. Die sich hieraus ergebende bpy-Liganden können daher auch asymmetrisch substituiert sein. Die Verfahren und relevante Literatur sind im Übersichtsartikel von G. Chelucci (G. Chelucci, R. P. Thummel, Chem. Rev. 2002, 102, 3129) zusammengefasst.

In einer besonderen Ausführungsform kann der Rest R1 auch ein elektronenleitender, lochleitender oder die Löslichkeit erhöhender Substituent sein. Das führt zu folgenden Diiminliganden:

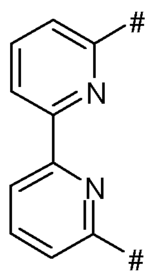

**Substituierte 1,10-Phenanthrolin-Liganden**

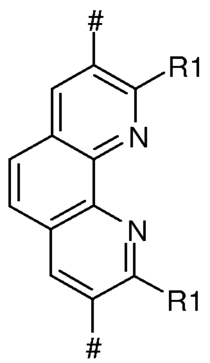

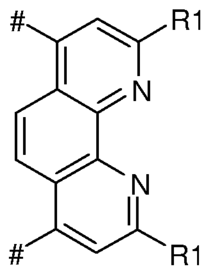

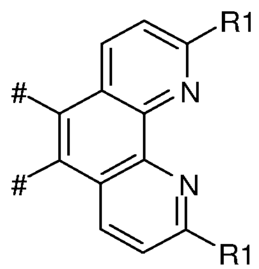

Bei den Substituenten R1 handelt es sich um ein Alkyl-Rest [$CH_3$-$(CH_2)_n$-] (n = 1 - 20), der auch verzweigt oder mit Halogenen (F, Cl, Br, I) substituiert sein kann oder um einen Aryl-Rest (insbesondere Phenyl), der mit Alkyl-Gruppen, Halogenen (F, Cl, Br, I), Silan- (-$SiR'_3$) oder Ethergruppen -OR' (R' definiert wie R1) substituiert sein kann.

Für die Verknüpfung der Funktions-Gruppe an den Phenanthrolin-Liganden stehen wieder die identischen

Verfahren zur Verfügung, die bereits bei den bpy-Liganden ausgeführt wurden: An den mit "#" gekennzeichneten Positionen ist der Diiminligand mit einer der weiter unten definierten Funktionsgruppe substituiert. Hierbei kann die Funktions-Gruppe FG entweder direkt über $C_{FG}$-$C_{NN}$ oder $N_{FG}$-$C_{NN}$ Bindungen (wobei $C_{FG}$ = C-Atom der Funktions-Gruppe, $N_{FG}$ = N-Atom der Funktions-Gruppe, $C_{NN}$ = C-Atom des Diiminliganden) an dem Diiminliganden gebunden sein, oder über eine Brücke verknüpft werden, z. B. $C_{FG}$-O-$C_{NN}$, $C_{FG}$-S-$C_{NN}$, -$C_{FG}$-C(O)-O-$C_{NN}$-, $C_{FG}$-C(O)-NH-$C_{NN}$-, $C_{FG}$-$CH_2$-$C_{NN}$, $C_{FG}$-SiR'$_2$-$C_{NN}$, $C_{FG}$-CH=CH-$C_{NN}$, $C_{FG}$-C=C-$C_{NN}$, $N_{FG}$-$CH_2$-$C_{NN}$. Die Verfahren zur Verknüpfung der Funktionsgruppe an den Diiminliganden, entweder direkt oder über eine Brücke, sind dem Fachmann bekannt. Die Beispiele 1 - 9 veranschaulichen exemplarisch die Möglichkeiten zur Synthese der substituierten Phen-Liganden.

In einer besonderen Ausführungsform kann der Rest R1 auch ein elektronenleitender, lochleitender oder die Löslichkeit erhöhender Substituent sein. Das führt zu folgenden Diiminliganden:

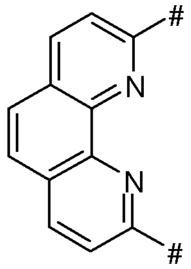

**Andere Diiminliganden**

Der Diiminligand kann auch ausgewählt sein aus folgenden Molekülen:

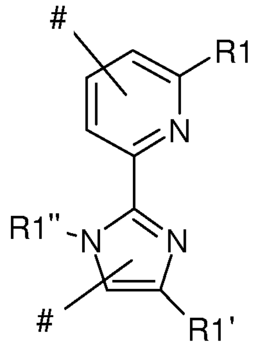
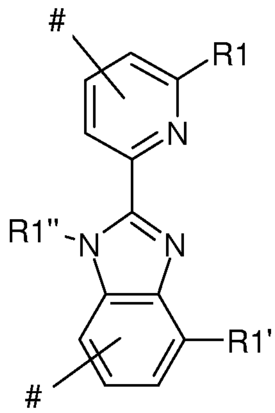
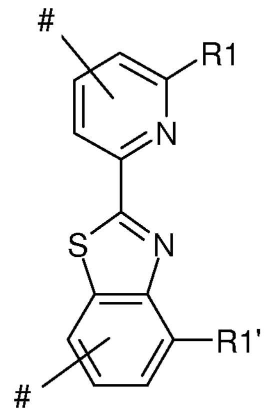
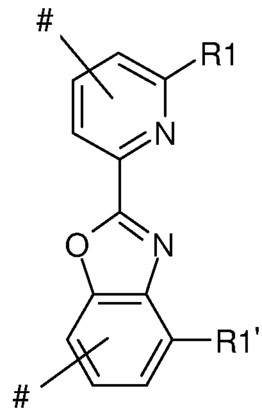
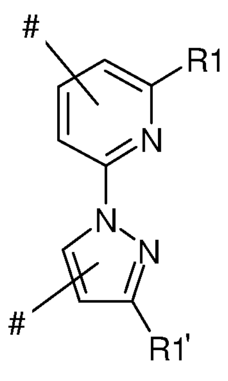
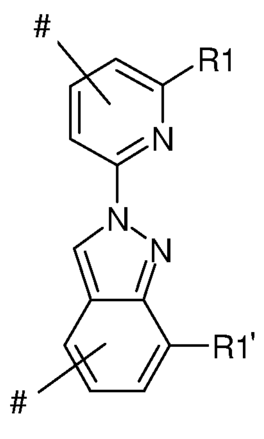
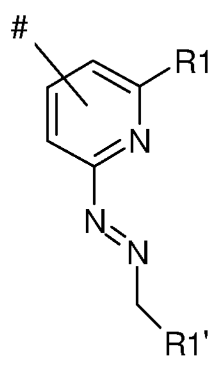
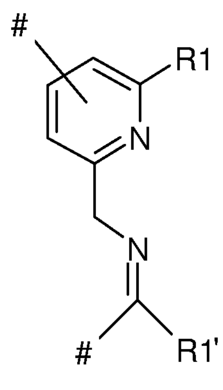

wobei es sich bei den Substituenten R1, R1' und R1" um ein Alkyl-Rest [$CH_3$-$(CH_2)_n$-] (n = 1 - 20), der auch verzweigt oder mit Halogenen (F, Cl, Br, I) substituiert sein kann, oder um einen Aryl-Rest (insbesondere Phenyl), der mit Alkyl-Gruppen, Halogenen (F, Cl, Br, I), Silan- (-SiR'$_3$) oder Ethergruppen -OR' (R' definiert wie R1, R1' oder R1") substituiert

sein kann, handelt. Substituenten # sind wie oben (bpy und phen Liganden) definiert.

**Definition des L∩L Liganden**

Bei dem einfach negativ geladenen Liganden LnL kann es sich um eines der unten dargestellten Moleküle handeln:

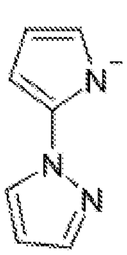 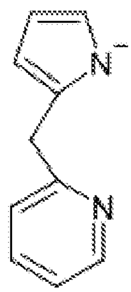 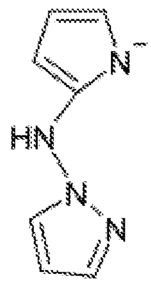 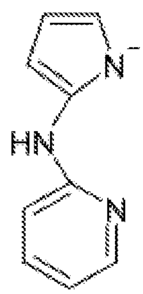 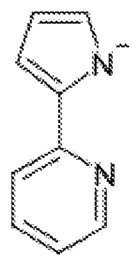

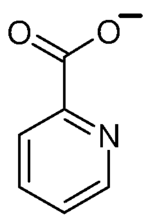 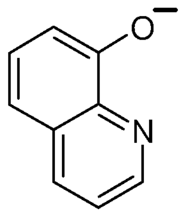 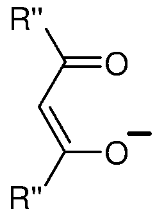 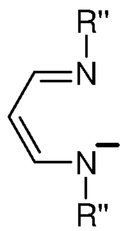

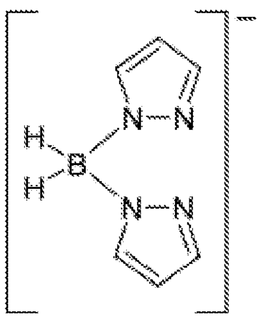 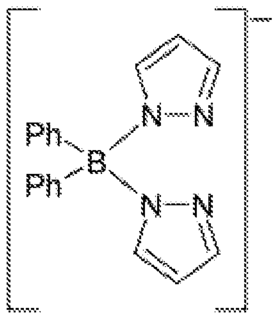 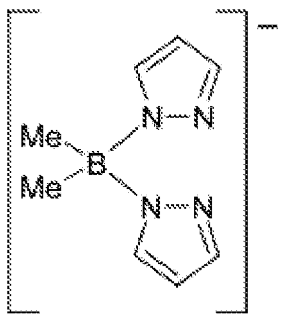 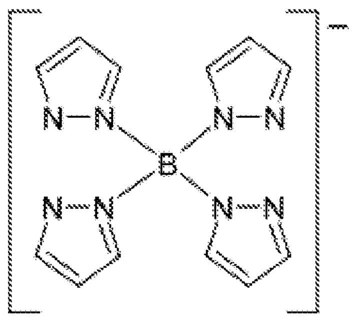

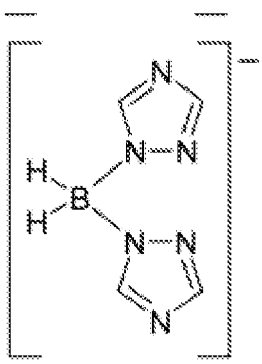 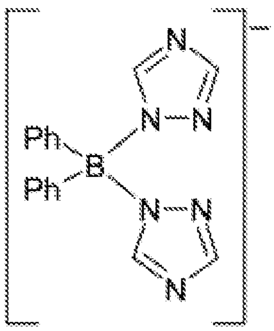 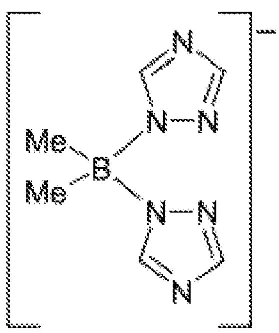 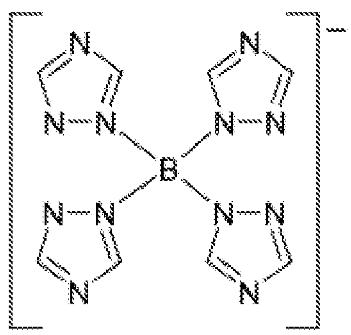

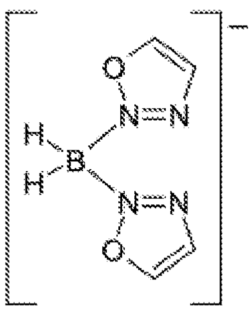 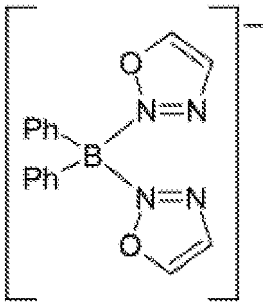 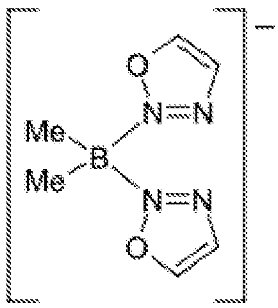 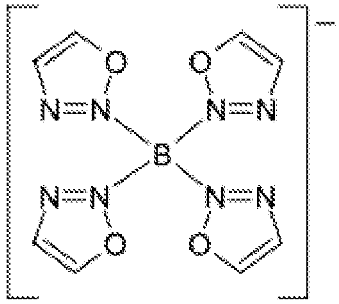

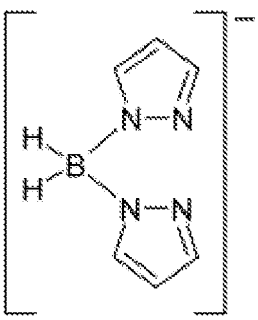 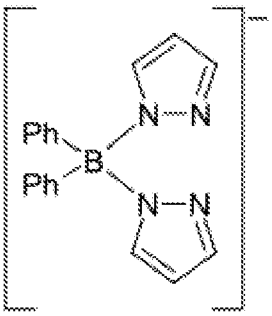 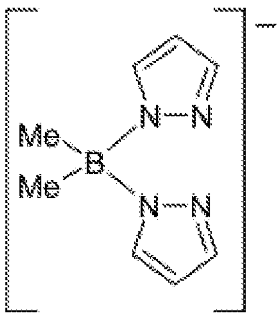 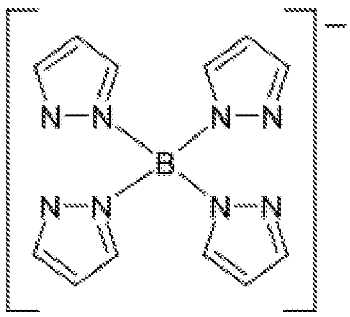

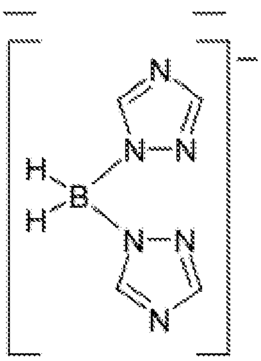 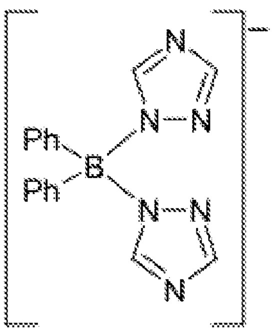 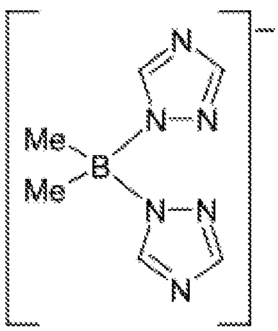 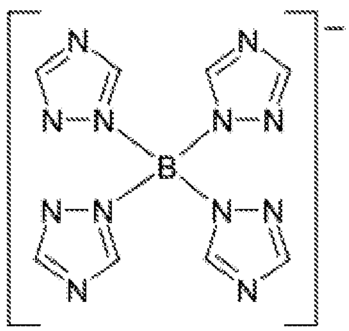

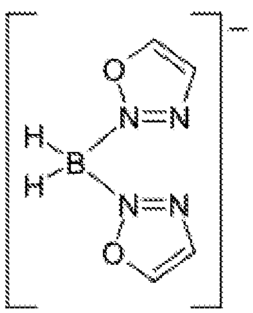 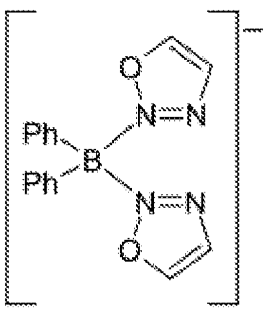 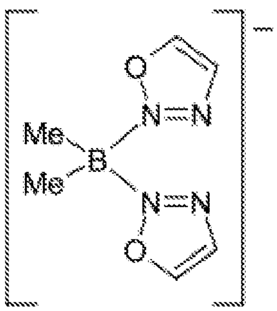 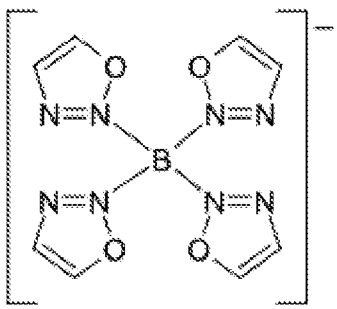

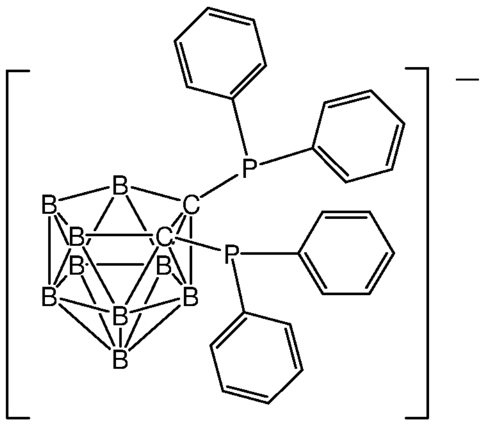

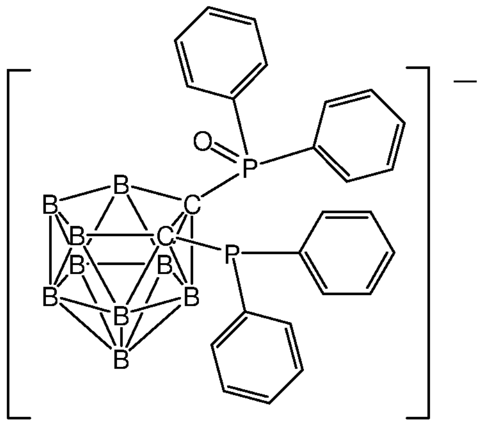

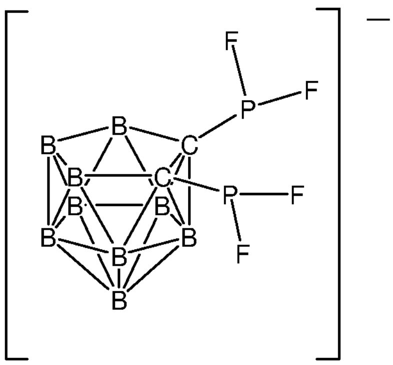

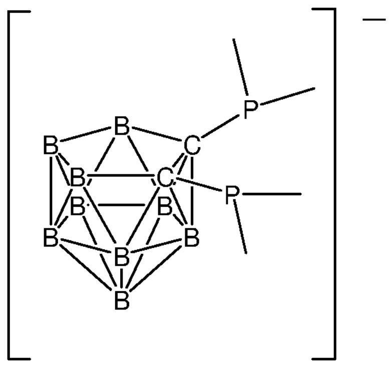

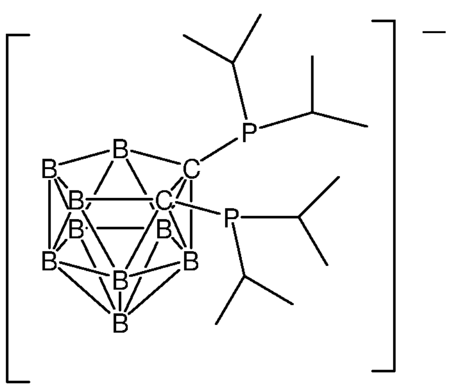

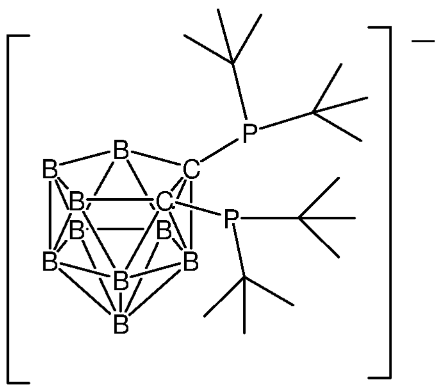

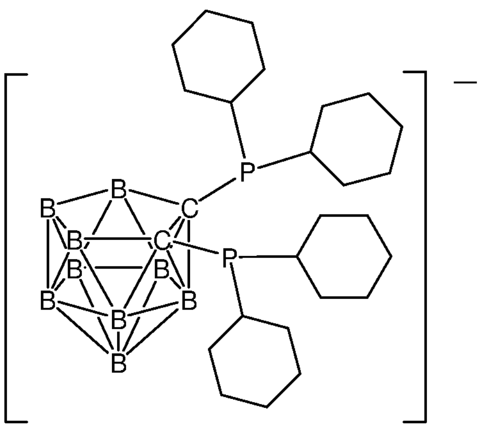

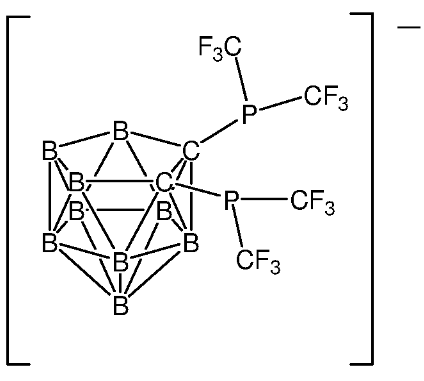

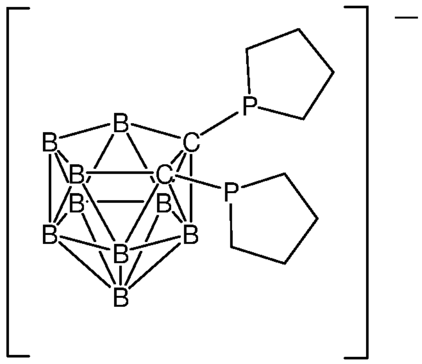
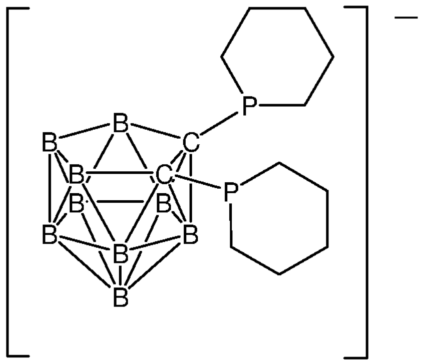
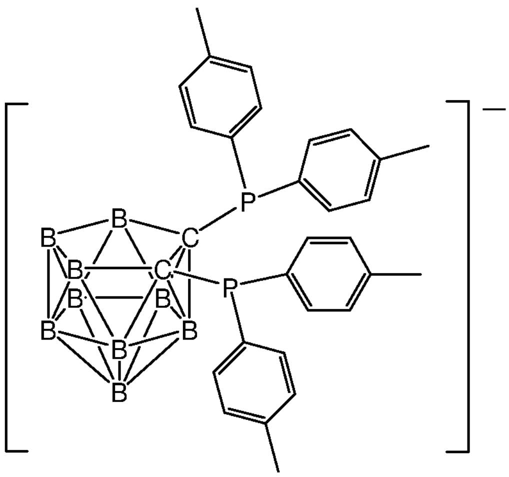
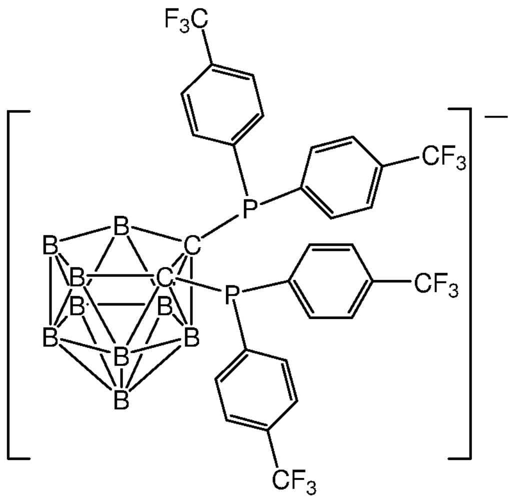
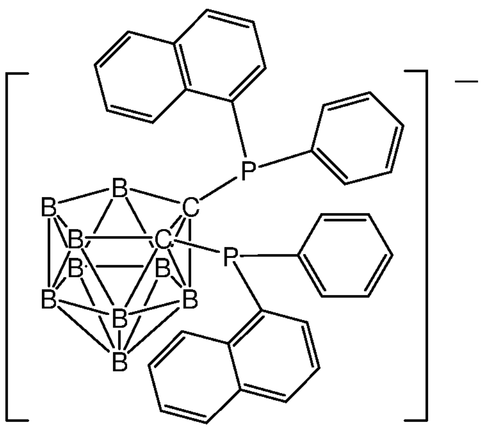
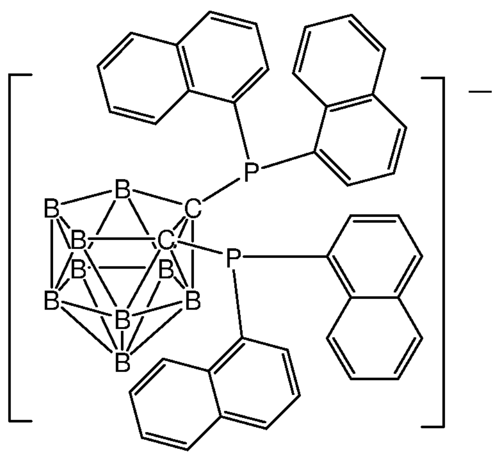

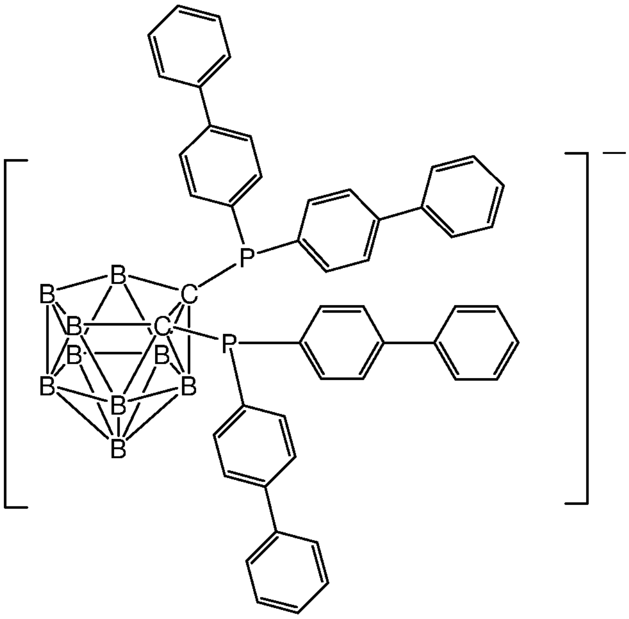

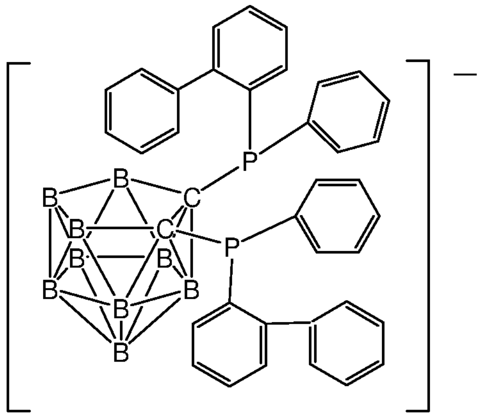

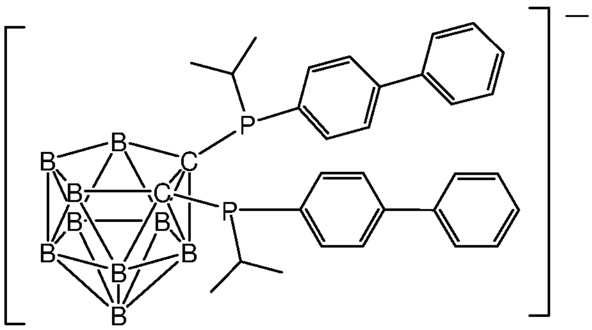

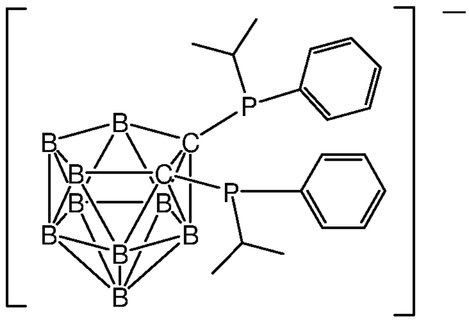

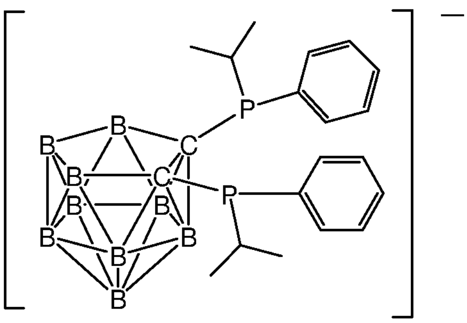

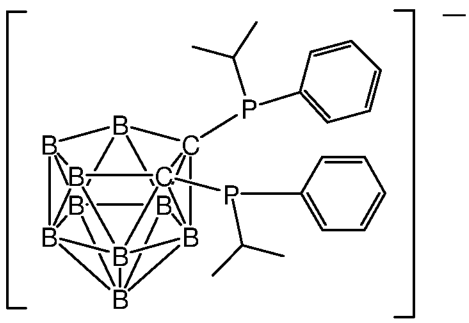

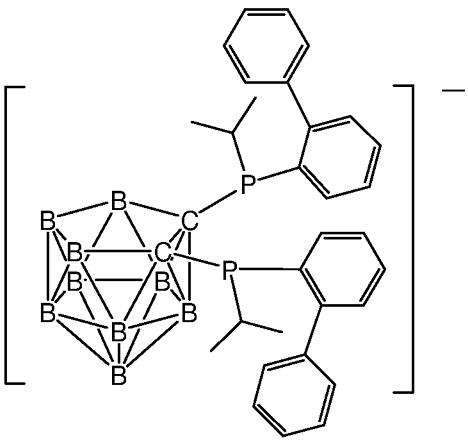

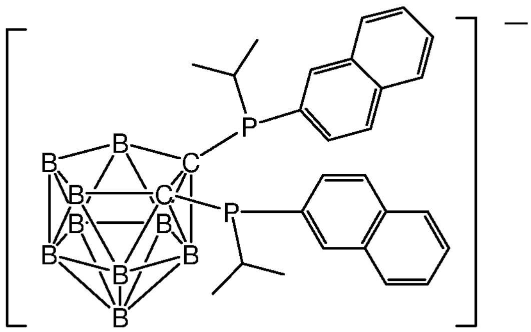

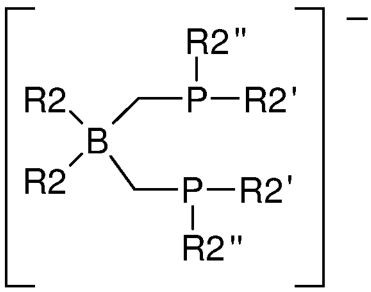

pop = $[P_2O_5H_2]^{2-}$

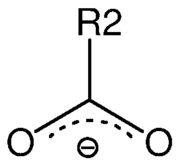

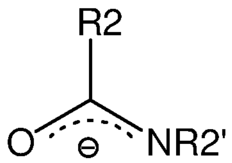

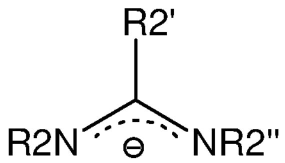

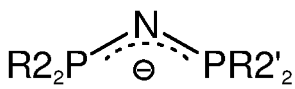

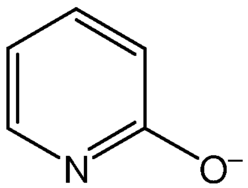

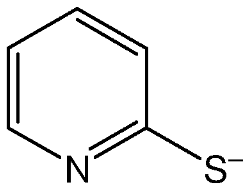

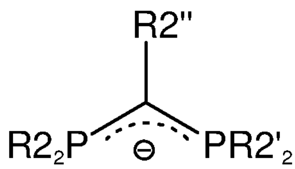

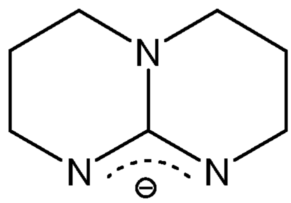

Bei den Substituenten R2, R2' und R2" handelt es sich um einen Alkyl-Rest $[CH_3-(CH_2)_n-]$ (n = 0 - 20), der auch verzweigt oder mit Halogenen (F, Cl, Br, I) substituiert sein kann, oder um einen Aryl-Rest (insbesondere Phenyl), der mit Alkyl-Gruppen, Halogenen (F, Cl, Br, I), Silan- ($-SiR'''_3$) oder Ethergruppen -OR"' (R"' definiert wie R2, R2' oder R2") substituiert sein kann.

**Definition der Funktions-Gruppen FG:**

Die Funktions-Gruppen (FG) können entweder einfach oder mehrfach am NnN Liganden gebunden sein. Es können identische oder unterschiedliche Funktions-Gruppen verwendet werden. Die Funktions-Gruppen können auch symmetrisch oder unsymmetrisch vorhanden sein. Meist ist aus synthetischen Gründen eine Zweifachsubstitution identischer Funktions-Gruppen vorteilhaft.

**Elektronenleiter**

Da es sich bei den Elektronenleiter-Materialien ausschließlich um aromatische Verbindungen handelt, ist eine Substitution mithilfe einer der gängigen Kupplungsreaktionen möglich. Als Kupplungsreaktionen kommen z. B. die Suzuki-, Still-, Heck-, Sonogashira-, Kumuda-, Ullmann-, Buchwald-Hartwig-Kupplung sowie deren Varianten zu Einsatz.

Dabei geht man von einem mit Halogenid (Cl, Br, I), insbesondere Br, substituierten phen oder bpy-Derivat aus und setzt es mit dem entsprechenden, mit einer geeigneten Abgangsgruppe substituierten Elektronenleiter-Material um. Vorteilhaft ist die Durchführung einer Suzuki-Kupplung unter Verwendung der entsprechenden Arylboronsäuren und -estern sowie die Buchwald-Hartwig-Kupplung für die Knüpfung von Aryl-N-Bindungen. Abhängig von den Fuktions-Gruppen können auch weitere, gängige Verknüpfungreaktion angewandt werden, z. B. über eine Brücke zwischen Funktions-Gruppe FG und Diimin-Ligand. Bei Anwesenheit von -OH Gruppen kommen hier z. B. Veresterungen und Etherbildung in Frage, bei $-NH_2$ Gruppen Imin- und Amidbildung, bei -COOH Gruppen Esterbildung. Entsprechend muss das Subsitutionsmuster des Diimins angepasst werden (siehe oben unter Punkt "diimin-Ligand"). Die entsprechenden Verfahren zur Anbringung der Funktions-Gruppen FG sind dem Fachmann bekannt.

Als Elektronentransport-Substituenten können beispielsweise folgende Gruppen verwendet werden (Verknüpfung findet an der mit # gekennzeichneten Stelle statt):

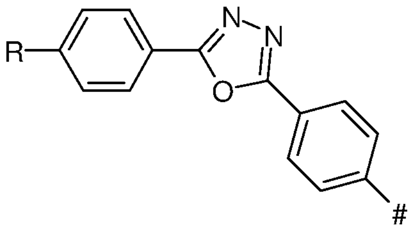

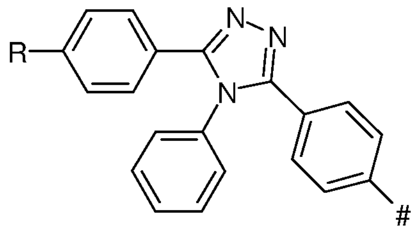

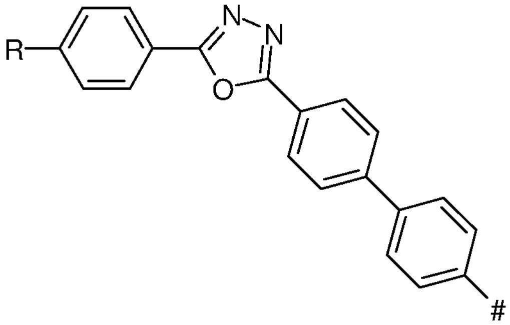

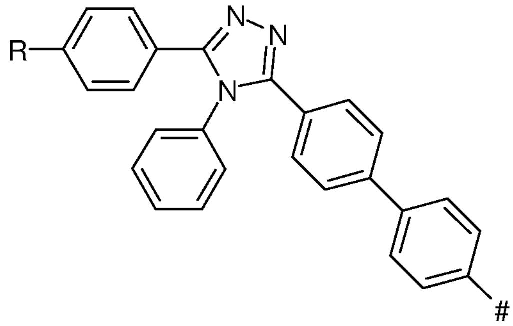

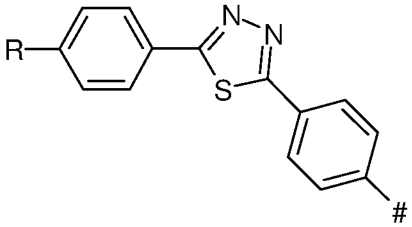

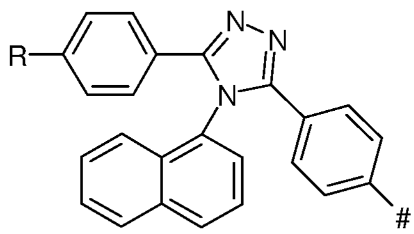

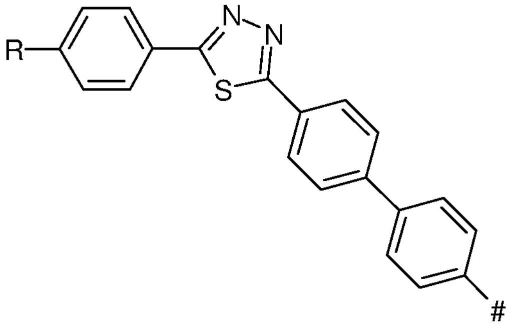

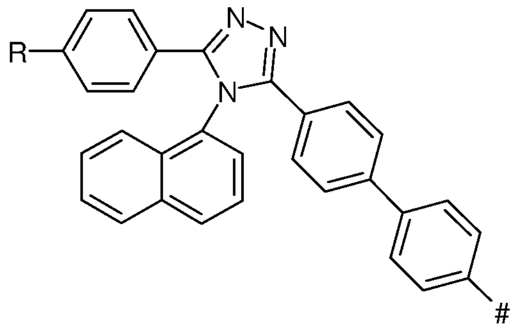

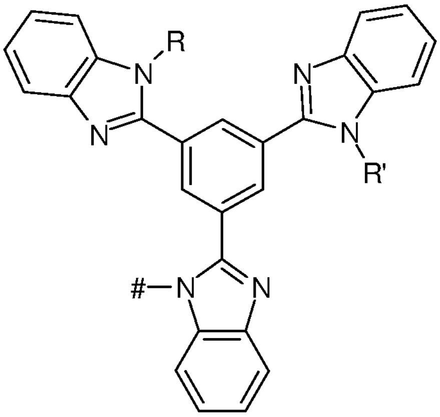

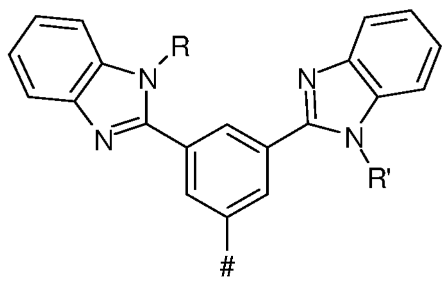

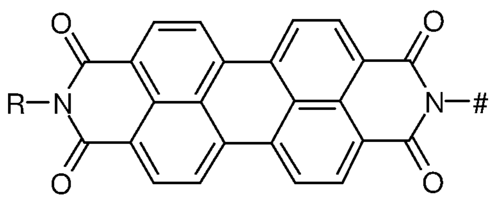

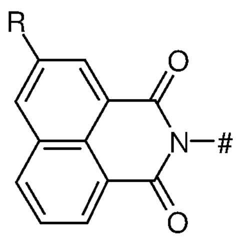

Bei den Substituenten R und R’ handelt es sich um einen Alkyl-Rest $[CH_3-(CH_2)_n-]$ (n = 0 - 20), der auch verzweigt oder mit Halogenen (F, Cl, Br, I) substituiert sein kann, oder um einen Aryl-Rest (insbesondere Phenyl), der mit Alkyl-Gruppen, Halogenen (F, Cl, Br, I), Silan- ($-SiR'''_3$) oder Ethergruppen -OR’” (R’” definiert wie R) substituiert sein kann.

**Lochleiter**

Für den Lochleiter gelten analog die gleichen Aussagen wie für die Elektronenleiter. Auch hier wird die Verknüpfung des Lochleiters an den Diimin-Liganden am einfachsten durch Palladium katalysierte Kupplungsreaktionen verwirklicht; weitere Verknüpfungen, auch über eine Brücke, sind ebenfalls möglich.

Als Lochtransport-Substituenten können beispielsweise folgende Gruppen verwendet werden (Verknüpfung findet an der mit # gekennzeichneten Stelle statt):

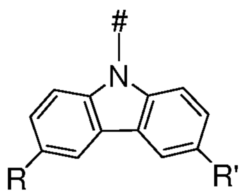

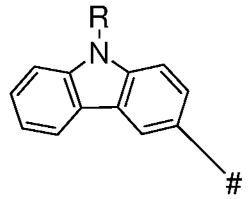

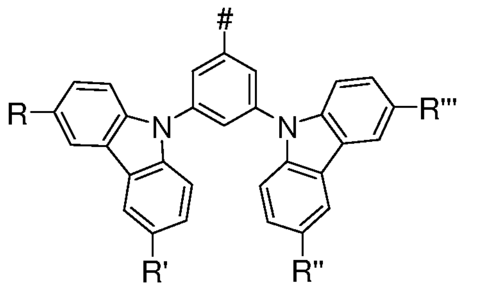

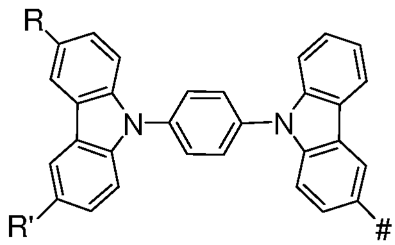

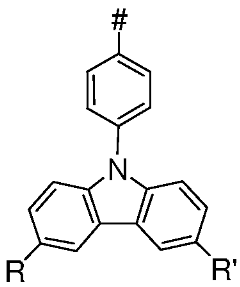

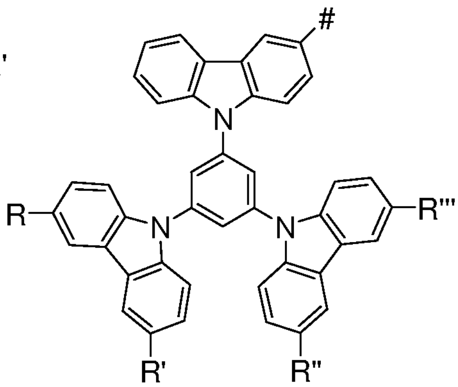

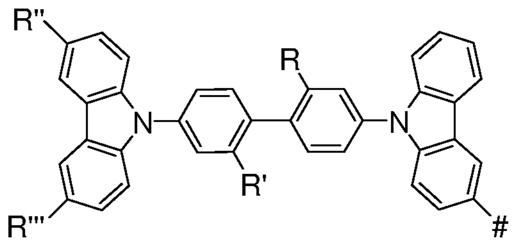

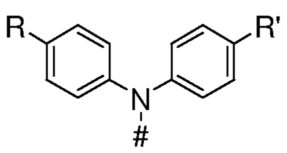

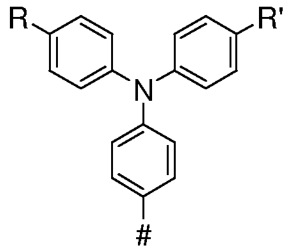

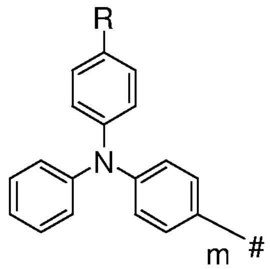

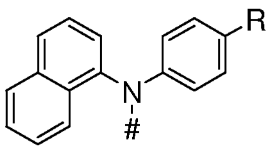

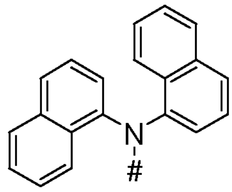

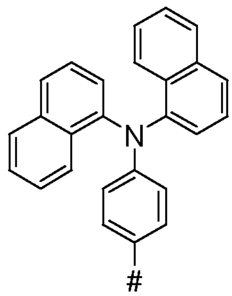

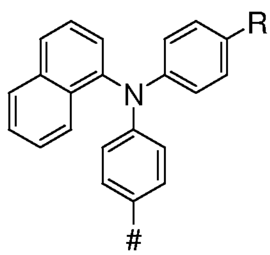

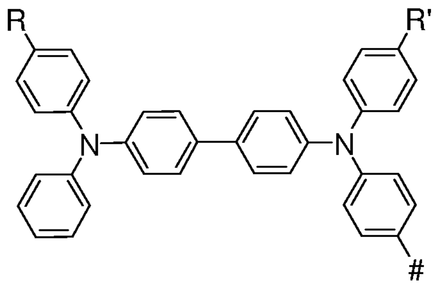

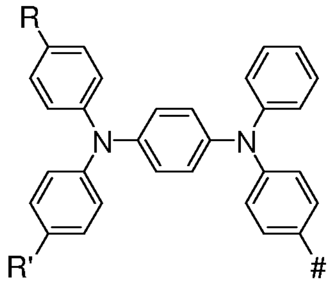

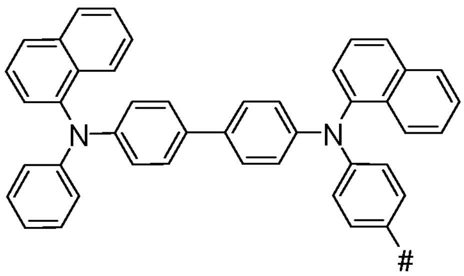

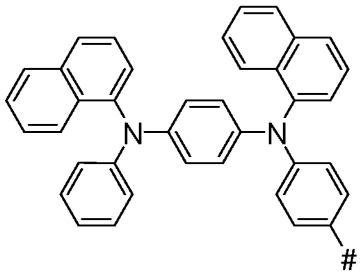

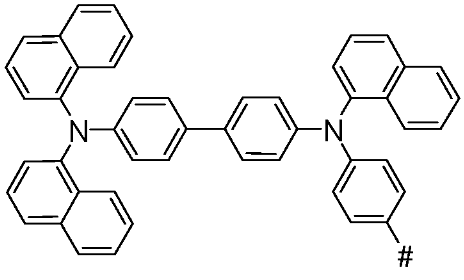

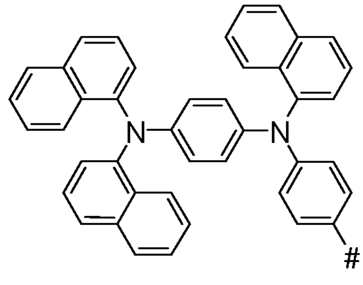

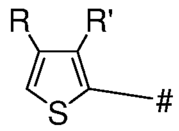 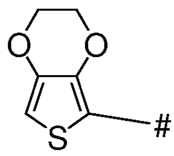 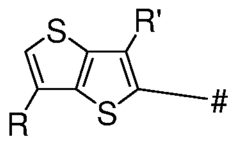 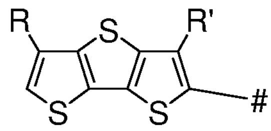

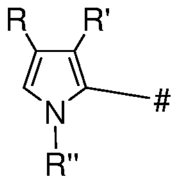 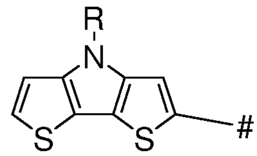

Bei den Substituenten R, R" und R’" handelt es sich um einen Alkyl-Rest [$CH_3$-$(CH_2)_n$-] (n = 0 - 20), der auch verzweigt oder mit Halogenen (F, Cl, Br, I) substituiert sein kann, oder um einen Aryl-Rest (insbesondere Phenyl), der mit Alkyl-Gruppen, Halogenen (F, Cl, Br, I), Silan- (-SiR""$_3$) oder Ethergruppen -OR"" (R"" definiert wie R) substituiert sein kann.

**Löslichkeit**

Bei einer Herstellung von opto-elektronischen Bauteilen mittels nass-chemischer Prozesse ist es vorteilhaft, die Löslichkeit gezielt einzustellen. Hierdurch kann das Auf- bzw. Anlösen einer bereits aufgebrachten Schicht vermieden werden. Durch das Einbringen spezieller Substituenten können die Löslichkeitseigenschaften stark beeinflusst werden. Dadurch ist es möglich, orthogonale Lösungsmittel zu verwenden, die jeweils nur die Substanzen des aktuellen Verarbeitungsschrittes lösen, aber nicht die Substanzen der darunter liegenden Schicht(en):

**Löslichkeit in unpolaren Medien**

Unpolare Funktions-Gruppen FG erhöhen die Löslichkeit in unpolaren Lösungsmitteln und erniedrigen die Löslichkeit in polaren Lösungsmitteln. Unpolare Gruppen sind z. B. Alkylgruppen [$CH_3$-$(CH_2)_n$-] (n = 1 - 30), auch verzweigte, substituierte Alkylgruppen, z. B. mit Halogenen. Hierbei sind besonders hervorzuheben: teil- oder perfluorierte Alkylgruppen sowie perfluorierte Oligo- und Polyether, z. B. [-$(CF_2)_2$-O]$_n$- und (-$CF_2$-O)$_n$- (n = 2 - 500). Weitere unpolare Gruppen sind: Ether -OR, Thioether -SR, unterschiedlich substituierte Silane $R_3$Si- (R = Alkyl oder Aryl), Siloxane $R_3$Si-O-, Oligosiloxane R’(-$R_2$Si-O)$_n$- (R’ = R, n = 2 - 20), Polysiloxane R’(-$R_2$Si-O)$_n$- (n > 20); Oligo/polyphosphazene R’(-$R_2$P=N-)$_n$- (n = 1 - 200).

**Löslichkeit in polaren Medien**

Polare Funktionsgruppen erhöhen die Löslichkeit in polaren Medien. Diese können sein:

- Alkohol-Gruppen: -OH
- Thioalkohole -SH
- Carbonsäure-, Phosphonsäure-, Sulfonsäure-Reste sowie deren Salze und Ester (R = H, Alkyl, Aryl, Halogen; Kationen: Alkalimetalle, Ammonium-Salze): -COOH, -P(0)$(OH)_2$, -P(S)$(OH)_2$, -S(O)$(OH)_2$, -COOR, -P(O)$(OR)_2$, -P(S)$(OR)_2$, -S(O)$(OR)_2$, -CONHR, -P(O)$(NR_2)_2$, -P(S)$(NR_2)_2$, -S(O)$(NR_2)_2$
- Sulfoxide: -S(O)R, -$S(O)_2$R
- Carbonylgruppen: -C(O)R
- Amine: -$NH_2$, -$NR_2$, -N$(CH_2CH_2OH)_2$,
- Hydroxylamine =NOR
- Oligoester, -O$(CH_2O-)_n$, -O$(CH_2CH_2O-)_n$ (n = 2 - 200)
- Positiv geladene Substituenten: z. B. Ammonium-Salze -$N^+R_3X^-$, Phosphonium-Salze -$P^+R_3X^-$
- Negativ geladene Substituenten, z. B. Borate -$(BR_3)$-, Aluminate -$(AlR_3)$- (als Anion kann ein Alkalimetal oder Ammoniumion fungieren).

Um die Anwesenheit frei beweglicher Ionen zu vermeiden, können auch positiv und negativ geladene Substituenten in einer Funktions-Gruppe FG vereinigt werden.

Demgemäß betrifft die Erfindung in einem weiteren Aspekt ein Verfahren zur Herstellung einer opto-elektronischen Vorrichtung, insbesondere wobei die Herstellung naß-chemisch erfolgt und das Verfahren die folgenden Schritte aufweist:

Aufbringung eines in einem ersten Lösungsmittel gelösten ersten Emitterkomplexes auf einen Träger, und Aufbringung eines in einem zweiten Lösungsmittel gelösten zweiten Emitterkomplexes auf den Träger;
wobei der erste Emitterkomplexes nicht in dem zweiten Lösungsmittel löslich ist und der zweite Emitterkomplexes nicht in dem ersten Lösungsmittel löslich ist; und wobei der erste Emitterkomplex und/oder der zweite Emitterkomplex ein erfindungsgemäßer Kupfer(I)komplex ist. Das Verfahren kann weiterhin den folgenden Schritt aufweisen: Aufbringung eines in dem ersten Lösungsmittel oder in einem dritten Lösungsmittel gelösten dritten Emitterkomplexes auf den Träger, wobei der dritte Kupfer(I)komplex ein erfindungsgemäßer Kupfer(I)komplex ist.

In einer bevorzugten Ausführungsform ist die opto-elektronische Vorrichtung eine Weißlicht-OLED, wobei der erste Emitterkomplexe ein Rotlichtemitter ist, der zweite Emitterkomplexe ein Grünlichtemitter ist und der dritte Emitterkomplexe ein Blaulichtemitter ist.

## Figuren

**Figur 1:** Prinzipieller Aufbau eines OLEDs. Die Abbildung ist nicht maßstabsgerecht gezeichnet.

**Figur 2:** Erläuterungen des Elektro-Lumineszenz-Verhaltens a für Übergangsmetall-Komplexe mit kleiner oder sich nur in geringem Maße auswirkender Spin-Bahn-Kopplung (z. B. Metall-Komplexe der ersten Periode der Übergangsmetalle) und **b** für erfindungsgemäß selektierte Cu(I)-Komplexe. Der in **a** für $\tau(T_1)$ angegebene Wert repräsentiert ein Beispiel.

**Figur 3.** ORTEP Bild eines Cu(dmphen)(*nido*-CB($PPh_2)_2$)-Moleküls.

**Figur 4.** Photolumineszenzspektrum von Cu(dmphen)(*nido*-CB($PPh_{2h}$) gemessen an einer Festkörperprobe bei Raumtemperatur.

**Figur 5.** Photolumineszenzspektrum von Cu(dmdpphen)(*nido*-CB($PPh_2)_2$) gemessen an einer Festkörperprobe bei Raumtemperatur.

**Figur 6.** ORTEP Bild eines Cu(dbphen)(*nido*-CB($PPh_2)_2$)-Moleküls.

**Figur 7.** Photolumineszenzspektrum von Cu(dbphen)(*nido*-CB($PPh_2)_2$) gemessen an einer Festkörperprobe bei Raumtemperatur.

**Figur 8.** Temperaturabhängigkeit der Emissionslebensdauer von Cu(dbphen)(*nido*-CB($PPh_2)_2$) gemessen für eine Festkörperprobe bei Anregung mit 532 nm und Detektion mit 590 nm. Der Energieabstand $\Delta E(S_1-T_1)$ und die Emissionslebensdauern der $S_1$ und $T_1$ Zustände wurden durch Anpassung unter Verwendung der Gleichung (4) ermittelt.

## Beispiele

### Beispiel 1

### 1,10-Phenanthrolinliganden, die die Löslichkeit in unpolaren Lösungsmitteln erhöhen

2,9-Dimethyl-4,7-di(n-butyl)-1,10-phenanthrolin, **Phen2**

4,7-Dichloro-2,9-dimethyl-1,10-phenanthrolin, **Phen1,** wird gemäß Literatur (M. Schmittel, H. Ammon Eur. J. Org. Chem. 1998, 785.) hergestellt. Zur Subsitution mit einer n-ButylGruppe wird mit äquimolare Mengen an n-HexMgBr und CuBr zugeben. Die Reinigung von **Phen2** erfolgt mittels Säulenchromatographie über Silikagel.

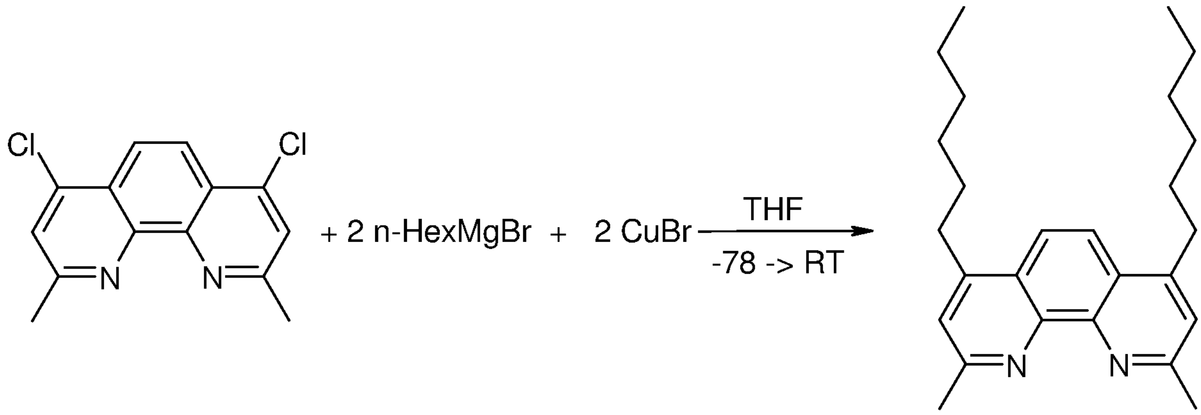


**Phen1** **Phen2**

**Beispiel 2**

5,8-Di(n-butyl)-1,2,3,4,9,10,11,12-octahydrodibenzo[b,j]-[1,10]phenanthrolin, **Phen4**

Das Phenanthrolin-dichlorid **Phen3** wird wieder nach (M. Schmittel, H. Ammon Eur. J. Org. Chem. 1998, 785.) hergestellt. Die Synthese von Phen4 erfolgt in Analogie zu **Phen2**.

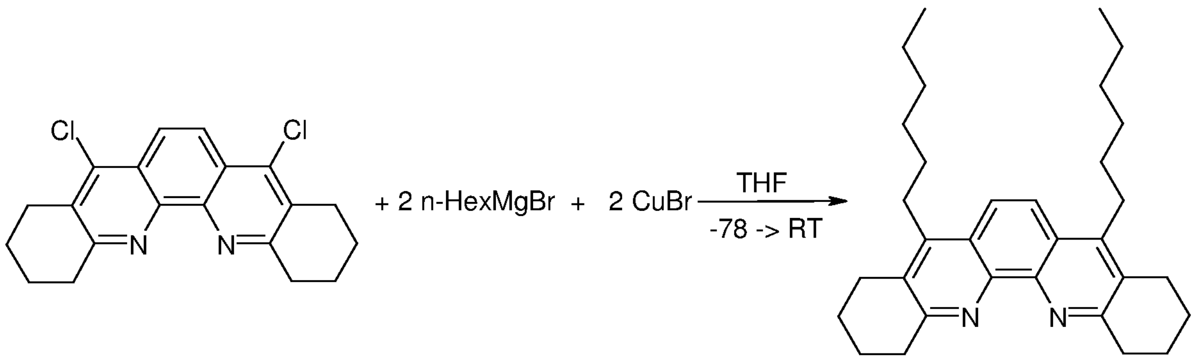


**Phen3** **Phen4**

**Beispiel 3**

2,4,7,9-Tetra(n-heptyl)-1,10-phenanthrolin, **Phen7**

2,4,7,9-Tetra-methyl-1,10-phenanthrolin, **Phen5,** hergestellt nach (G. Butt, R. D. Topsom, J. Heterocyclic Chem. 1981, 18, 641). Das 2,4,7,9-Tetrabromomethylen-1,10-phenanthrolin, **Phen6,** wird durch Seitenkettenbromierung mittels NBS hergestellt und durch Säulenchromatographie ($SiO_2$) isoliert. Die Umsetzung mit n-HexLi führt zu **Phen7**.

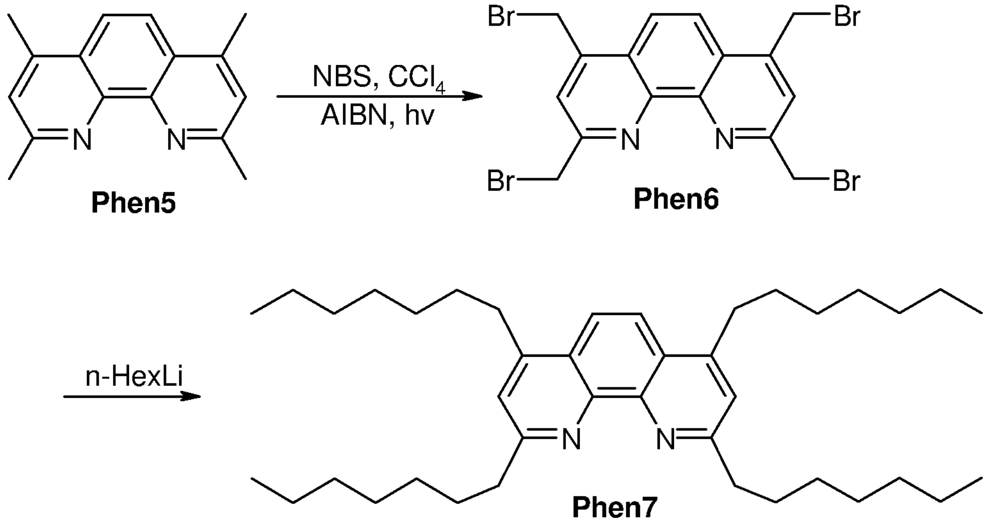


Phen5 Phen6

Phen7

**Beispiel** 4

2,9-Dimethyl-4,7-di-(p-hexylphenyl)-1,10-phenanthrolin, **Phen9**

Ausgehend vom 4,7-Dibromo-2,9-dimethyl-1,10-phenanthrolin, **Phen8,** (M. Schmittel, H. Ammon Eur. J. Org. Chem. 1998, 785) und der kommerziell erhältlichen p-Hexylphenylboronsäure wird der Ligand **Phen9** durch eine Suzuki-Kupplung dargestellt.

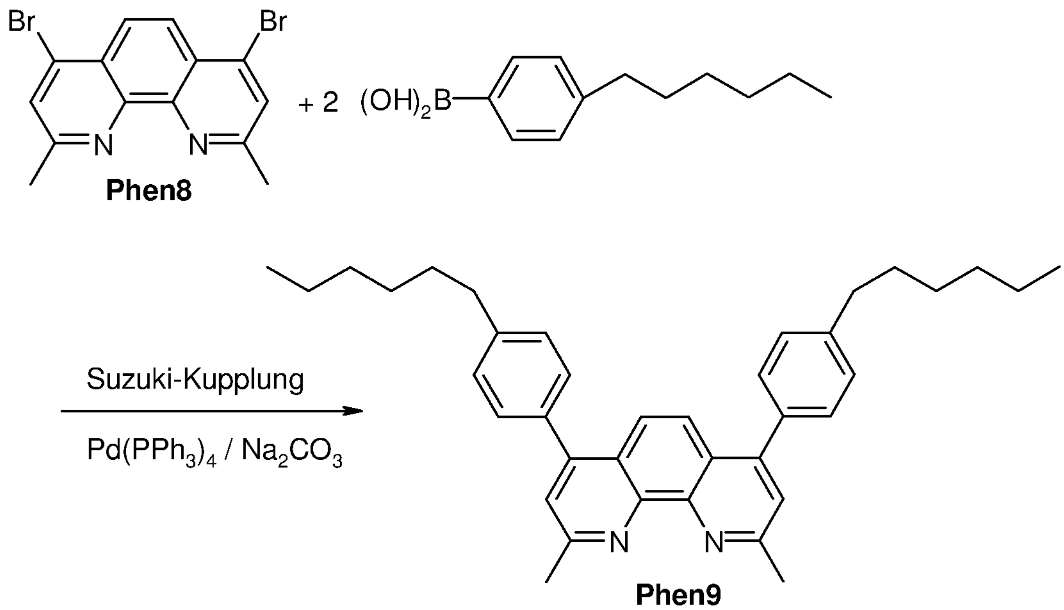


Phen8

Phen9

**1,10-Phenanthrolinliganden, die die Löslichkeit in polaren Lösungsmitteln, insbesondere Wasser, erhöhen**

**Beispiel 5**

4,7-Bis(methoxy-triethylenglykol)-2,9-dimethyl-[1,10]phenanthrolin-4-ol, **Phen10a**

Der Diether **Phen10a** und der Monoether **Phen10b** wird analog zur Literatur (B. Koning, J. W. de Boer, A. Meetsma, R. M. Kellogg, ARKIVOC 2004, 189) dargestellt. Die Isolierung erfolgt mittels Säulenchromatographie.

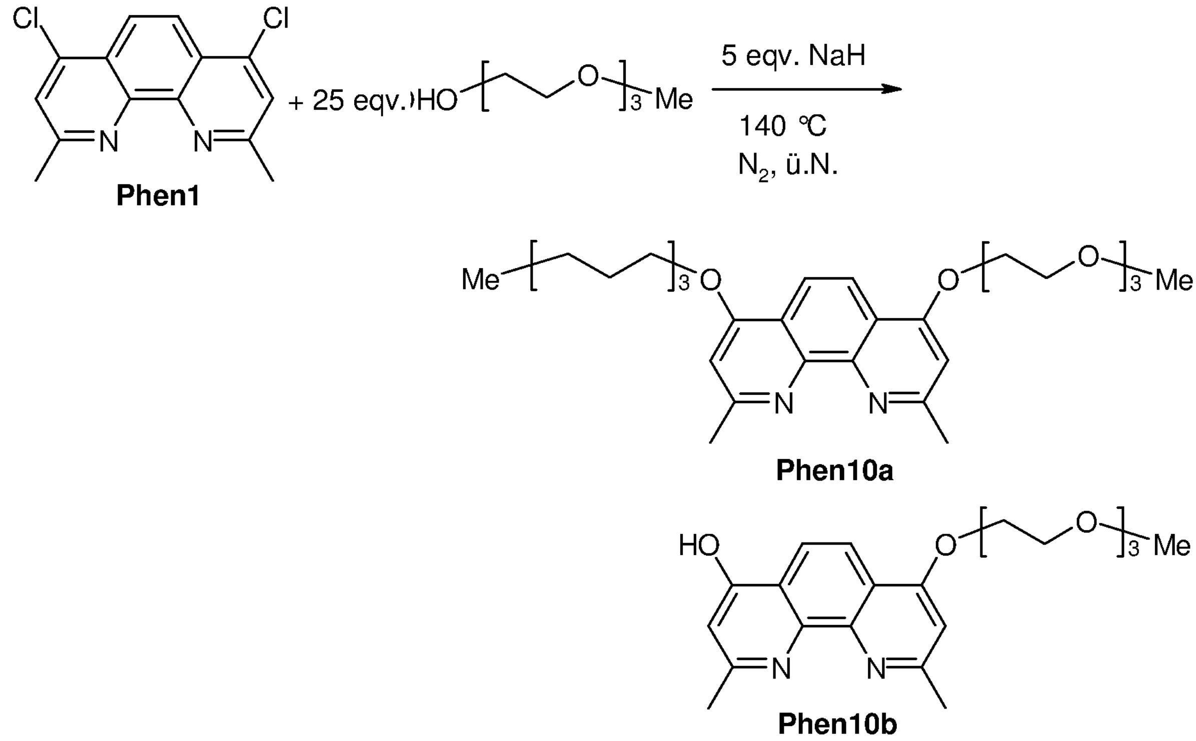


Phen1

Phen10a

Phen10b

**Beispiel 6**

4,7-Bis(methoxy-polyethylenglykol)-2,9-dimethyl-[1,10]phenanthrolin, **Phen11a**

Die Darstellung der Liganden **Phen11a/b** erfolgt analog zu **Phen10a/b.** Zum Einsatz kommt Methoxypolyethylenglykol mit einer durchschnittlichen Molmasse $M_n$ = 350 g/mol (CAS Nr. 9004-74-4), was n ≈ 8 entspricht.

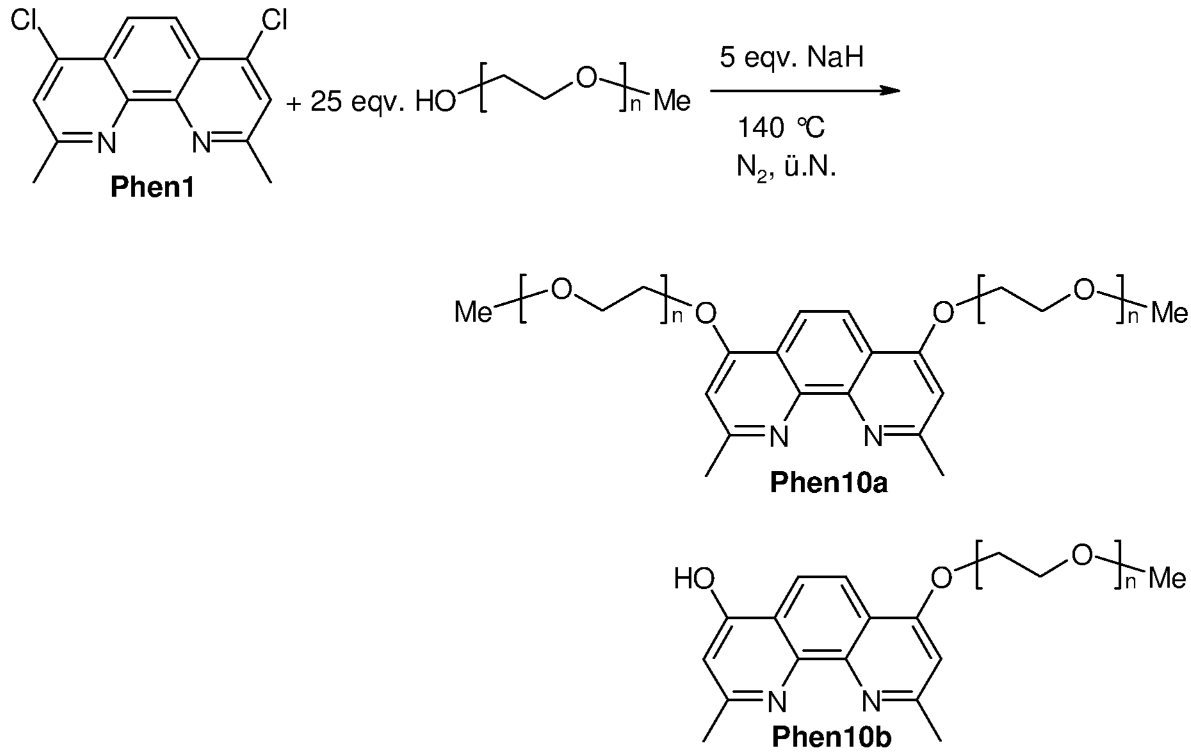


Phen1

Phen10a

Phen10b

n ≈ 8.

**1,10-Phenanthrolinliganden, die mit einem Lochleitergruppen funktionalisiert sind**

**Beispiel 7**

4,7-Bis[(diphenylamino)phenylboronsäure]-2,9-dimethyl-[1,10]phenanthrolin, **Phen12**

Kommerziell erhältliche 4-(Diphenylamino)phenylboronsäure (Aldrich) wird mit **Phen8** zu **Phen12** gekoppelt.

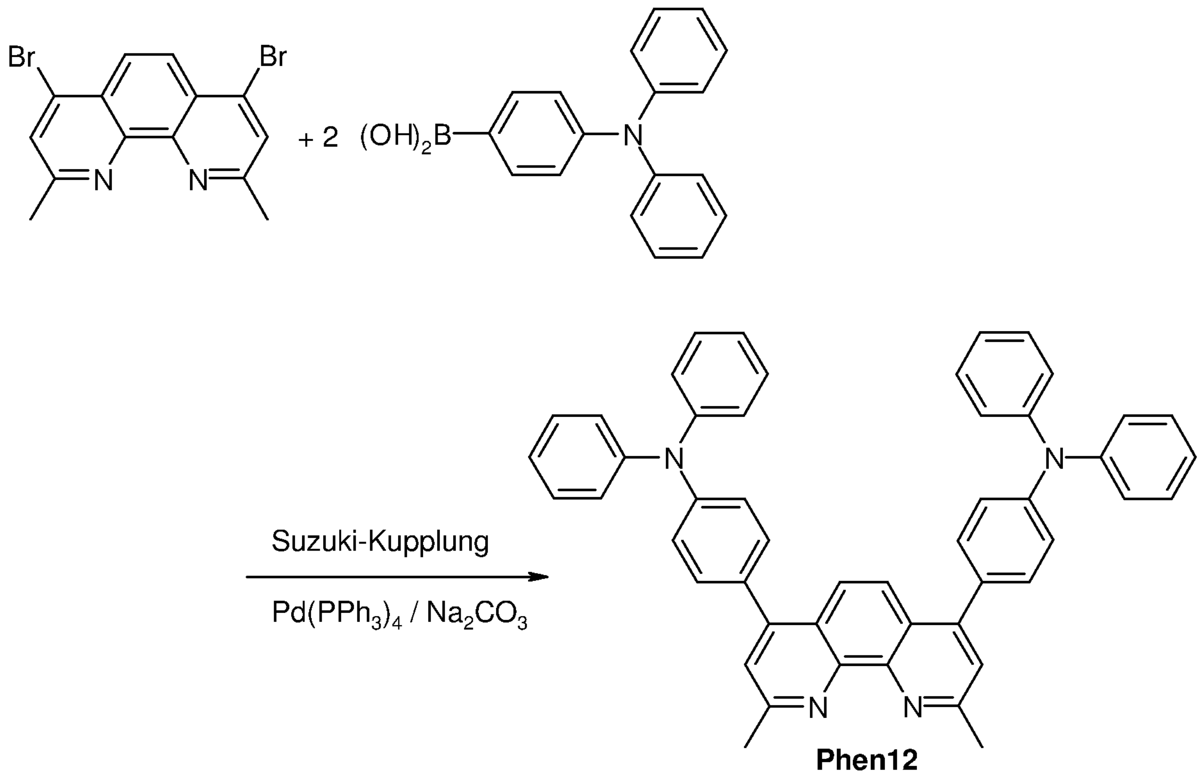


**Phen12**

**Beispiel 8**

4,7-Bis[4-(9H-carbozol-9-yl)phenylboronsäure]-2,9-dimethyl-[1,10]phenanthrolin, **Phen13**

Kommerziell erhältliche 4-(9H-carbozol-9-yl)phenylboronsäure wird mit **Phen8** zu **Phen13** gekoppelt.

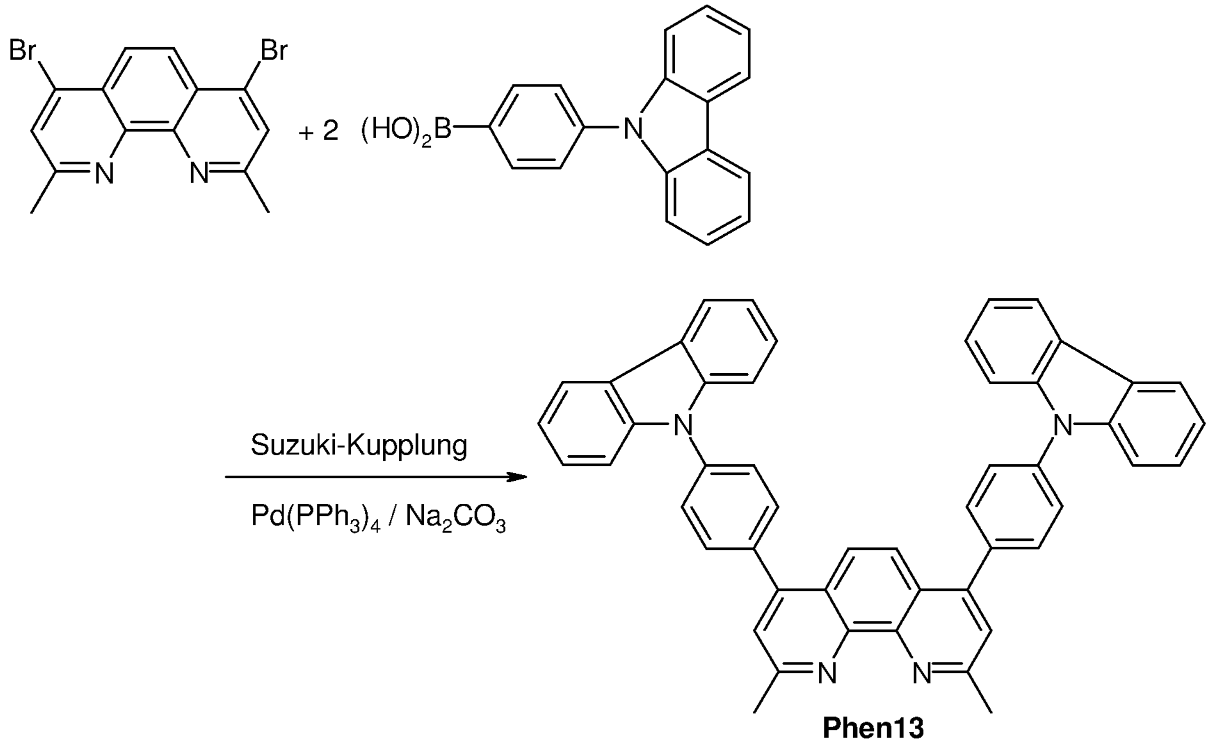


**Phen13**

**1,10-Phenanthrolinliganden, die mit einer Elektronenleitergruppen funktionalisiert sind**

**Beispiel 9**

4,7-Bis(1,2,4-Triazol)-2,9-dimethyl-[1,10]phenanthrolin, **Phen14**

Das bromierte 1,2,4-Triazol wird gemäß (X. J. Feng, P. L. Wu, H. L. Tam, K. F. Li, M. S. Wong, K. W. Cheah, Chem. - Eur. J. 2009, 15, 11681) hergestellt. Durch Umsetzung mit nBuLi und $B(OMe)_3$ und anschließender Hydrolyse mit verdünnter HCl wird die Boronsäure synthetisiert. Die Boronsäure wird schließlich analog zu Beispiel 7 zum Liganden **Phen14** umgesetzt.

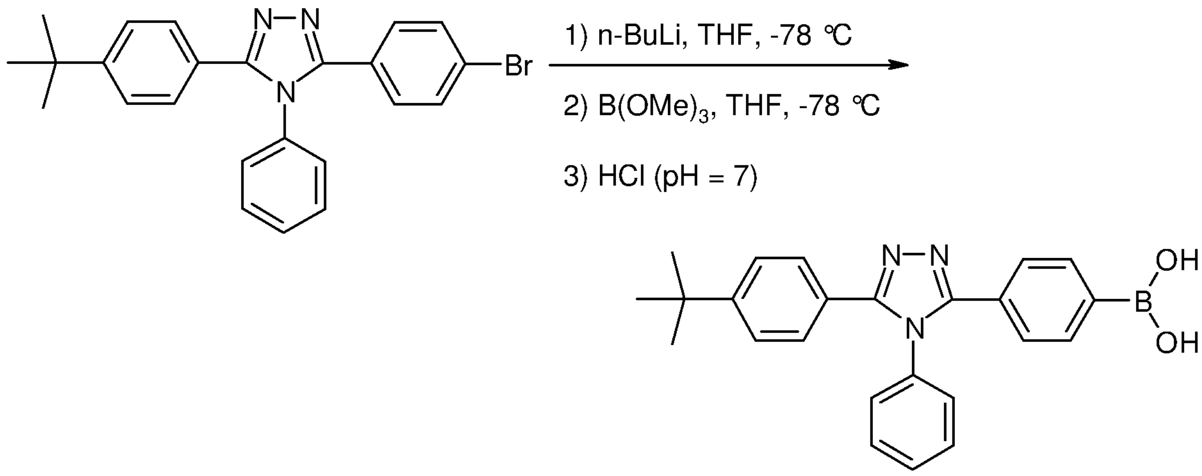


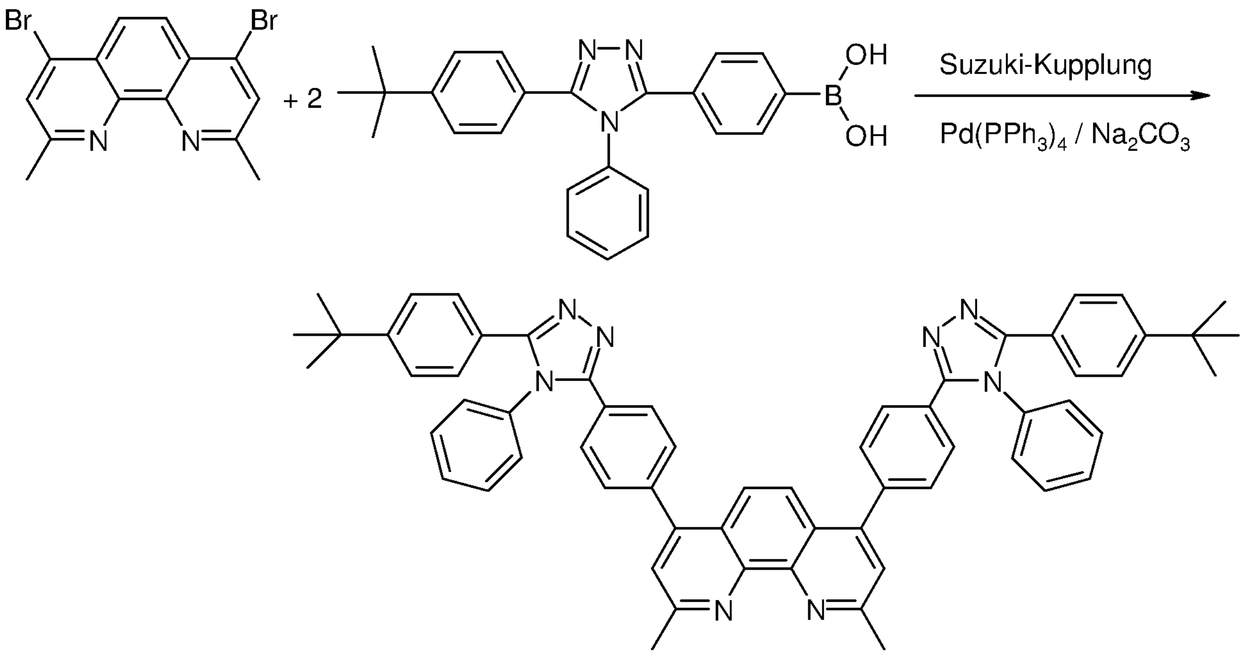


**Phen14**

**1,10-Phenanthrolinliganden, die die Löslichkeit in organischen Lösungsmitteln erhöhen**

**Beispiel 10:**

4,7-Bis(Hexyloxy)-2,9-dimethyl-1,10-phenanthroline, **Phen16**
4,7-Bis((2-ethylhexyl)oxy)-2,9-dimethyl-1,10-phenanthroline, **Phen17**

Das entsprechende 2,9-Dimethyl-l,10-phenanthroline-4,7-diol **Phen15** wurde gemäß (A. F. Larsen, T. Ulven, Org. Lett. 2011, 13, 3546) hergestellt. Durch Deprotonierung mit NaH und Umsetzung mit den entsprechenden Alkylbromiden (H. Frisell, B. Akermark, Organometallics 1995, 14, 561) wurden die alkylierten Liganden **Phen16** und **Phen17** erhalten.

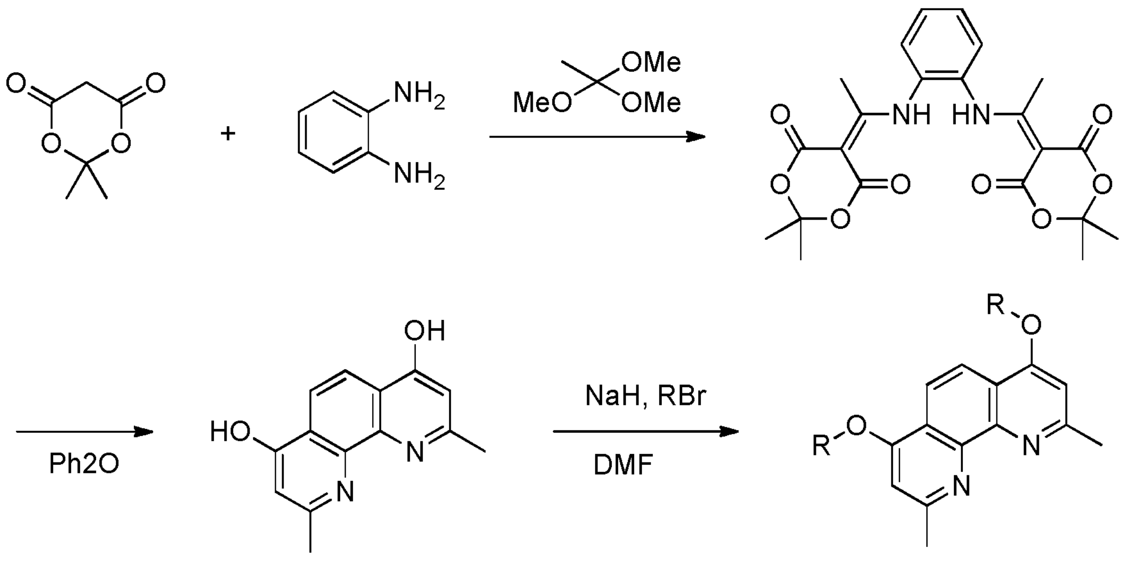


**Phen15**

R = Hexyl **Phen16**
2-Ethylhexyl **Phen17**

**Synthese der einfach negativ geladenen L∩L-Liganden**

**Beispiel 11:**

2-(2-Pyridinyl)-1H-indol, (**N∩N1**)

1-(2-Pyrdinyl)ethanon wurde in einer Fischer-Indolsynthese mit Phenylhydrazin zu 2-(2-Pyridinyl)-1H-indol gemäß Literatur (R. P. Thummel, V. Hegde, J. Org. Chem. 1989, 54 (7), 1720) umgesetzt.

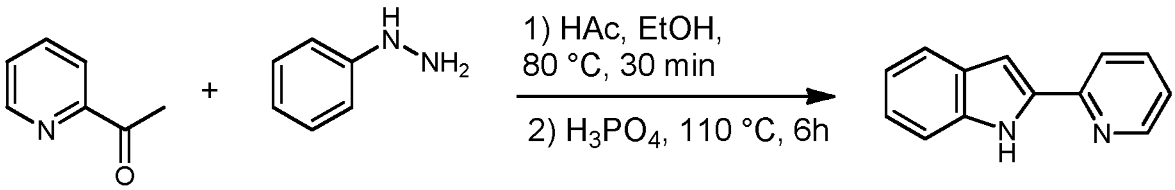


**Beispiel 12:**

Bis(1-pyrazolyl)borat, **(N∩N2)**

Pyrazol wurde in der Schmelze mit NaB(Ph)4 gemäß Literatur (S. Trofimenko, J. Am. Chem. Soc. 1967, 89, 6288) zu dem entsprechenden Natriumsalz des Diphenyldi(1H-1-pyrazolyl)borats umgesetzt, das in einer weiteren Salzmetathese in das lösliche Tetrabutylammonium-diphenyldi(1H-1-pyrazolyl)borat überführt wurde.

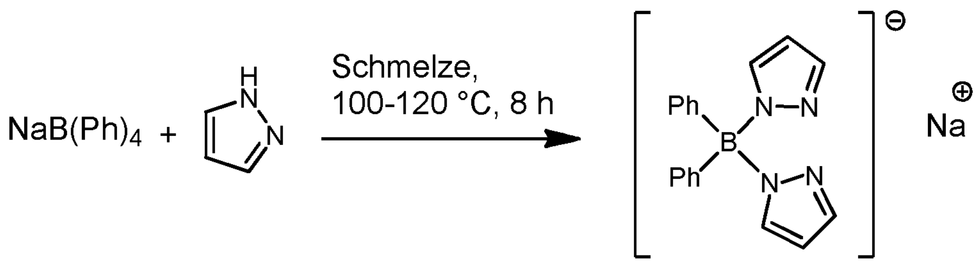

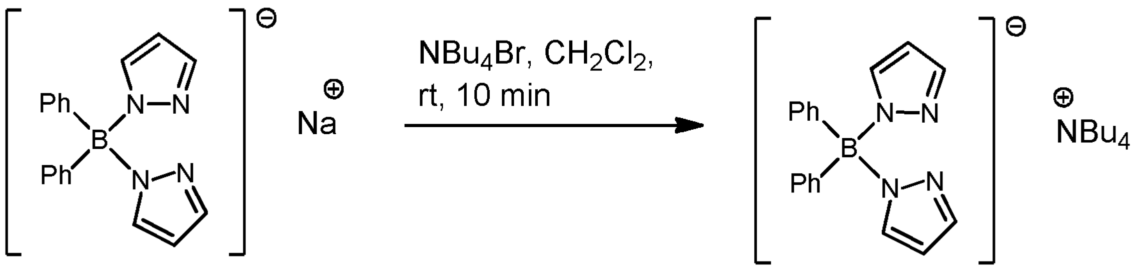


**N⌒N2**

**Synthese der Cu(I)-Komplexe:**

Unter Verwendung der oben dargestellten Phen-Liganden werden die entsprechenden neutralen Cu(**Phen**)(P∩P)-Komplexe mit folgendem, einfach negativ geladenem Bisphosphan hergestellt:

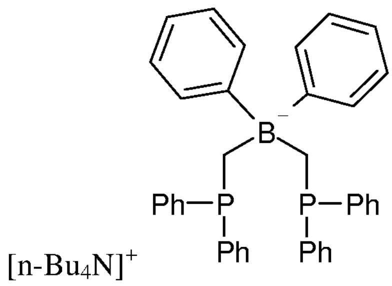

Das [n-$Bu_4$N](P∩P) wird gemäß (J. C. Peters, J. C. Thomas, Inorg. Synth. 2004, 34, 11) hergestellt.

Unter Stickstoffatmosphäre werden äquimolare Mengen an [n-$Bu_4$N](P∩P) und [$Cu(CH_3CN)_4$]$PF_6$ in Acetonitril gerührt. Nach 2 h wird der entsprechende Phen-Ligand zugeben. Das Produkt fällt aus und wird abfiltriert. Nach dem Waschen mit Wasser, kaltem Acetonitril und Diethylether wird das Produkt im Vakuum getrocknet.

Derart werden folgende Komplexe hergestellt:

**Beispiel 13: Cu(Phen2)(P∩P)**

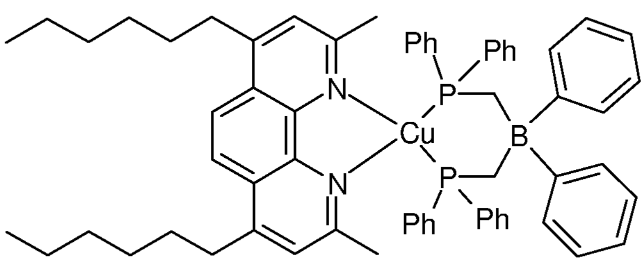

**Beispiel 14: Cu(Phen4)(P∩P)**

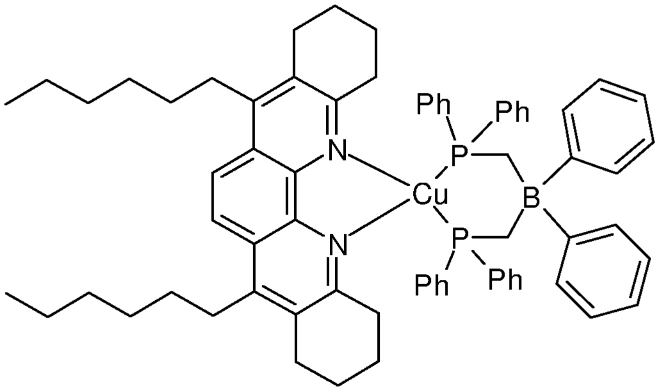

**Beispiel 15: Cu(Phen7)(P∩P)**

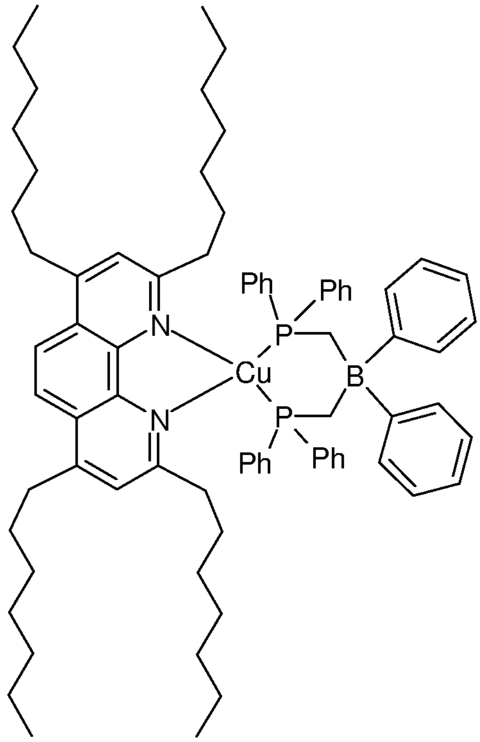

**Beispiel 16: Cu(Phen9)(P∩P)**

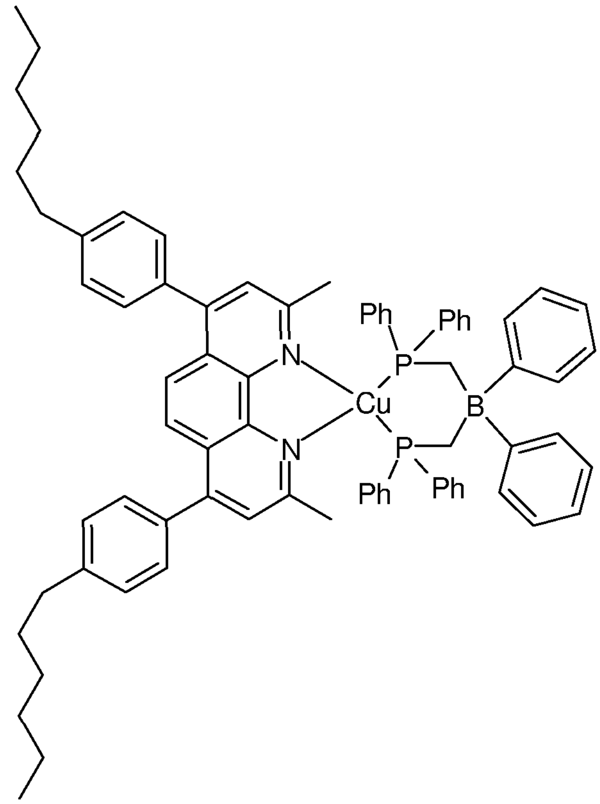

**Beispiel 17: Cu**(**Phen10a**)(**P∩P**)

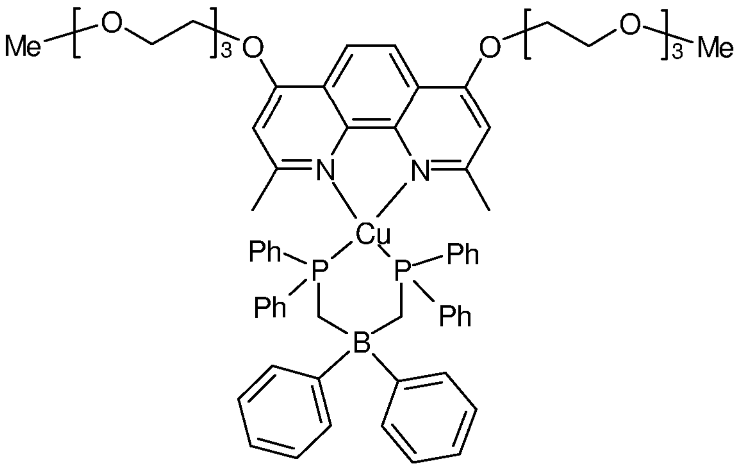

**Beispiel 18: Cu**(**Phen11a**)(**P∩P**)

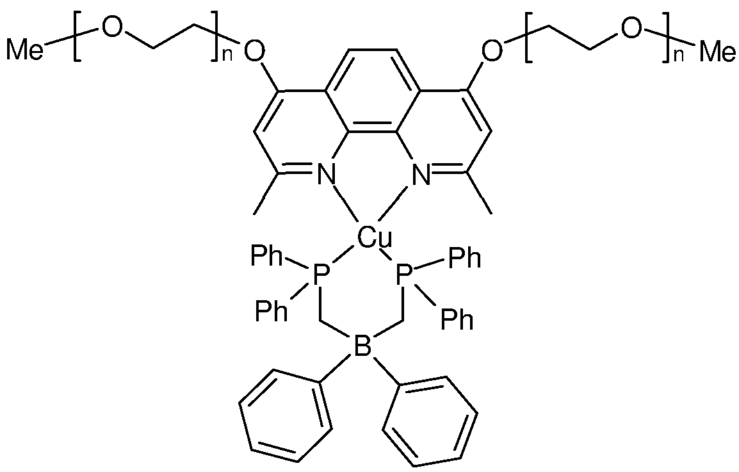

n ≈ 8.

**Beispiel 19: Cu(Phen12)(P∩P)**

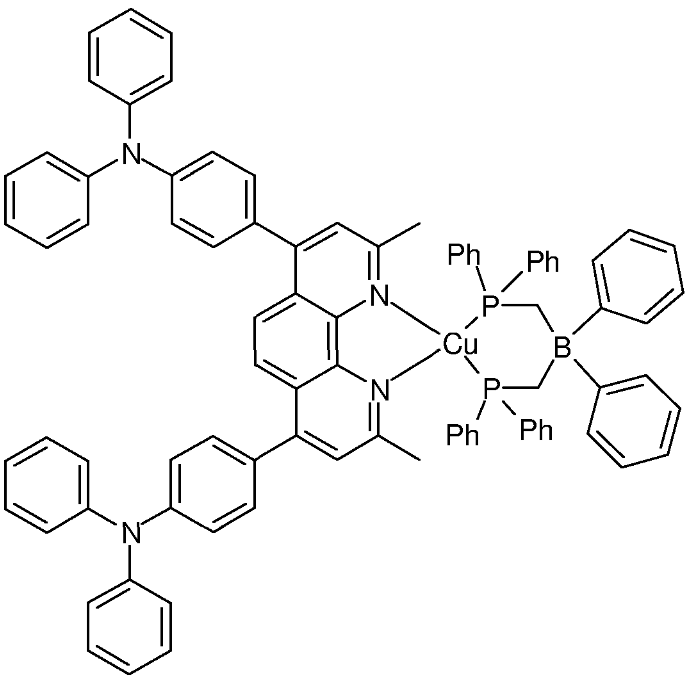

**Beispiel 20: Cu(Phen13)(P∩)**

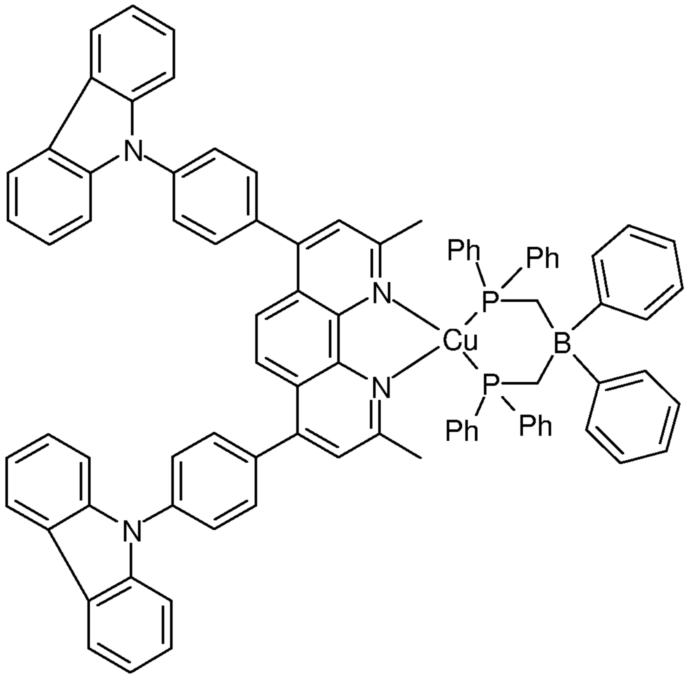

**Beispiel 21:** Cu(**Phen14)(P∩P)**

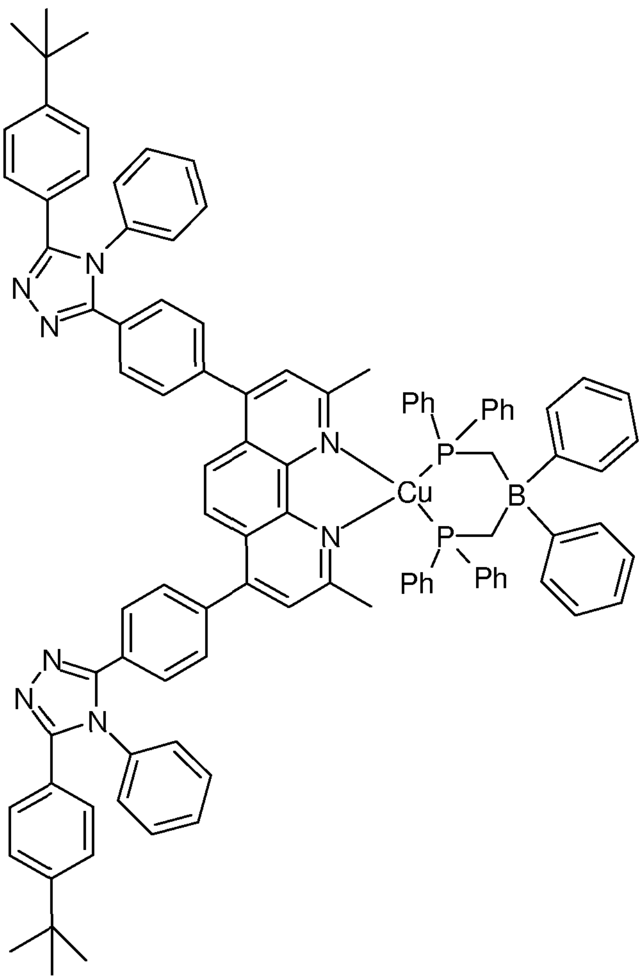

**Beispiel 22: Cu**(**Phen16**)(**P**∩**P**)

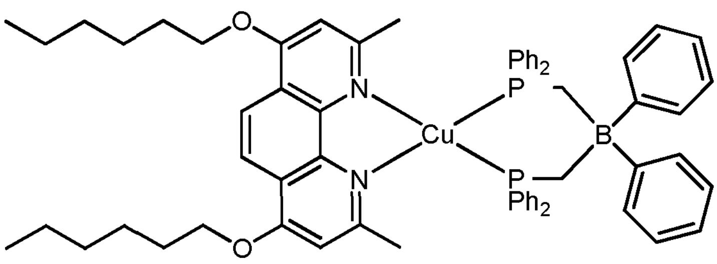

**Beispiel 23: Cu**(**Phen17**)(**P**∩**P**)

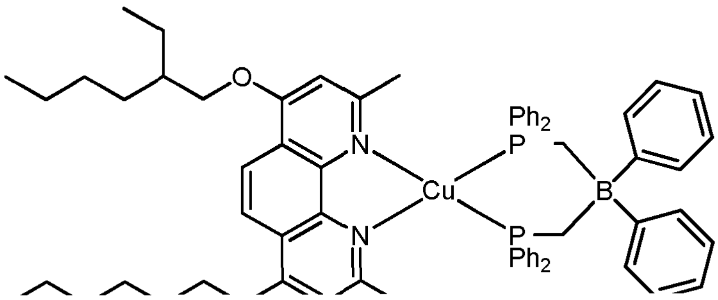

**Beispiel 24**

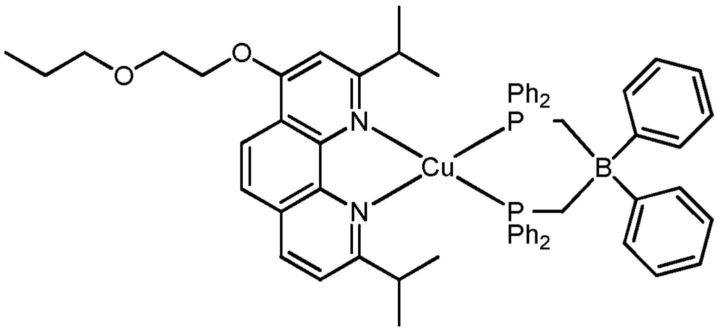

**Beispiel 25**

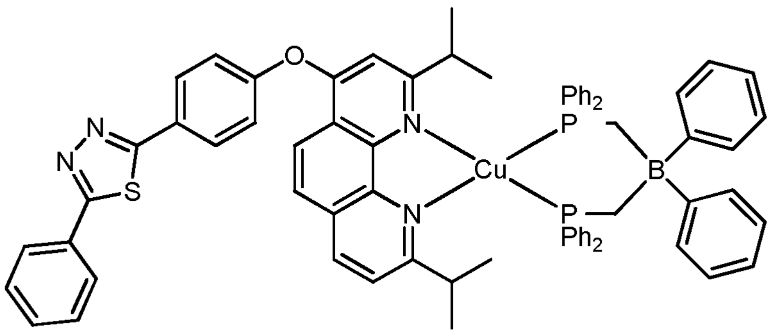

**Beispiel 26**

**Cu(dmphen)(*nido*-CB($PPh_2$)$_2$)** (CB = $C_2B_9H_{10}$)

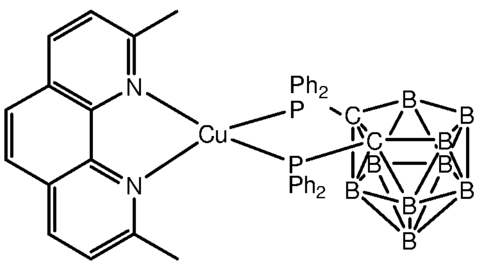

Syntheseweg:

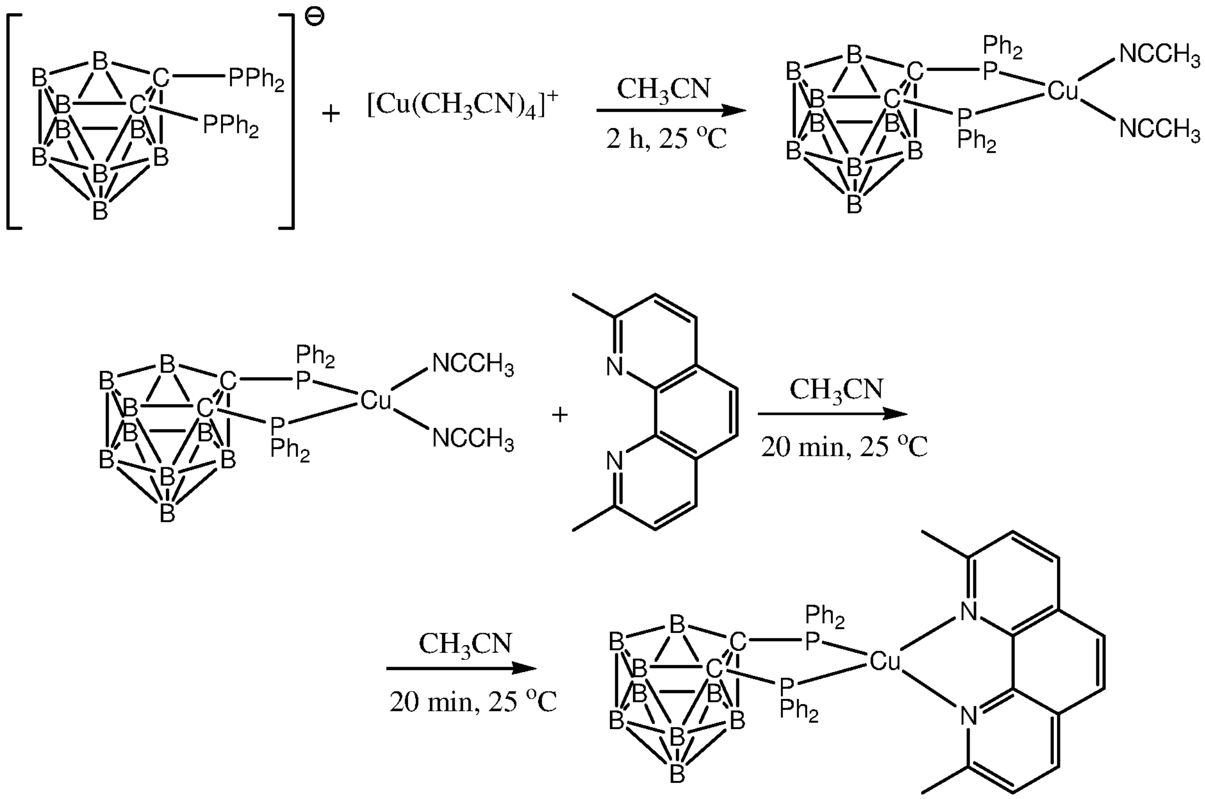


Eine Lösung von 330 mg (0.44 mmol) Tetrapropylammonium 7,8-Bis-(diphenylphosphino)-7,8-dicarba-*nido*-undecarborat ($NPr_4^+$ *nido*-$CB(PPh_2)_2^-$, (synthetisiert nach Zakharkin, L. I.; Kalinin, V. N. Tetrahedron Lett. 1965, 7, 407.) und 163 mg (0.44 mmol) $Cu(CH_3CN)_4^+PF_6^-$ in 50 ml Acetonitril wurde unter Stickstoff 2 h bei 25 °C gerührt. Anschließend wurden 91 mg (0.44 mmol) Neocuproine (dmphen) zugegeben. Das Produkt Cu(dmphen)(*nido*-$CB(PPh_2)_2$) fällt als gelbes Pulver aus und wird abfiltriert. Das Rohprodukt wird mit Wasser, kaltem Acetonitril und Diethylether gewaschen und im Vakuum getrocknet.

Ausbeute: 300 mg (88 %).

Elementalanalyse: C - 61.23 %, H - 5.36 %, N - 3.52 %.

Für $C_{40}H_{42}N_2B_9CuP_2$ erwartet: C - 62.11 %, H - 5.47 %, N - 3.62 %.

ES-MS: e/z = 773.4 ($M^+$)

Kristallisation aus einer Dichlormethan/Toluol Mischung liefert Kristalle von Cu(dmphen)(*nido*-$CB(PPh_2)_2$) $\times$ $CH_2Cl_2$ geeignet für Röntgenstrukturanalyse. In Figur 3 ist ein ORTEP Bild eines Cu(dmphen)(*nido*-$CB(PPh_2)_2$) Moleküls gezeigt. Seine Lumineszenzeigenschaften, gemessen an einer Festkörperprobe bei Raumtemperatur, sind in Figur 3 dargestellt.

Tabelle 1. Photolumineszenzdaten für Cu(dmphen)(*nido*-$CB(PPh_2)_2$) (gemessen unter Stickstoff-Schutzgasatmosphere)

| Bedingungen: | Festkörper (300 K) | PMMA (300 K) | PMMA (77 K) |
|---|---|---|---|
| Emissionsmaximum: | 557 nm | 577 nm | 597 nm |
| Emissionslebensdauer $\tau$ : | 11 jus | 14 jus | 600 $\mu$s |
| Quantenausbeute $\phi_{PL}$: | 55 % | 40 % | |

Die starke Abnahme der Emissionslebensdauer bei Temperaturerhöhung von T = 77 K (600 $\mu$s) auf 300 K (14 $\mu$s) um einen Faktor größer als 40 ist - bei dem beobachteten $\phi_{PL}$-Wert von 40 % - ein Beleg für die thermische Besetzung eines kürzerlebigen emittierenden elektronischen Zustandes, d. h. ein Beleg für das Auftreten des Singulett-Harvesting-Effektes.

**Beispiel 27:**

**Cu(dmdpphen)(*nido*-CB($PPh_2$)$_2$)** (CB = $C_2B_9H_{10}$)

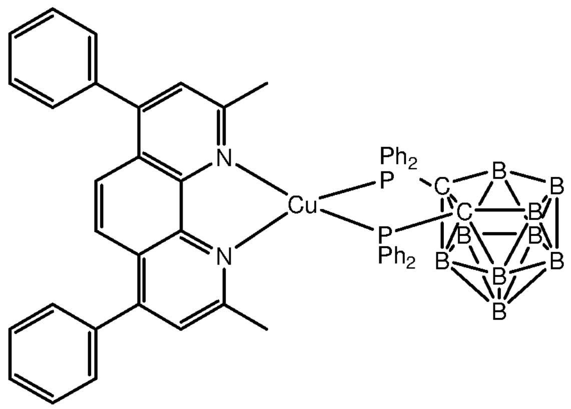

Syntheseweg:

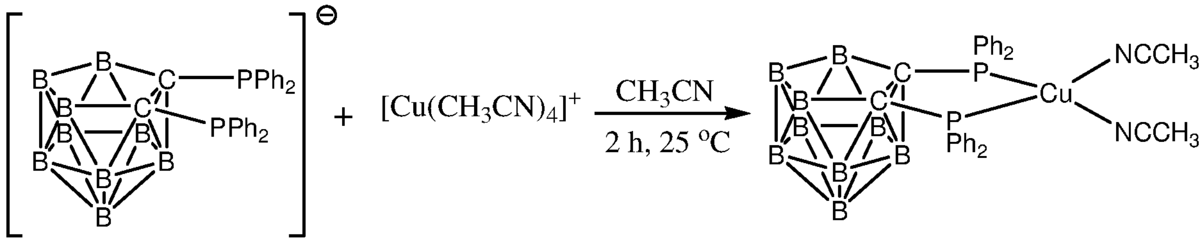

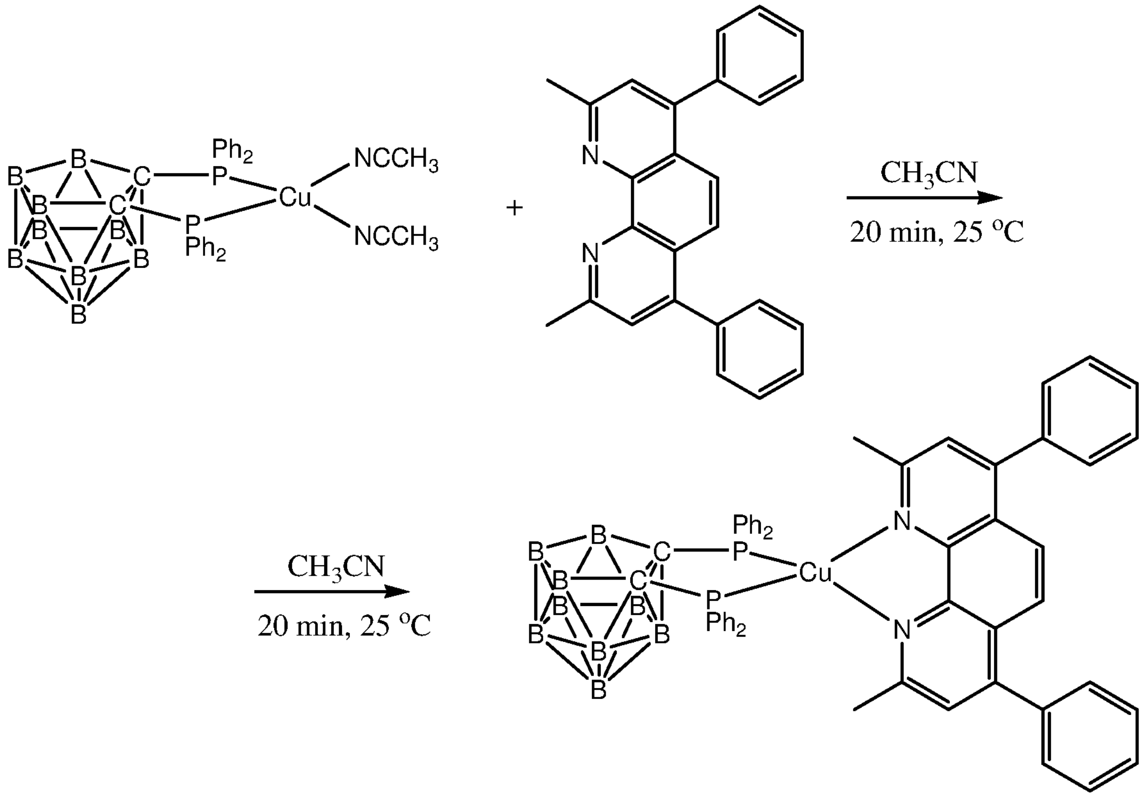


Eine Lösung von 188 mg (0.25 mmol) Tetrapropylammonium-7,8-Bis-(diphenylphosphino)-7,8-dicarba-*nido*-undecarborat ($NPr_4^+$ *nido*-$CB(PPh_2)_2^-$) und 93 mg (0.25 mmol) $Cu(CH_3CN)_4^+PF_6^-$ in 50 ml Acetonitril wurde unter Stickstoff 2 h bei 25 °C gerührt. Anschließend wurden 90 mg (0.25 mmol) Bathocuproine (dmdpphen) zugegeben. Das Produkt Cu(dmdpphen)(*nido*-$CB(PPh_2)_2$) fällt als gelbes Pulver aus und wird abfiltriert. Das Rohprodukt wird mit Wasser, kaltem Acetonitril und Diethylether gewaschen und im Vakuum getrocknet.
Ausbeute: 200 mg (86 %).

Die Lumineszenz-Eigenschaften von Cu(dmdpphen)(*nido*-$CB(PPh_2)_2$), gemessen an einer Festkörperprobe bei Raumtemperatur, sind in Figur 5 dargestellt.

**Beispiel 28:**

**Cu(dbphen)(*nido*-$CB(PPh_2)_2$)** (CB = $C_2B_9H_{10}$)

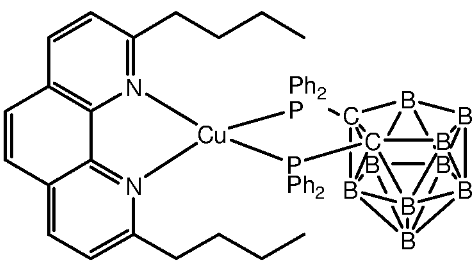

Syntheseweg:

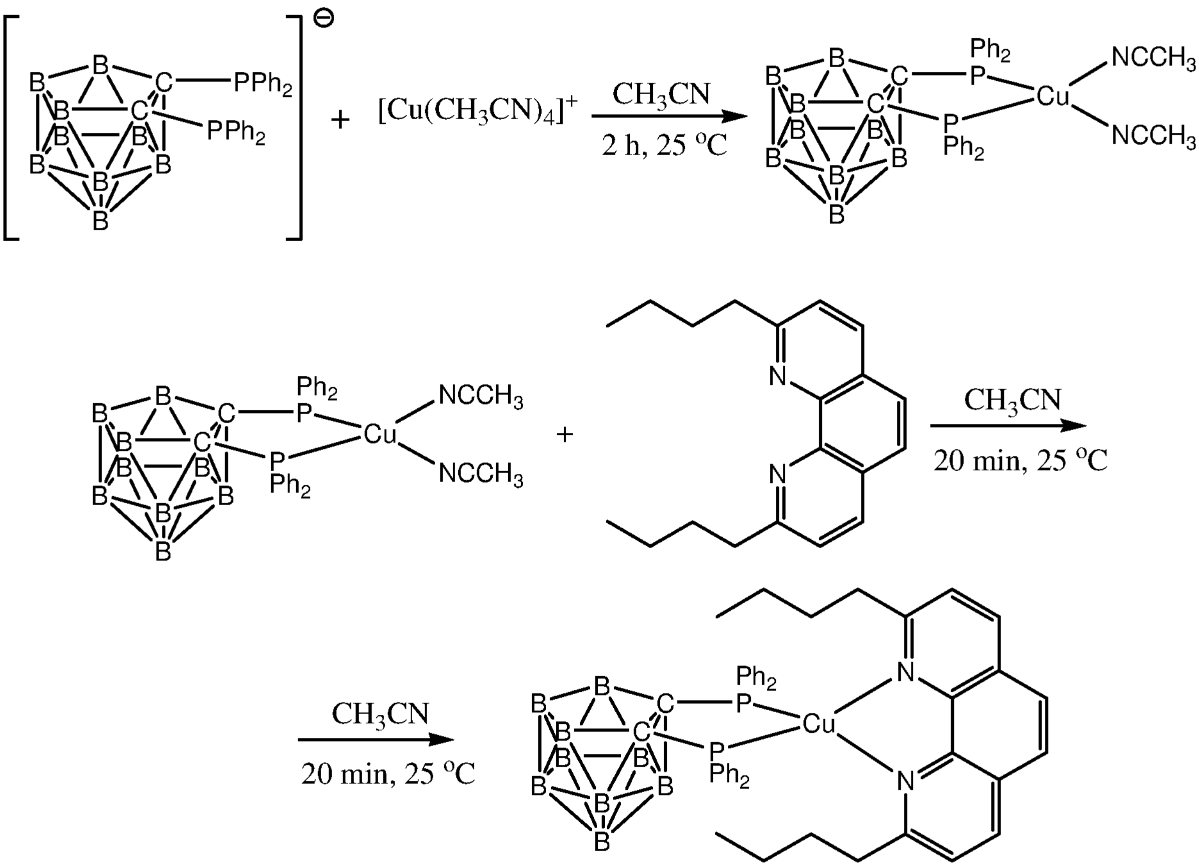


Eine Lösung von 150 mg (0.2 mmol) Tetrapropylammonium-7,8-Bis-(diphenylphosphino)-7,8-dicarba-*nido*-undecarborat ($NPr_4^+$ *nido*-$CB(PPh_2)_2^-$) und 74 mg (0,2 mmol) $Cu(CH_3CN)_4^+PF_6^-$ in 50 ml Acetonitril wurde unter Stickstoff 2 h bei 25 °C gerührt. Anschließend wurden 58 mg (0,2 mmol) 2,9-Di-n-butylphenanthrolin (dbphen) zugegeben. Das Produkt Cu(dmphen)(*nido*-$CB(PPh_2)_2$) fällt als gelbes Pulver aus und wird abfiltriert. Das Rohprodukt wird mit Wasser, kaltem Acetonitril und Diethylether gewaschen und im Vakuum getrocknet.
Ausbeute: 145 mg (85 %).
Elementalanalyse: C - 63.51 %, H - 6.12 %, N - 3.14 %.
Für $C_{46}H_{54}N_2B_9CuP_2$ erwartet: C - 64.41 %, H - 6.35 %, N - 3.27 %.
ES-MS: e/z = 858.5 ($M^+$)
Cu(dbphen)(*nido*-$CB(PPh_2)_2$) wird aus einer Mischung aus Dichlormethan und Toluol kristallisiert. In Figur 6 ist ein ORTEP Bild einer Cu(dbphen)(*nido*-$CB(PPh_2)_2$) Moleküls gezeigt.

Ein Photoluminescenzspektrum von Cu(dbphen)(*nido*-$CB(PPh_2)_2$), gemessen an einer Festkörperprobe bei Raumtemperatur, ist in Figur 7 gezeigt.

Tabelle 2. Photolumineszenzdaten für Cu(dbphen)(*nido*-$CB(PPh_2)_2$) (gemessen unter Stickschtoff Schutzgasatmosphere)

| Bedingungen: | Festkörper (300 K) | PMMA (300 K) | PMMA (77 K) |
|---|---|---|---|
| Emissionsmaximum: | 575 nm | 575 nm | 579 nm |
| Emissionslebensdauer $\tau$: | 5 $\mu$s | 11 $\mu$s | 700 $\mu$s |

Die starke Abnahme der Emissionslebensdauer bei Temperaturerhöhung von T = 77 K (700 $\mu$s) auf 300 K (11 $\mu$s) um einen Faktor größer als 60 ist - bei der beobachteten hohen Emissionsquantenausbeute - ein Beleg für die thermische Besetzung eines kürzerlebigen emittierenden elektronischen Zustandes, d. h. ein Beleg für das Auftreten des Singulett-Harvesting-Effektes.

Figur 8 zeigt die Abhängigkeit der Emissionslebensdauer als Funktion der Temperatur gemessen für eine Cu(dbphen)(*nido*-$CB(PPh_2)_2$)-Festkörperprobe. Etwa unterhalb von T = 100 K zeigt sich ein Plateau, das die Emissionslebensdauer des $T_1$ Zustandes von ca. 440 $\mu$s widerspiegelt. Die eingezeichnete Kurve repräsentiert eine Fit-Funktion

gemäß Gleichung (4). Die resultierenden Fit-Werte für $\pi(S_1)$ und $\Delta E(S_1\text{-}T_1)$ sind in der Figur 8 eingetragen.

**Weitere Beispiele**

**Beispiel 29**

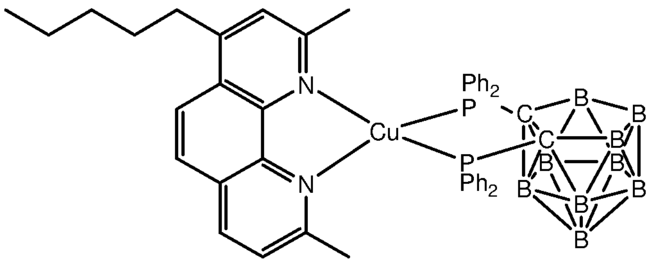

**Beispiel 30**

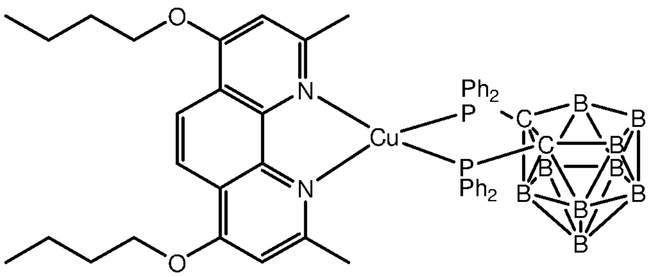

**Beispiel 31**

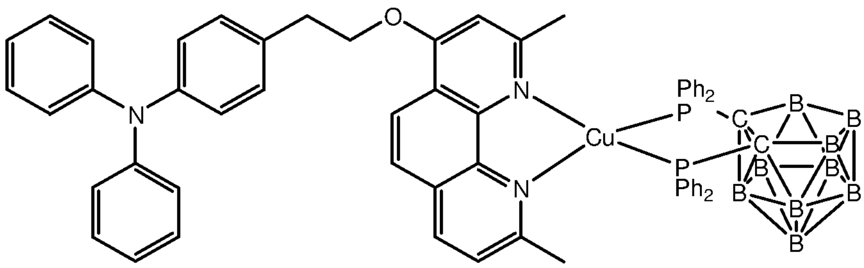

**Beispiel 32**

Unter Verwendung der oben dargestellten Phen-Liganden und der einfach negativ geladenen L∩L-Liganden wurden die entsprechenden neutralen Cu(**Phen**)(L∩L)-Komplexe hergestellt:

Unter Stickstoffatmosphäre wurde 2-(2-Pyridinyl)indol, **N∩N1** in THF mit nBuLi bei Raumtemperatur deprotoniert, 1 Stunde rühren gelassen und mit der äquimolaren Menge an $[Cu(CH_3CN)_4]PF_6$ versetzt. Nach 2 h wurde der entsprechende Phen-Ligand zugeben. Das Produkt fiel aus und wurde abfiltriert. Nach dem Waschen mit Wasser, kaltem Acetonitril und Diethylether wurde das Produkt im Vakuum getrocknet.

**Cu(Phen16)(N∩N1)**

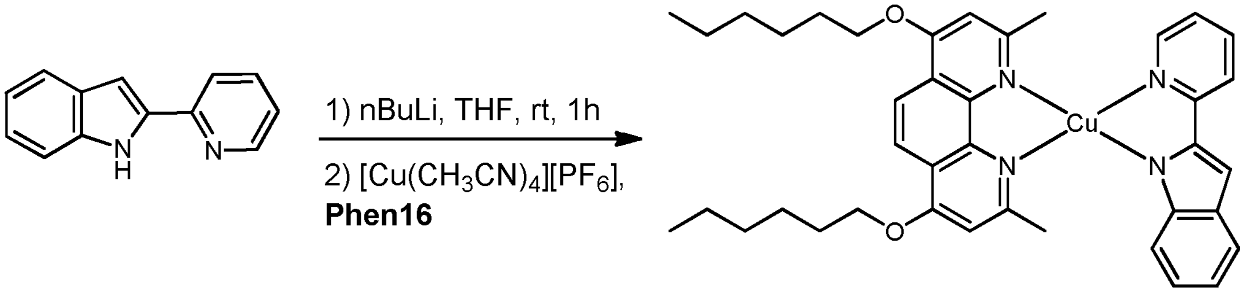


**Beispiel 32: Cu(Phen17)(N∩N1)**

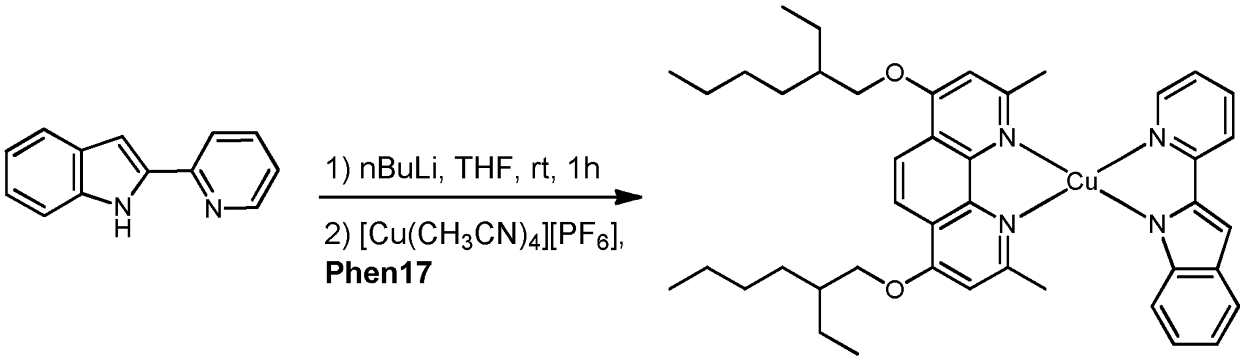


**Beispiel 33: Cu(BCP)(N∩N1)**

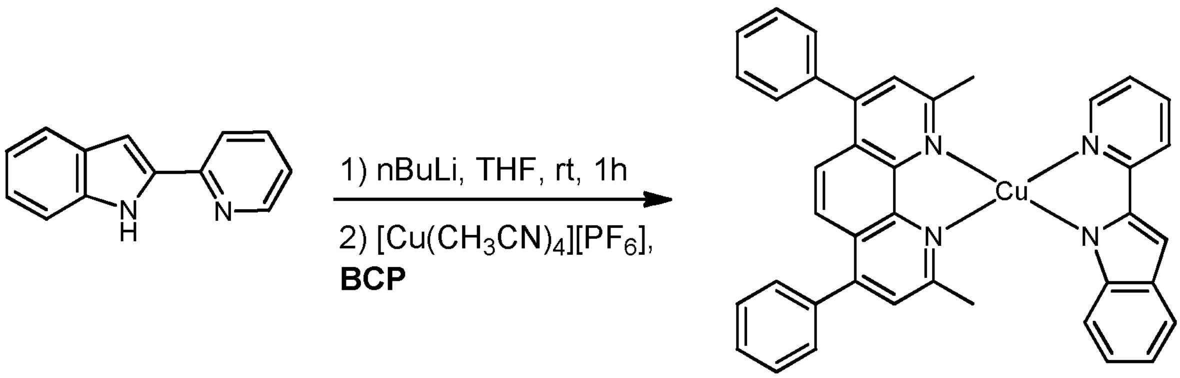


**Beispiel 34: Cu(Phen17)(N∩N)**

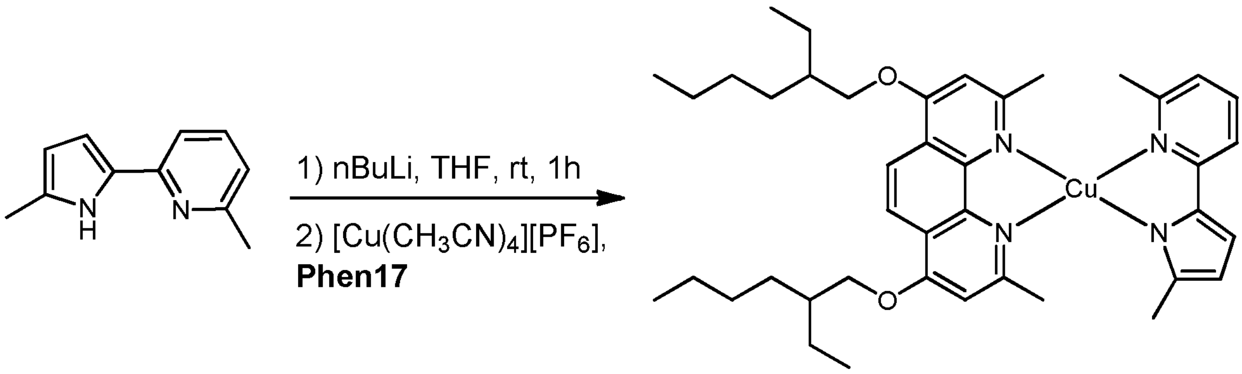


**Beispiel 35**

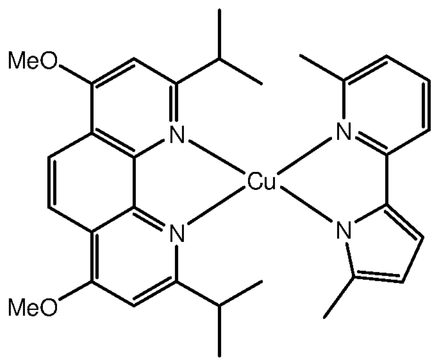

**Beispiel 36**

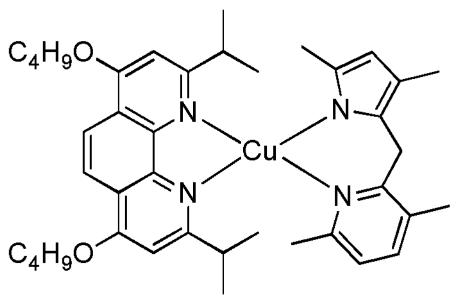

**Beispiel 37**

Unter Stickstoffatmosphäre wurde 8-Quinolinol in THF mit nBuLi bei Raumtemperatur deprotoniert, 1 Stunde rühren gelassen und mit der äquimolaren Menge an $[Cu(CH_3CN)_4]PF_6$ versetzt. Nach 2 h wurde der entsprechende Phen-Ligand in äquimolarer Menge zugeben. Das Produkt fiel aus und wurde abfiltriert. Nach dem Waschen mit Wasser, kaltem Acetonitril und Diethylether wurde das Produkt im Vakuum getrocknet.

**Cu(Phen16)(N∩O)**

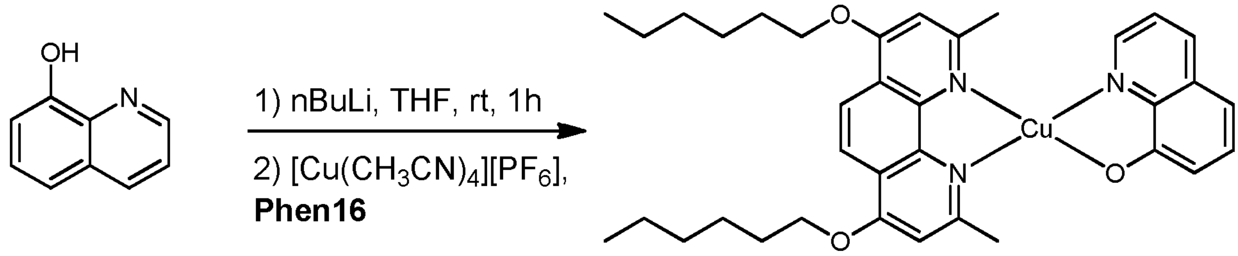


**Beispiel 38: Cu(Phen17)(N∩O)**

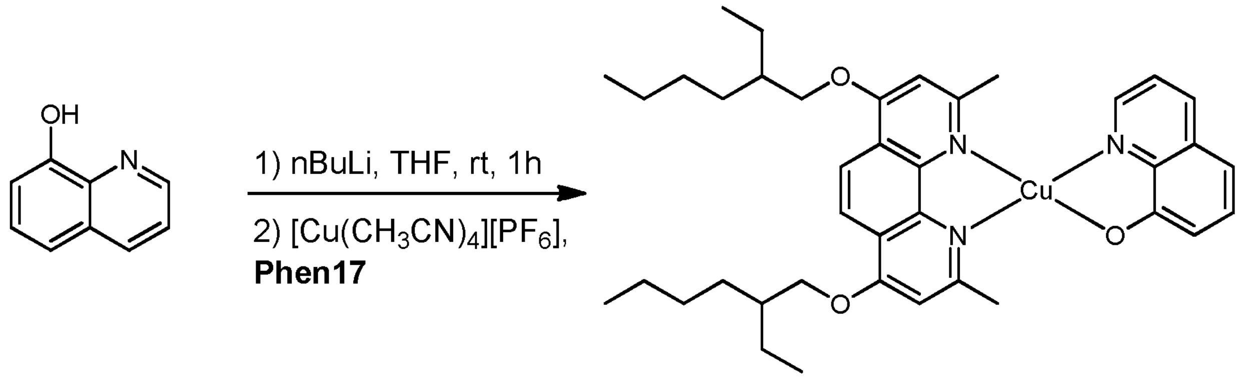


**Beispiel 39: Cu(BCP)(N∩O)**

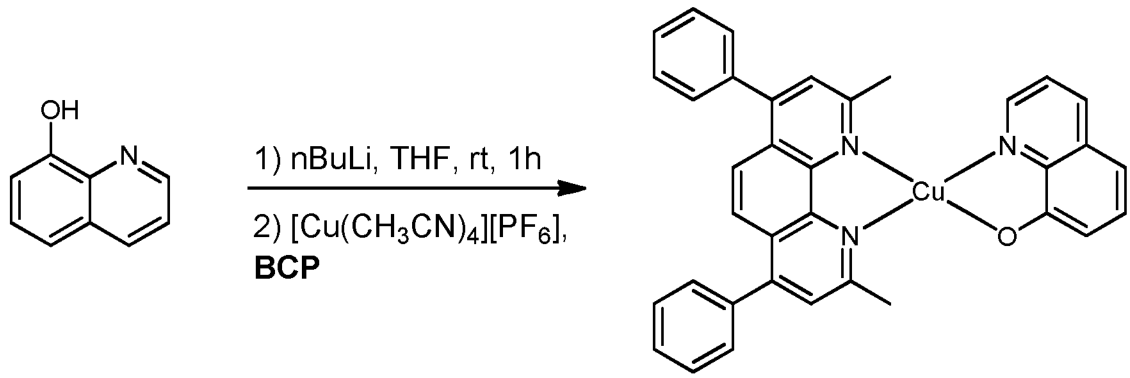


**Beispiel 40**

Unter Stickstoffatmosphäre wurden äquimolare Mengen an [n-$Bu_4$N](N∩N2) und [$Cu(CH_3CN)_4$]$PF_6$ in Acetonitril gerührt. Nach 2 h wurde der entsprechende Phen-Ligand in äquimolarer Menge zugeben. Das Produkt fiel aus und wurde abfiltriert. Nach dem Waschen mit Wasser, kaltem Acetonitril und Diethylether wurde das Produkt im Vakuum getrocknet.

**Cu(Phen16)(N∩N2)**

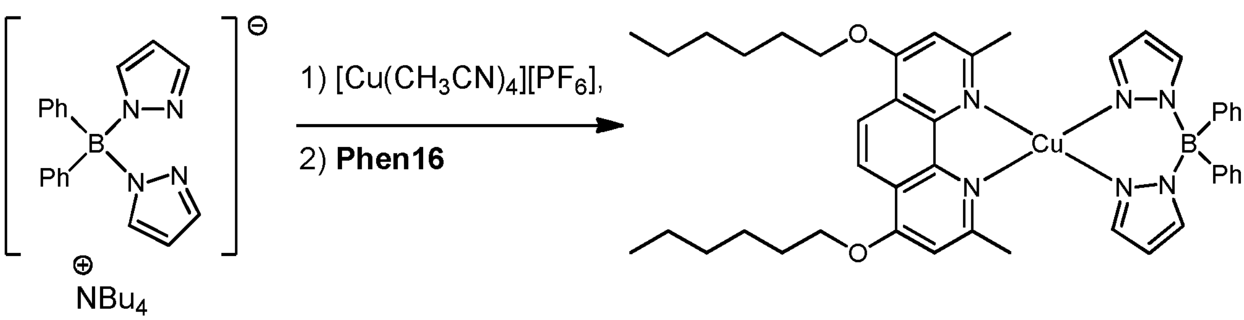


**Beispiel 41: Cu(Phen17)(N∩N2)**

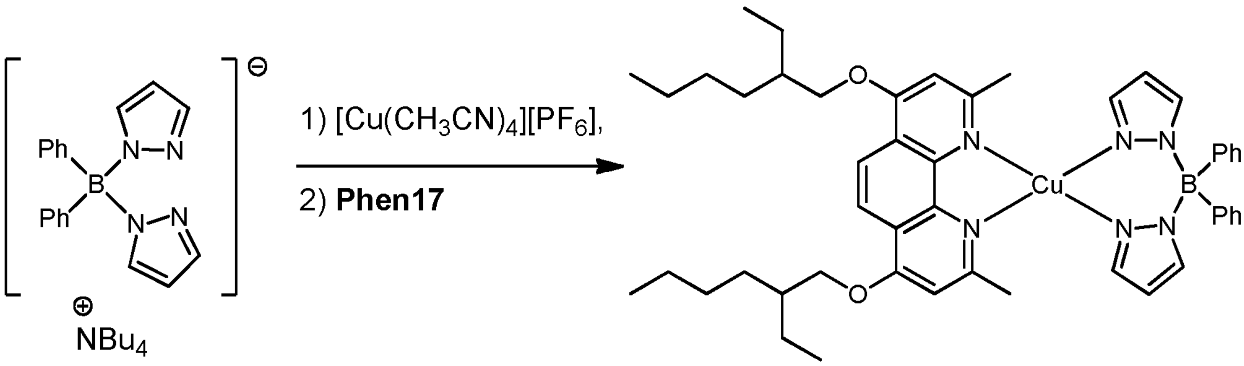


## Patentansprüche

1. Neutraler einkerniger Kupfer(I)komplex zur Emission von Licht mit einer Struktur gemäß Formel A

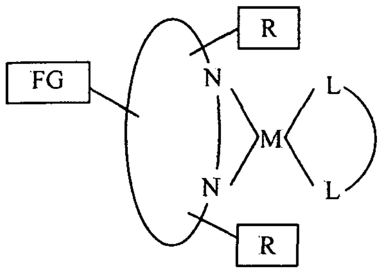

Formel A

worin bedeutet:

- M: Cu(I);
- L∩L: ein einfach negativ geladener, zweizähniger Ligand;
- N∩N: ein (durch R und FG substituierter) Diiminligand, nämlich ein substituiertes 2,2'-Bipyridin-Derivat (bpy) oder ein 1,10-Phenanthrolin-Derivat (phen);
- R: mindestens ein sterisch anspruchsvoller Substituent in 3,3'-Position (bpy) oder 2,9-Position (phen) zur Verhinderung einer Geometrieveränderung des Kupfer(I)komplexes in Richtung einer Planarisierung im angeregten Zustand;
- FG = Funktions-Gruppe: mindestens ein zweiter Substituent zur Elektronenleitung und zur Löslichkeitserhöhung in organischen Lösungsmitteln, oder mindestens ein zweiter Substituent zur Lochleitung und zur Löslichkeitserhöhung in organischen Lösungsmitteln,

wobei die Funktions-Gruppe werden entweder direkt oder über eine Brücke an den Diimin-Liganden gebunden

ist;

wobei der Kupfer(I)komplex

- einen $\Delta E(S_1-T_1)$-Wert zwischen dem untersten angeregten Singulett ($S_1$)- und dem darunter liegenden Triplett ($T_1$)-Zustand von kleiner als 2500 $cm^{-1}$ aufweist;
- eine Emissionslebensdauer von höchstens 20 $\mu$s aufweist;
- eine Emissionsquantenausbeuten von größer 40 % aufweist, und
- eine Löslichkeit in organischen Lösungsmitteln von mindestens 1 g/L aufweist.

**2.** Kupfer(I)komplex nach Anspruch 1, wobei

- der bpy-Ligand in 3,3'-Position mit einem Substituenten wie Alkyl [$CH_3$-$(CH_2)_n$-] (n = 1 - 20) (optional verzweigt) oder Aryl (insbesondere Phenyl) substituiert ist, oder wobei
- der phen-Ligand in 2,9-Position mit einem Substituenten wie Alkyl [$CH_3$-$(CH_2)_n$-] (n = 1 - 20) (optional verzweigt) oder Aryl (insbesondere Phenyl) substituiert ist.

**3.** Kupfer(I)komplex nach Anspruch 1 oder 2, wobei der mindestens eine sterisch anspruchsvolle Substituent R zur Verhinderung der Planarisierung des Komplexes im angeregten Zustand ausgewählt ist aus der Gruppe bestehend aus:

Alkylresten -$(CH_2)_n$-$CH_3$ (n = 0 - 20) (optional verzweigt), Arylresten mit 6 - 20 Kohlenstoffatomen (z. B. -OPh), Alkoxyresten -O-$(CH_2)_n$-$CH_3$ (n = 0 - 20), Aryloxyresten (z. B. -OPh) und Silanresten (z. B -$SiMe_3$), wobei die Alkyl- und Arylreste substituiert (z. B. mit Halogenen, Alkoxy- oder SilanGruppen, usw.) und/oder zu anellierten Ringsystemen fusioniert sein können.

**4.** Kupfer(I)komplex nach Anspruch 1 bis 3, wobei der mindestens eine sterisch anspruchsvolle Substituent R die Löslichkeit des Kupfer(I)komplexes in organischen Lösungsmitteln erhöht und/oder die Loch- oder die Elektronenleitung erhöht.

**5.** Kupfer(I)komplex nach Anspruch 1 bis 4, wobei der mindestens eine sterisch anspruchsvolle Substituent R zur Verhinderung der Planarisierung des Komplexes im angeregten Zustand eine aliphatische Gruppe ist.

**6.** Kupfer(I)komplex nach Anspruch 1 bis 5, wobei der Kupfer(I)komplex

- einen $\Delta E(S_1-T_1)$-Wert von kleiner als 1500 $cm^{-1}$, bevorzugt von kleiner als 1000 $cm^{-1}$, besonders bevorzugt von kleiner als 500 $cm^{-1}$ aufweist;
- eine Emissionsquantenausbeuten von größer 40 %, bevorzugt größer 60 %, besonders bevorzugt größer 70 % aufweist;
- eine Emissionslebensdauer von höchstens 10 $\mu$s, bevorzugt kleiner 6 $\mu$s, besonders bevorzugt kleiner 3 $\mu$s aufweist; und/oder
- eine Löslichkeit in organischen Lösungsmitteln von mindestens 10 g/L aufweist.

**7.** Verwendung eines Kupfer(I)komplexes nach Anspruch 1 bis 6 zur Emission von Licht, insbesondere in einer Emitterschicht in einer opto-elektronischen Vorrichtung.

**8.** Verfahren zur Herstellung einer opto-elektronischen Vorrichtung,
wobei ein Kupfer(I)komplex nach Anspruch 1 bis 7 verwendet wird.

**9.** Verfahren zur Herstellung einer opto-elektronischen Vorrichtung nach Anspruch 8,
wobei die Herstellung naß-chemisch erfolgt und das Verfahren die folgenden Schritte aufweist:

- Aufbringung eines in einem ersten Lösungsmittel gelösten ersten Emitterkomplexes auf einen Träger, und
- Aufbringung eines in einem zweiten Lösungsmittel gelösten zweiten Emitterkomplexes auf den Träger;

wobei

- der erste Emitterkomplexes nicht in dem zweiten Lösungsmittel löslich ist und

- der zweite Emitterkomplexes nicht in dem ersten Lösungsmittel löslich ist;

und wobei der erste Emitterkomplex und/oder der zweite Emitterkomplex ein Kupfer(I)komplex nach Anspruch 1 bis 6 ist.

**10.** Verfahren nach Anspruch 9, wobei das Verfahren weiterhin den folgenden Schritt aufweist:

- Aufbringung eines in dem ersten Lösungsmittel oder in einem dritten Lösungsmittel gelösten dritten Emitterkomplexes auf den Träger,

wobei der dritte Kupfer(I)komplex ein Kupfer(I)komplex nach Anspruch 1 bis 6 ist.

**11.** Verfahren nach Anspruch 10, wobei die opto-elektronische Vorrichtung eine Weißlicht-OLED ist, wobei

- der erste Emitterkomplexe ein Rotlichtemitter ist,
- der zweite Emitterkomplexe ein Grünlichtemitter ist und
- der dritte Emitterkomplexe ein Blaulichtemitter ist.

**12.** Opto-elektronische Vorrichtung, aufweisend einen Kupfer(I)komplex nach den Ansprüchen 1 bis 7.

**13.** Opto-elektronische Vorrichtung nach Anspruch 12, wobei der Anteil des Kupfer(I)komplexes in einer Emitterschicht 2 bis 100 Gew.-%, bevorzugt 4 bis 30 Gew.-%, bezogen auf das Gesamtgewicht der Emitterschicht, beträgt.

**14.** Opto-elektronische Vorrichtung nach Anspruch 12 oder 13 in Form einer organischen Leuchtdiode (OLED), **gekennzeichnet durch** einen Kupfer(I)komplex nach Anspruch 1 bis 7 aufweisende Emitterschicht, wobei der Anteil des Kupfer(I)komplexes in der Emitterschicht zwischen 2 bis 100 Gew.-%, bevorzugt 4 bis 30 Gew.-%, bezogen auf das Gesamtgewicht der Emitterschicht, beträgt.

**15.** Verwendung nach Anspruch 7, Verfahren nach Anspruch 8 bis 10, opto-elektronische Vorrichtung nach Anspruch 12 oder 13 wobei die opto-elektronische Vorrichtung ausgewählt ist aus der Gruppe bestehend aus organischen Leuchtidioden (OLEDs), lichtemittierenden elektrochemischen Zellen (LEECs oder LECs), OLED-Sensoren, insbesondere nicht hermetisch nach außen abgeschirmten Gas- und Dampf-Sensoren, optischen TemperaturSensoren, organischen Solarzellen (OSCs), organischen Feldeffekttransistoren, organischen Lasern, organischen Dioden, organischen Photodioden und "down conversion" Systemen.

**16.** Verfahren zur Auswahl von Cu(I)-Komplexen nach Anspruch 1 bis 6, deren $\Delta E(S_1\text{-}T_1)$-Wert zwischen dem untersten angeregten Singulett- ($S_1$) und dem darunter liegenden TriplettZustand ($T_1$) kleiner als 2500 $cm^{-1}$, bevorzugt kleiner als 1500 $cm^{-1}$, besonders bevorzugt kleiner als 1000 $cm^{-1}$, ganz besonders bevorzugt kleiner 500 $cm^{-1}$ ist, **gekennzeichnet durch**

- Bestimmung des $\Delta E(S_1\text{-}T_1)$-Wertes

  - mittels einer ab-initio Molekülrechnung oder
  - mittels Messung der Temperaturabhängigkeit der Fluoreszenz- und der Phosphoreszenz-Intensität oder
  - mittels der Messung der Temperaturabhängigkeit der Emissionslebensdauer und

- Ermittlung der von organischen Molekülen, deren $\Delta E(S_1\text{-}T_1)$-Wert kleiner als 2500 $cm^{-1}$, bevorzugt kleiner als 1500 $cm^{-1}$, besonders bevorzugt kleiner als 1000 $cm^{-1}$, ganz besonders bevorzugt kleiner 500 $cm^{-1}$ ist.

## Claims

**1.** Neutral monodentate copper(I) complex for the emission of light with a structure according to formula A

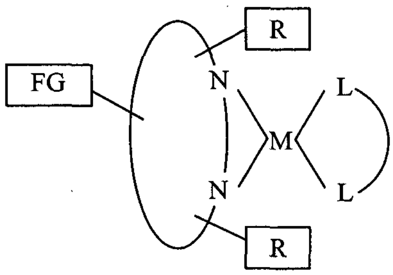

Formula A

wherein:

- M: Cu(I);
- L∩L: a singly negatively charged, bidentate ligand;
- N∩N: a diimine ligand (substituted by R and FG) that is a substituted 2,2'-bipyridine derivative (bpy) or a 1,10-phenanthroline derivative (phen);
- R: at least one sterically demanding substituent in 3,3'-position (bpy) or 2,9-position (phen) for preventing a change in geometry of the copper(I)complex in the direction of a planarization in the excited state;
- FG = function group: at least a second substituent for conducting electrons and for increasing solubility in organic solvents, or at least a second substituent for conducting holes and for increasing solubility in organic solvents,

wherein the function group is bound either directly or via a bridge to the diimin-ligand;

wherein the copper(I) complex has

- a $\Delta E(S_1-T_1)$-value between the lowermost exited singlet ($S_1$) and the triplet ($T_1$) state beneath it of smaller than 2500 $cm^{-1}$;
- an emission decay time of at the most 20 $\mu s$;
- an emission quantum yield of greater than größer 40 %; and
- a solubility in organic solvents in of least 1 g/L.

**2.** Copper(I)complex of claim 1, wherein

- the bpy-ligand in 3,3'-position is substituted with a substituent like alkyl $[CH_3-(CH_2)_n-]$ (n = 1 - 20) (optionally branched) or aryl (in particular phenyl) or, wherein
- the phen-ligand is substituted in 2,9-position with a substituent like alkyl $[CH_3-(CH_2)_n-]$ (n = 1 - 20) (optionally branched) or aryl (in particular phenyl).

**3.** Copper(I)complex of claims 1 or 2, wherein the at least one sterically demanding substituent R for preventing the planarization of the complex in the exited state is choosen from the group of consisting of:

alkyl $-(CH_2)_n-CH_3$ (n = 0 - 20) (optionally branched), aryl with 6 - 20 carbon atoms (e.g. -OPh), alkoxyl $-O-(CH_2)_n-CH_3$ (n = 0 - 20), aryloxy (e.g. -OPh) and silane (e.g. $-SiMe_3$),

wherein alkyl- and aryl- are optionally substituted (e.g. with halogen, alkoxy- or silane-groups, etc.) and/or are optionally fused to form annulated ring systems.

**4.** Copper(I)complex of claims 1 to 3, wherein the at least one sterically demanding substituent R increases the solubility of the copper(I)complexes in organics solvents and/or increases the hole or electron conductivity.

**5.** Copper(I)complex of claims 1 to 4, wherein the at least one sterically demanding substituent R for preventing the planarizatioin of the complex in the exited state is an aliphatic group.

**6.** Copper(I)complex of claims 1 to 5, wherein the copper(I) complex has

- an $\Delta E(S_1-T_1)$-value of smaller than 1500 $cm^{-1}$, preferably of smaller than 1000 $cm^{-1}$, particularly preferred of

smaller than 500 $cm^{-1}$;
- an emission quantum yield of larger 40 %, preferably of larger 60 %, particularly preferred of larger 70 % ;
- an emission decay time of at the most 10 μs, preferably smaller than 6 μs, particulary preferred smaller than 3 μs; and/or
- a solibility in organic solvents of at least 10 g/L.

**7.** Use of a copper(I) complex of claims 1 to 6 for the emission of light, in particular in an emitter layer in an optoelectronic device.

**8.** A method for manufacturing an optoelectronic device, wherein a copper(I) complex of claims 1 to 6 is used.

**9.** The method for manufacturing an optoelectronic device of claim 8,
wherein the manufacture is performed wet-chemically and the method comprises the following steps:

- applying a first emitter complex dissolved in a first solvent onto a carrier, and
- applying a second emitter complex dissolved in a second solvent onto the carrier;

wherein

- the first emitter complex is not soluble in the second solvent and
- the second emitter complex is not soluble in the first solvent;

and wherein the first emitter complex and/or the second emitter complex is an emitter complex of claims 1 to 6.

**10.** The method of claim 9, wherein the method further comprises the following step:

- applying a first emitter complex dissolved in the first solvent or in a third solvent on to the carrier, wherein the third copper(I) complex is a copper(I) complex of claims 1 to 6.

**11.** The method of claim 10, wherein the optoelectronic device is a whitelight OLED, wherein

- the first emitter complex is a redligth emitter,
- the second emitter complex is a greenlight emitter, and
- the third emitter complex is a bluelight emitter.

**12.** Optoelectronic device comprising a copper(I) complex of claims 1 to 6.

**13.** Optoelectronic device of claim 12,
wherein the fraction of the copper(I) complexes in an emitter layer is 2 to 100 weight-%, preferably 4 to 30 weight-%, in respect to the total weight of the emitter layer.

**14.** Optoelectronic device of claims 12 or 13 in form of an organic light emitting diode (OLED) **characterized by** an emitter layer comprising a copper(I) complex of claims 1 to 6, wherein the fraction of the copper(I) complexes in the emitter layer is between 2 to 100 weight-%, preferably 4 to 30 weight-%, in relation to the total weight of the emitter layer.

**15.** Use of claim 7, method of claims 8 to 10, optoelectronic device of claims 12 or 13, wherein the optoelectronic device is selected from the group consisting of organic light emitting diodes (OLEDs), light emitting electrochemical cells (LEECs or LECs), OLED-sensores, in particular non-hermetically sealed gas and vapor sensors, optical temperature sensors, organic solar cells (OSCs), organic fieldeffect transistors, organic lasers, organic diodes, organic photo diodes and "down conversion" systems.

**16.** A method for selecting Cu(I) complexes of claims 1 to 6, whose $\Delta E(S_1\text{-}T_1)$-value between the lowermost exited singlet ($S_1$) and the triplet ($T_1$) state beneth it is smaller than 2500 $cm^{-1}$, preferably smaller than 1500 $cm^{-1}$, particulary preferred smaller than 1000 $cm^{-1}$, especially preferred smaller than 500 $cm^{-1}$, **characterized by**

- determining the $\Delta E(S_1\text{-}T_1)$-value

- using an ab-initio molecul calculation or
- measuring the temparature dependence of fluorescence and the phosphorescence intensity, or
- measuring the temperature dependence of the emission decay time, and

- determining those organic molecules whose $\Delta E(S_1-T_1)$-value is smaller than 2500 $cm^{-1}$, preferably smaller than 1500 $cm^{-1}$, particulary preferred smaller than 1000 $cm^{-1}$, specially preferred smaller than 500 $cm^{-1}$.

**Revendications**

**1.** Complexe de cuivre(I) mononucléaire neutre destiné à l'émission de lumière, ayant une structure selon la formule A

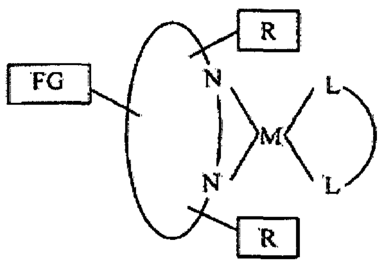

formule A

dans laquelle :

- M : Cu(I) ;
- L∩L : un ligand bidenté à une seule charge négative ;
- N∩N : un ligand diimine (substitué par R et FG), à savoir un dérivé de 2,2'-bipyridine (bpy) substitué ou un dérivé de 1,10-phénanthroline (phén) ;
- R : au moins un substituant à encombrement stérique en position 3,3' (bpy) ou position 2,9 (phén) pour empêcher une variation de géométrie du complexe de cuivre(I) dans le sens d'une planarisation à l'état excité ;
- FG = groupe fonctionnel : au moins un deuxième substituant pour la conduction par électrons et pour l'augmentation de la solubilité dans des solvants organiques, ou au moins un deuxième substituant pour la conduction par trous et pour l'augmentation de la solubilité dans des solvants organiques,
le groupe fonctionnel étant lié soit directement soit par un pont au ligand diimine ;

le complexe de cuivre(I)

- présentant une valeur $\Delta E(S_1-T_1)$ inférieure à 2 500 $cm^{-1}$ entre l'état singulet excité le plus bas ($S_1$) et l'état triplet se trouvant au-dessous ($T_1$) ;
- présentant une durée de vie d'émission d'au maximum 20 $\mu s$ ;
- présentant un rendement quantique d'émission supérieur à 40 %, et
- présentant une solubilité dans des solvants organiques d'au moins 1 g/l.

**2.** Complexe de cuivre(I) selon la revendication 1, dans lequel

- le ligand bpy est substitué en position 3,3' par un substituant tel qu'un groupe alkyle $[CH_3-(CH_2)_n-]$ (n = 1 - 20) (en option ramifié) ou aryle (en particulier phényle), ou dans lequel
- le ligand phén est substitué en position 2,9 par un substituant tel qu'un groupe alkyle $[CH_3-(CH_2)_n$ (n = 1 - 20) (en option ramifié) ou aryle (en particulier phényle).

**3.** Complexe de cuivre(I) selon la revendication 1 ou 2, dans lequel ledit au moins un substituant à encombrement stérique R destiné à empêcher la planarisation du complexe à l'état excité est choisi dans le groupe constitué par :

des radicaux alkyle $-(CH_2)_n-CH_3$ (n = 0 - 20) (en option ramifiés), des radicaux aryle ayant de 6 à 20 atomes de carbone (par exemple -OPh), des radicaux alcoxy $-O-(CH_2)_n-CH_3$ (n = 0 - 20), des radicaux aryloxy (par exemple -OPh), et des radicaux silane (par exemple $-SiMe_3$),

les radicaux alkyle et aryle pouvant être substitués (par exemple par des halogènes, des groupes alcoxy ou silane, etc.) et/ou fusionnés en systèmes cycliques condensés.

**4.** Complexe de cuivre(I) selon l'une quelconque des revendications 1 à 3, dans lequel ledit au moins un substituant R à encombrement stérique augmente la solubilité du complexe de cuivre(I) dans des solvants organiques et/ou augmente la conduction par électrons ou la conduction par trous.

**5.** Complexe de cuivre(I) selon l'une quelconque des revendications 1 à 4, dans lequel ledit au moins un substituant R à encombrement stérique destiné à empêcher la planarisation du complexe à l'état excité est un groupe aliphatique.

**6.** Complexe de cuivre(I) selon l'une quelconque des revendications 1 à 5, dans lequel le complexe de cuivre(I)

- présente une valeur $\Delta E(S_1\text{-}T_1)$ inférieure à 1 500 $cm^{-1}$, de préférence inférieure à 1 000 $cm^{-1}$, de façon particulièrement préférée inférieure à 500 $cm^{-1}$;
- présente un rendement quantique d'émission supérieur à 40 %, de préférence supérieur à 60 %, de façon particulièrement préférée supérieur à 70 %,
- présente une durée de vie d'émission d'au maximum 10 $\mu$s, de préférence inférieur à 6 $\mu$s, de façon particulièrement préférée inférieur à 3 $\mu$s : et/ou
- présente une solubilité dans des solvants organiques d'au moins 10 g/l.

**7.** Utilisation d'un complexe de cuivre(I) selon l'une quelconque des revendications 1 à 6, pour l'émission de lumière, en particulier dans une couche émettrice dans un dispositif optoélectronique.

**8.** Procédé pour la fabrication d'un dispositif optoélectronique,
dans lequel on utilise un complexe de cuivre(I) selon l'une quelconque des revendications 1 à 6.

**9.** Procédé pour la fabrication d'un dispositif optoélectronique selon la revendication 8, dans lequel la fabrication s'effectue chimiquement par voie humide et le procédé comporte les étapes suivantes :

- application sur un support d'un premier complexe émetteur dissous dans un premier solvant, et
- application sur le support d'un deuxième complexe émetteur dissous dans un deuxième solvant ;
- le premier complexe émetteur n'étant pas soluble dans le deuxième solvant et
- le deuxième complexe émetteur n'étant pas soluble dans le premier solvant ;

et le premier complexe émetteur et/ou le deuxième complexe émetteur étant un complexe de cuivre(I) selon l'une quelconque des revendications 1 à 6.

**10.** Procédé selon la revendication 9, le procédé comportant en outre l'étape suivante :

- application sur le support d'un troisième complexe émetteur, dissous dans le premier solvant ou dans un troisième solvant,
le troisième complexe de cuivre(I) étant un complexe de cuivre(I) selon l'une quelconque des revendications 1 à 6.

**11.** Procédé selon la revendication 10, dans lequel le dispositif optoélectronique est une OLED à lumière blanche,

- le premier complexe émetteur étant un émetteur de lumière rouge,
- le deuxième complexe émetteur étant un émetteur de lumière verte et
- le troisième complexe émetteur étant un émetteur de lumière bleue.

**12.** Dispositif optoélectronique, comportant un complexe de cuivre(I) selon les revendications 1 à 6.

**13.** Dispositif optoélectronique selon la revendication 12,
dans lequel la proportion du complexe de cuivre(I) dans une couche émettrice vaut de 2 à 100 % en poids, de préférence de 4 à 30 % en poids, par rapport au poids total de la couche émettrice.

**14.** Dispositif optoélectronique selon la revendication 12 ou 13, sous forme d'une diode électroluminescente organique (OLED), **caractérisé par** une couche émettrice comportant du complexe de cuivre(I) selon l'une quelconque des revendications 1 à 6, la proportion du complexe de cuivre(I) dans la couche émettrice étant comprise entre 2 et 100

% en poids, de préférence entre 4 et 30 % en poids, par rapport au poids total de la couche émettrice.

**15.** Utilisation selon la revendication 7, procédé selon l'une quelconque des revendications 8 à 10, dispositif optoélectronique selon la revendication 12 ou 13, dans lesquels le dispositif optoélectronique est choisi dans le groupe constitué par des diodes électroluminescentes organiques (OLED), des cellules électrochimiques photoémettrices (LEEC ou LEC), des capteurs OLED, en particulier des capteurs de gaz et de vapeurs non hermétiquement isolés de l'extérieur, des capteurs optiques de température, des cellules solaires organiques (OSC), des transistors à effet de champ organiques, des lasers organiques, des diodes organiques, des photodiodes organiques et des systèmes de « down conversion ».

**16.** Procédé pour le choix de complexes de Cu(I) selon l'une quelconque des revendications 1 à 6, dont la valeur $\Delta E(S_1-T_1)$ entre l'état singulet excité le plus bas ($S_1$) et l'état triplet se trouvant au-dessous ($T_1$) est inférieure à 2 500 $cm^{-1}$, de préférence inférieure à 1 500 $cm^{-1}$, de façon particulièrement préférée inférieure à 1 000 $cm^{-1}$, de façon tout particulièrement préférée inférieure à 500 $cm^{-1}$, **caractérisé par**

- la détermination de la valeur $\Delta E(S_1-T_1)$

  - au moyen d'un calcul moléculaire ab initio ou
  - par mesure de la dépendance de l'intensité de la fluorescence et de la phosphorescence à l'égard de la température ou
  - au moyen de la mesure de la dépendance de la durée de vie de l'émission à l'égard de la température et

- la détermination des molécules organiques dont la valeur $\Delta E(S_1-T_1)$ est inférieure à 2 500 $cm^{-1}$, de préférence inférieure à 1 500 $cm^{-1}$, de façon particulièrement préférée inférieure à 1 000 $cm^{-1}$, de façon tout particulièrement préférée inférieure à 500 $cm^{-1}$.

Figur 1

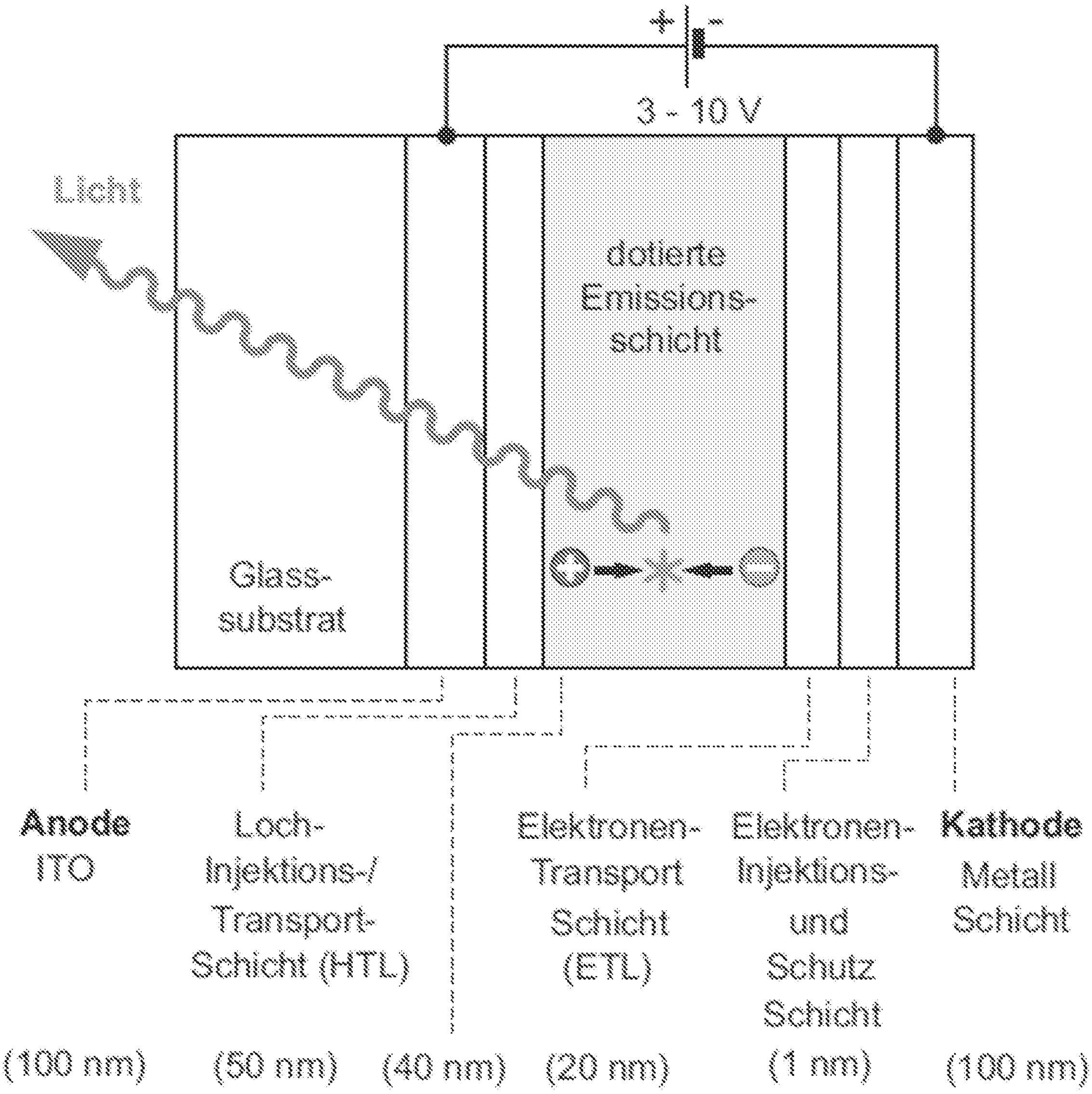
+
-
3 - 10 V
Licht
dotierte Emissions-schicht
Glass-substrat
Anode ITO
(100 nm)
Loch-Injektions-/Transport-Schicht (HTL)
(50 nm)
(40 nm)
Elektronen-Transport Schicht (ETL)
(20 nm)
Elektronen-Injektions- und Schutz Schicht
(1 nm)
Kathode Metall Schicht
(100 nm)

Figur 2

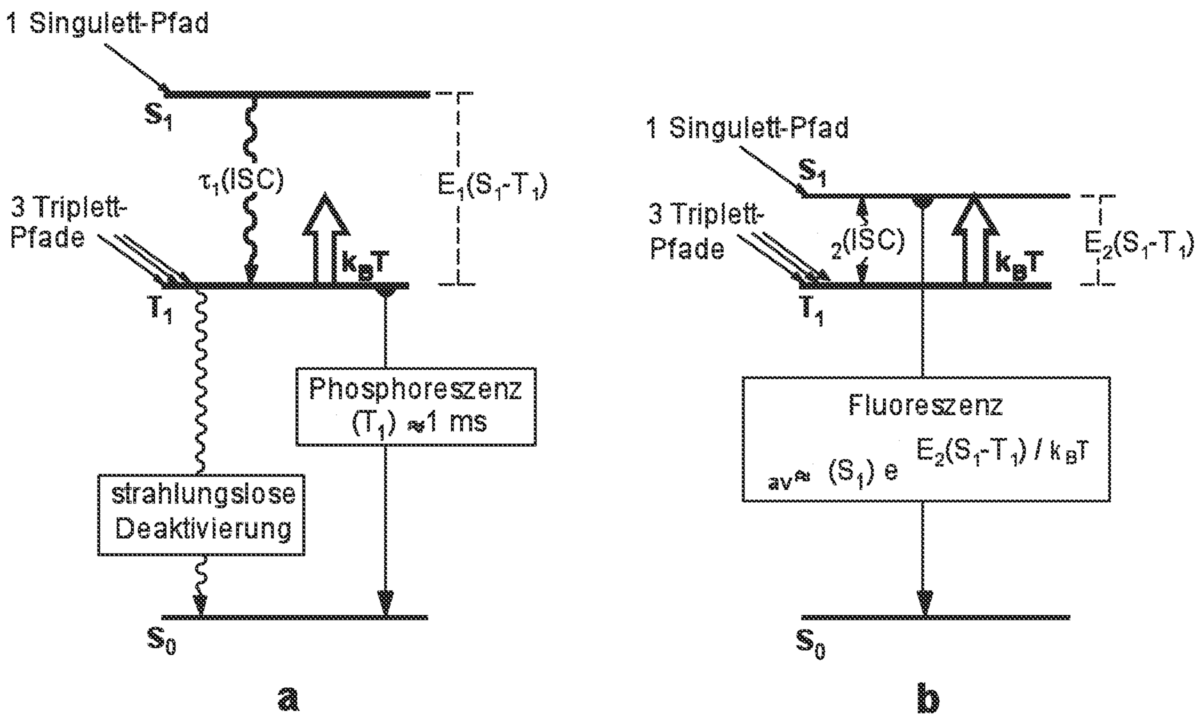
1 Singulett-Pfad
$S_1$
$\tau_1$(ISC)
$E_1(S_1-T_1)$
3 Triplett-Pfade
$k_BT$
$T_1$
Phosphoreszenz
$(T_1) \approx 1$ ms
strahlungslose Deaktivierung
$S_0$
a
1 Singulett-Pfad
$S_1$
3 Triplett-Pfade
2(ISC)
$k_BT$
$E_2(S_1-T_1)$
$T_1$
Fluoreszenz
av≈ $(S_1)$ e $E_2(S_1-T_1) / k_BT$
$S_0$
b

Figur 3

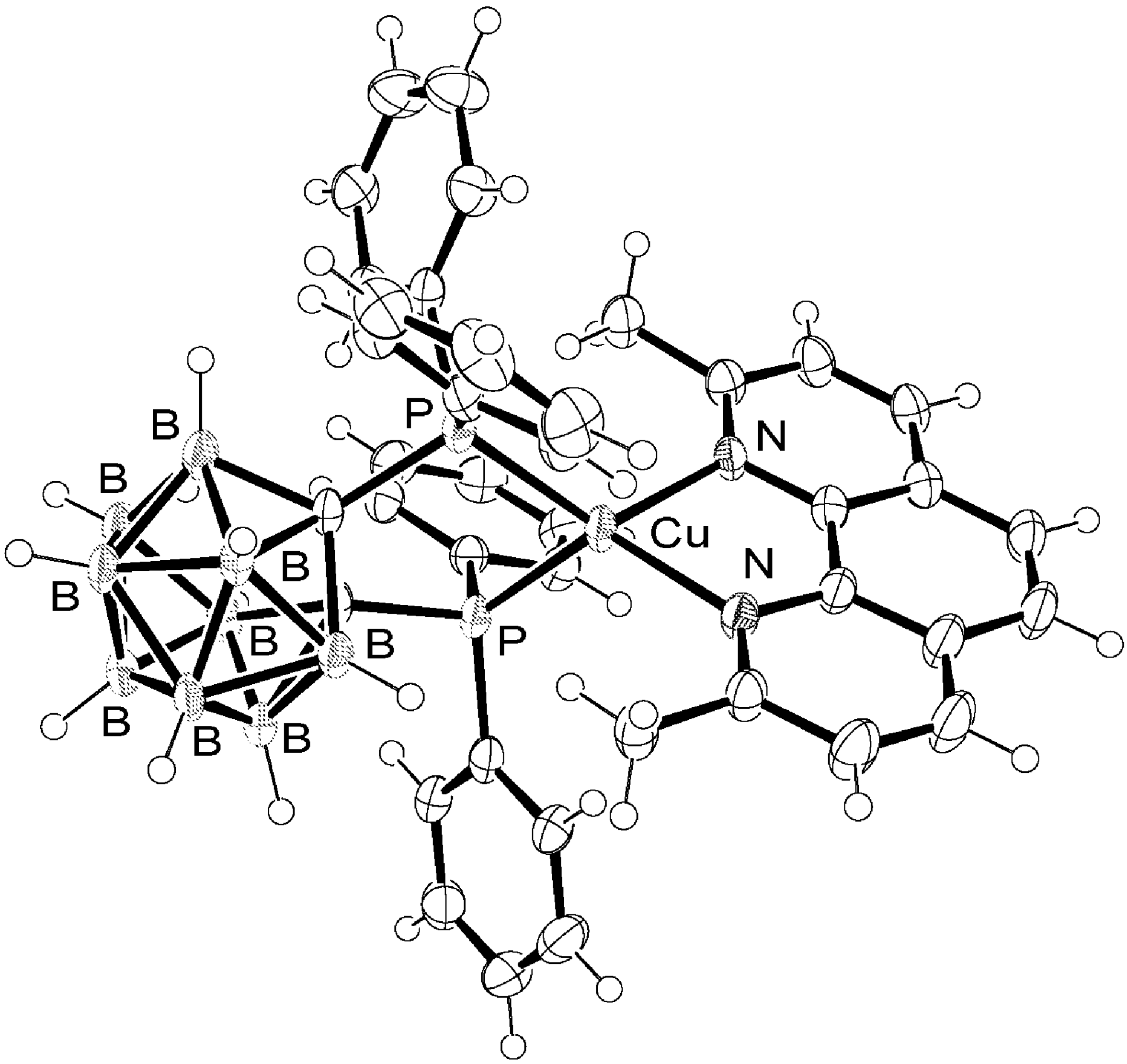

Figur 4

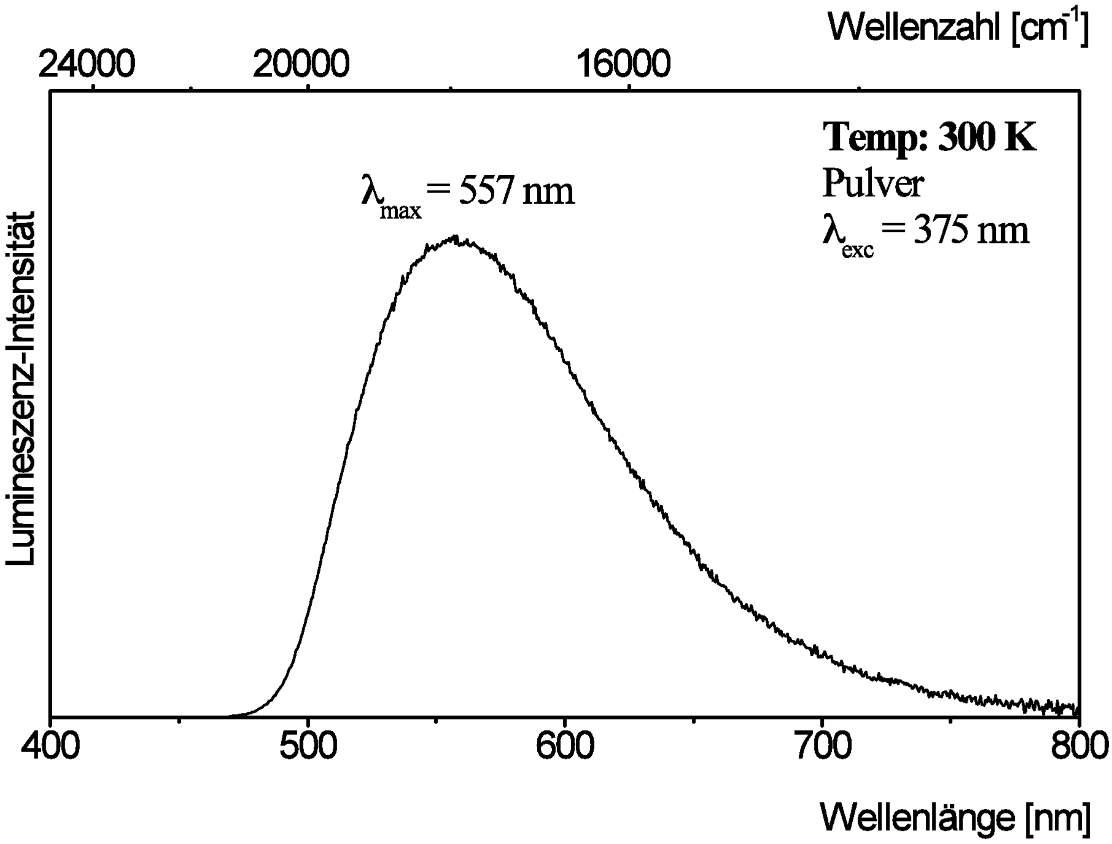
Wellenzahl [cm-1]
24000
20000
16000
Temp: 300 K
Pulver
λexc = 375 nm
λmax = 557 nm
Lumineszenz-Intensität
400
500
600
700
800
Wellenlänge [nm]

Figur 5

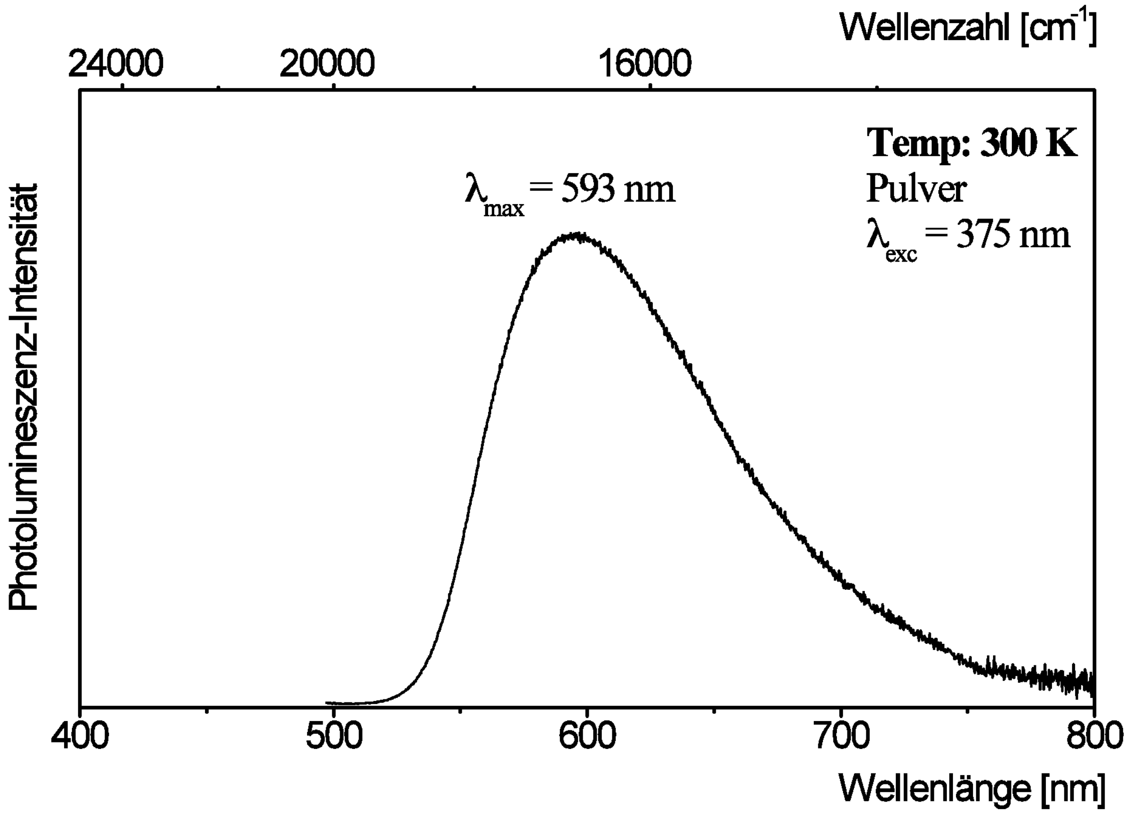
Wellenzahl [cm-1]
24000
20000
16000
Temp: 300 K
Pulver
λexc = 375 nm
λmax = 593 nm
Photolumineszenz-Intensität
400
500
600
700
800
Wellenlänge [nm]

**Figur 6**

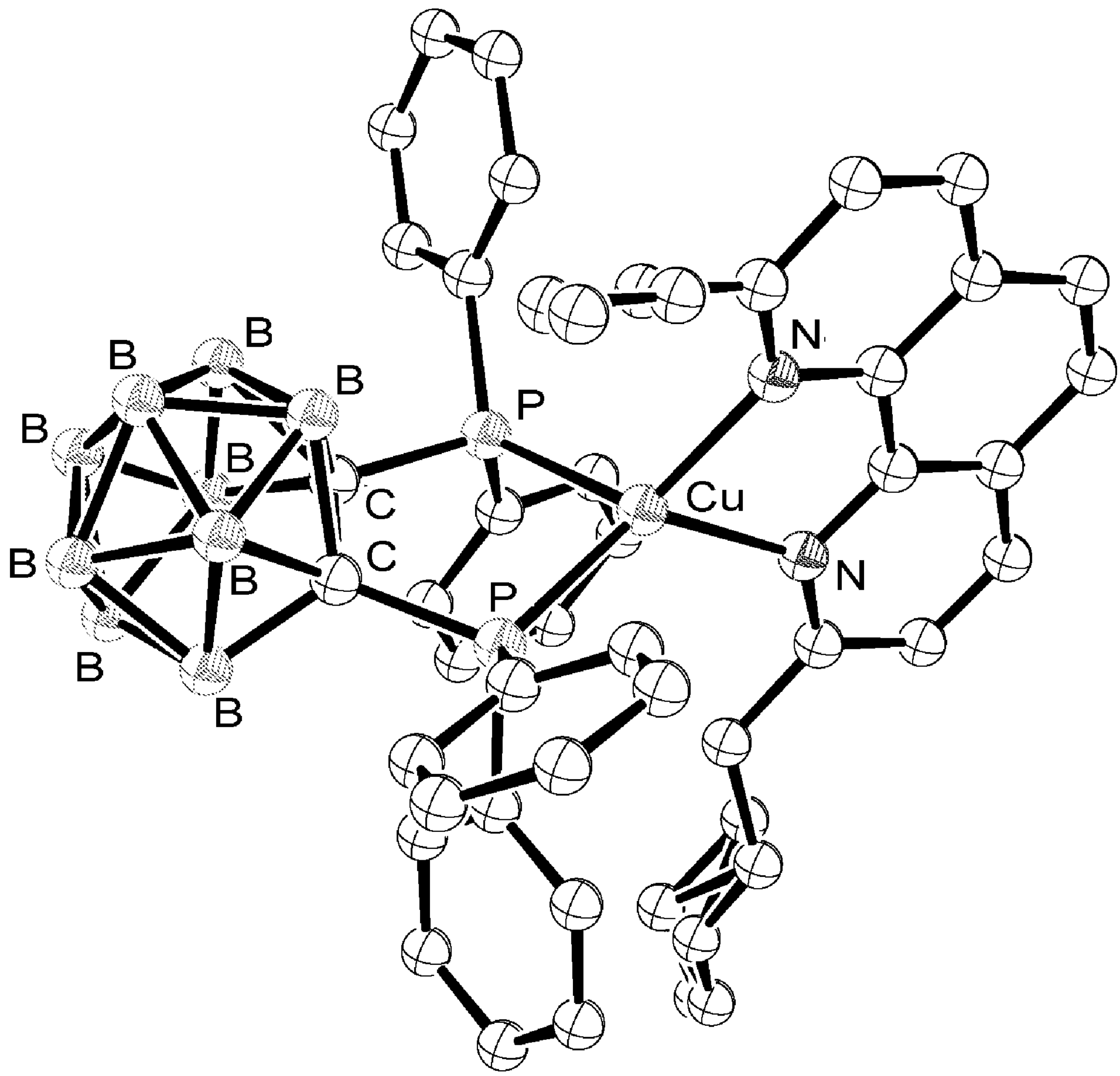

Figur 7

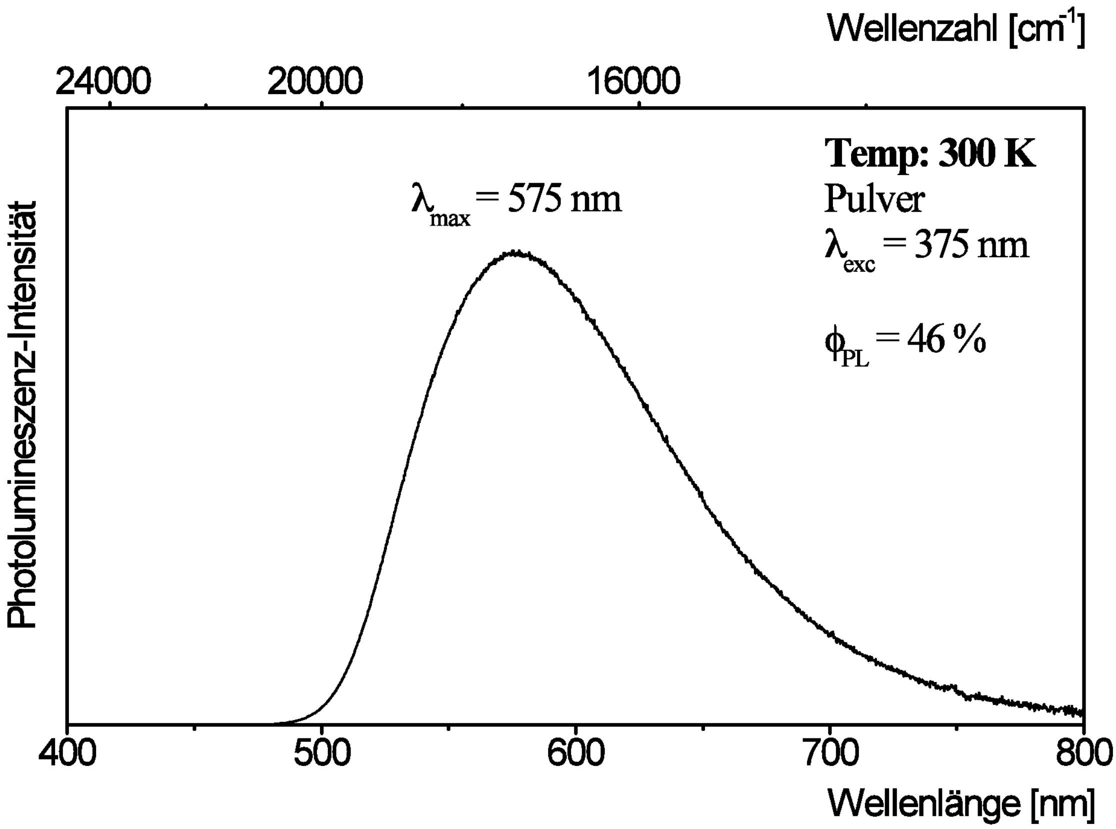
Wellenzahl [cm-1]
24000
20000
16000
Temp: 300 K
Pulver
λexc = 375 nm
φPL = 46 %
λmax = 575 nm
Photolumineszenz-Intensität
400
500
600
700
800
Wellenlänge [nm]

**Figur 8**

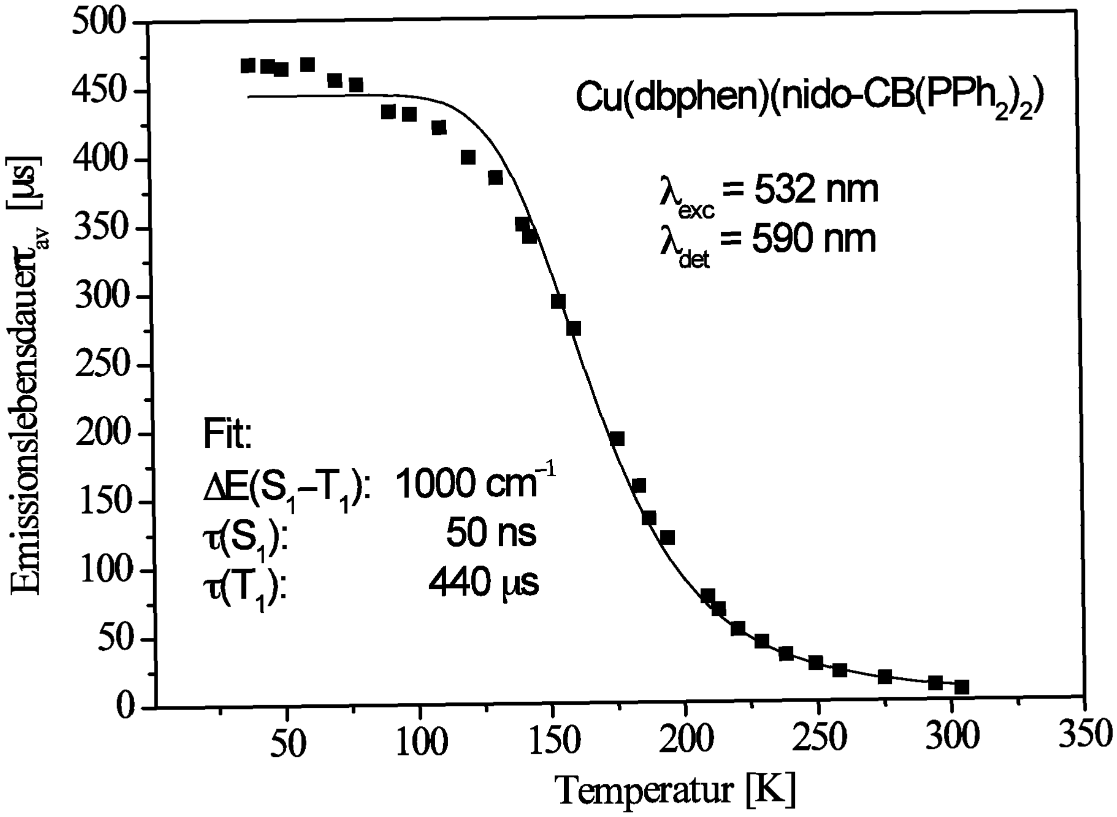
Cu(dbphen)(nido-CB(PPh2)2)
λexc = 532 nm
λdet = 590 nm
Fit:
ΔE(S1-T1): 1000 cm-1
τ(S1): 50 ns
τ(T1): 440 μs
Emissionslebensdauer τav [μs]
Temperatur [K]
0
50
100
150
200
250
300
350
400
450
500

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102007031261 A1


### In der Beschreibung aufgeführte Nicht-Patentliteratur


- **H. YERSIN.** *Top. Curr. Chem.,* 2004, vol. 241, 1
- **H. YERSIN.** Highly Efficient OLEDs with Phosphorescent Materials. Wiley-VCH, Weinheim, 2008
- **TANG et al.** *Appl. Phys. Lett.,* 1987, vol. 51, 913
- **M. A. BALDO ; D. F. O'BRIEN ; M. E. THOMPSON ; S. R. FORREST.** *Phys. Rev. B,* 1999, vol. 60, 14422
- **S. L. MUROV ; J. CARMICHEAL ; G. L. HUG.** Handbook of Photochemistry. Marcel Dekker, 1993, 338 ff
- **H. YERSIN.** Highly Efficient OLEDs with Phosphorescent Materials. Wiley-VCH, 2008
- **S. R. FORREST et al.** *Phys. Rev. B,* 2008, vol. 77, 235215
- **J. KIDO et al.** *Jap. J. Appl. Phys.,* 2007, vol. 46, L10
- **C.-Y. CHEN ; M. WANG ; J.-Y. LI ; N. POOTRAKULCHOTE ; L. ALIBABAEI ; C.-H. NGOC-LE ; J.-D. DECOPPET ; J.-H. TSAI ; C. GRÄTZEL ; C.-G. WU.** *ACS Nano,* 2009, vol. 3, 3103
- **G. CHELUCCI ; R. P. THUMMEL.** *Chem. Rev.,* 2002, vol. 102, 3129
- **M. SCHMITTEL ; H. AMMON.** *Eur. J. Org. Chem.,* 1998, 785
- **G. BUTT ; R. D. TOPSOM.** *J. Heterocyclic Chem.,* 1981, vol. 18, 641
- **B. KONING ; J. W. DE BOER ; A. MEETSMA ; R. M. KELLOGG.** *ARKIVOC,* 2004, 189
- **X. J. FENG ; P. L. WU ; H. L. TAM ; K. F. LI ; M. S. WONG ; K. W. CHEAH.** *Chem. - Eur. J.,* 2009, vol. 15, 11681
- **A. F. LARSEN ; T. ULVEN.** *Org. Lett.,* 2011, vol. 13, 3546
- **H. FRISELL ; B. AKERMARK.** *Organometallics,* 1995, vol. 14, 561
- **R. P. THUMMEL ; V. HEGDE.** *J. Org. Chem.,* 1989, vol. 54 (7), 1720
- **S. TROFIMENKO.** *J. Am. Chem. Soc.,* 1967, vol. 89, 6288
- **J. C. PETERS ; J. C. THOMAS.** *Inorg. Synth.,* 2004, vol. 34, 11
- **ZAKHARKIN, L. I. ; KALININ, V. N.** *Tetrahedron Lett.,* 1965, vol. 7, 407